# EUROPEAN PATENT APPLICATION

(11) **EP 4 190 345 A1**
(43) Date of publication of application: **07.06.2023**
(21) Application number: 21850144.3
(22) Date of filing: 29.07.2021
(51) Int. Cl.: A61K 38/10, A61K 31/138, A61K 31/704, A61K 39/395, A61P 15/00, A61P 17/02, A61P 27/02, A61P 29/00, A61P 35/00, A61P 35/02, A61P 43/00, C07K 7/08, C07K 14/505

(54) **TREATMENT OF DISEASE WITH EPOR ANTAGONIST**

(30) Priority: 30.07.2020 JP 2020129269
(71) Applicant: Epo-Med Inc., Tokyo 1050013 (JP)
(72) Inventor: KOUJI, Hiroyuki, Tokyo 105-0013 (JP); OCHI, Hiroshi, Kyoto-shi, Kyoto 602-0841 (JP); SAKAGUCHI, Takehisa, Kyoto-shi, Kyoto 602-0841 (JP); YASUDA, Yoshiko, Tokyo 105-0013 (JP)
(74) Representative: Vossius & Partner Patentanwälte Rechtsanwälte mbB
(86) International application number: PCT/JP2021/028154
(87) International publication number: WO 2022/025204

(57) **Abstract**

The present disclosure provides an anti-EpoR peptide for use in the treatment of a disease such as cancer. In the description, a peptide which has the structure: - [SCHFGPLTWVCK]- and which is intended to be used for antagonizing an Epo heteroreceptor selectively in the presence of erythropoietin (Epo) or an equivalent thereof, a modified peptide of the peptide, a prodrug of the modified peptide, or a salt of the peptide, the modified peptide or the prodrug (wherein, in the formula, an alphabetical letter represents a one-letter code for an amino acid residue) is provided. In one embodiment, a disease, a disorder or a symptom each characterized by the occurrence of a cell expressing an Epo heteroreceptor is treated or prevented.

## Description

### [Technical Field]

The present disclosure relates to treatment of diseases such as cancers with an anti-erythropoietin receptor (hereinafter, also referred to as "EpoR") peptide.

### [Background Art]

Erythropoietin (hereinafter, also referred to as "Epo") is involved in differentiation and proliferation of red blood cells. Unlike other cytokines, erythropoietin is not produced in hemocytes, but is produced in the kidney or liver and released into the blood. It is understood that erythropoietin acts on burst-forming unit-erythroid (BFU-E), colony-forming unit-erythroid (CFU-E), and proerythroblast among red blood cell progenitor cells and promotes the differentiation and proliferation thereof to induce the production of red blood cells (Krantz S.B., Blood, Vol.77, pp 419-434 (1991)). It is understood that when erythropoietin binds to an erythropoietin receptor that is present on the cellular membrane of a progenitor cell, a signal is transmitted into the cellular nucleus, inducing differentiation of red blood cells, i.e., intracellular accumulation of globin mRNA, production of hemoglobin, and proliferation of red blood cells (D'Andrea A. D. et al., Cell, Vol. 57, pp 277-285 (1989)). However, the detailed mechanism thereof is still not elucidated, with many that need to be resolved in the future.

An embryo immediately after implantation (Yasuda Y. et al., Develop. Growth Differ., Vol. 35, pp 711-722 (1993)), human, monkey, and mouse brain (Marti H. H. et al., Eur. J. NeuroSci., Vol.8, pp 666-676 (1996)), and mouse uterus (Yasuda Y. et al., J. Biol. Chem., Vol. 273, pp 25381-25387 (1998)) are known as sites where erythropoietin gene is expressed in tissue other than sites associated with erythroblasts.

Erythropoietin can act by binding to an erythropoietin receptor.

### [Citation List]

### [Non Patent Literature]

[NPL 1] Krantz S. B., Blood, Vol.77, pp 419-434 (1991)
[NPL 2] D'Andrea A. D. et al., Cell, Vol. 57, pp 277-285 (1989)
[NPL 3] Yasuda Y. et al., Develop. Growth Differ., Vol.35, pp 711-722 (1993)
[NPL 4] Marti H. H. et al., Eur. J. NeuroSci., Vol. 8, pp 666-676 (1996)
[NPL 5] Yasuda Y. et al., J. Biol. Chem., Vol. 273, pp 25381-25387 (1998)

### [Summary of Invention]

### [Solution to Problem]

The present disclosure provides a compound such as a peptide (peptide or a salt or solvate thereof, or a prodrug thereof) having a function of selectively antagonizing an erythropoietin (Epo) heteroreceptor in the presence of Epo, and a composition, use, and method for selectively antagonizing an Epo heteroreceptor in the presence of Epo. The present disclosure also provides a compound, composition, use, and method for treating or preventing a disease, disorder, or symptom associated with an Epo heteroreceptor. In a specific embodiment, the peptide of the present disclosure or a salt or solvate thereof, or a prodrug thereof is for selectively antagonizing an Epo heteroreceptor in the presence of Epo (an erythropoietin (Epo) heteroreceptor selective inhibitor), and has, for example, one or more characteristics from potent Epo heteroreceptor binding capability in the presence of Epo, weak Epo homodimer receptor binding capability in the presence of Epo, EpoR low affinity binding region selective binding or inhibitory capability, and low normal myeloid cell killing capability.

Therefore, the present disclosure provides the following.

### (Item 1)

A composition for use in selectively antagonizing an erythropoietin (Epo) heteroreceptor in the presence of Epo, comprising a peptide having a structure of -[SCHFGPLTWVCK]-, or a modified peptide based on said peptide, or a prodrug thereof, or a salt or solvate thereof (an alphabet indicates a one letter code of an amino acid).

### (Item 2)

The composition of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH (R^{A1}) -CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N (CH₃)₂, =O, -COOH, -OCH₃, and -OCH₂CH₃;
   or a prodrug thereof or a salt thereof.

### (Item 3)

The composition of any of the preceding items, satisfying at least one of the following conditions:
A² is Gly,
A³ is Phe,
A⁵ is Thr,
A⁷ is Cys, and
A⁸ is Lys.

### (Item 4)

The composition of any of the preceding items, wherein A¹ is Tyr, p-fluorophenylalanine, or phenethylglycine.

### (Item 5)

The composition of any of the preceding items, wherein A¹ is Tyr.

### (Item 6)

The composition of any of the preceding items, wherein A⁴ is Leu or Met.

### (Item 7)

The composition of any of the preceding items, wherein A⁴ is Met.

### (Item 8)

The composition of any of the preceding items, wherein A⁶ is Trp, Met, 8-naphthylalanine, 2-quinolylalanine, 5-chloro-Trp, or 2-benzothiazolylalanine.

### (Item 9)

The composition of any of the preceding items, wherein A⁶ is 2-benzothiazolylalanine.

### (Item 10)

The composition of any of the preceding items, wherein
A² is Gly,
A³ is Phe,
A⁵ is Thr,
A⁷ is Cys, and
A⁸ is Lys.

### (Item 11)

The composition of any of the preceding items, wherein X¹ is -COOH, a benzoyl group, a propionyl group, or a p-fluorophenylacetyl group.

### (Item 12)

The composition of any of the preceding items, wherein X² is NH₂.

### (Item 13)

The composition of any of the preceding items, wherein the peptide, or the modified peptide, or the prodrug thereof, or the salt or solvate thereof has substantially no affinity to an Epo homodimer receptor in the presence of Epo.

### (Item 14)

The composition of any of the preceding items, wherein the peptide, or the modified peptide, or the prodrug thereof, or the salt or solvate thereof has affinity to an Epo homodimer receptor in the absence of Epo.

### (Item 15)

The composition of any of the preceding items, wherein the Epo heteroreceptor comprises an Epo receptor and a βc receptor.

### (Item 16)

The composition of any of the preceding items, wherein the peptide, or the modified peptide based on said peptide, or the prodrug thereof, or the salt or solvate thereof, in the presence of Epo, does not antagonize an Epo homodimer receptor, but antagonizes an Epo heteroreceptor comprising an Epo receptor and a βc receptor.

### (Item 17)

The composition of any of the preceding items for administration in combination with an anticancer agent.

### (Item 18)

The composition of any of the preceding items for improving an adverse action elicited by an anticancer agent in the presence of Epo.

### (Item 19)

The composition of any of the preceding items, wherein the anticancer agent comprises an angiogenesis inhibitor.

### (Item 20)

The composition of any of the preceding items, wherein the anticancer agent comprises bevacizumab, tamoxifen, doxorubicin, or trastuzumab.

### (Item 21)

A composition for use in treating or preventing a disease, disorder, or symptom characterized by the presence of a cell expressing an Epo heteroreceptor, comprising a selective antagonist of an Epo heteroreceptor.

### (Item 22)

A composition for use in treating or preventing a disease, disorder, or symptom characterized by the presence of a cell expressing an Epo heteroreceptor, comprising a peptide having a structure of -[SCHFGPLTWVCK]-, or a modified peptide based on said peptide, or a prodrug thereof, or a salt or solvate thereof (an alphabet indicates a one letter code of an amino acid).

### (Item 23)

The composition of any of the preceding items, wherein administration of the composition does not induce anemia.

### (Item 24)

The composition of any of the preceding items, wherein the disease is a proliferative disease, rheumatism, diabetic retinopathy, keloid, or adenomyosis.

### (Item 25)

The composition of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item 26)

The composition of any of the preceding items for administration to a subject with a serum erythropoietin concentration of several mIU/mL or greater (e.g., 3 mIU/mL or greater).

### (Item 27)

The composition of any of the preceding items for administration to a subject with a hemoglobin concentration of 13 g/dL or less.

### (Item 28)

The composition of any of the preceding items for administration in combination with an anticancer agent.

### (Item 29)

The composition of any of the preceding items, wherein the anticancer agent comprises an angiogenesis inhibitor.

### (Item 30)

The composition of any of the preceding items, wherein the anticancer agent comprises bevacizumab, tamoxifen, doxorubicin, or trastuzumab.

### (Item 31)

The composition of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N (CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N (CH₃)₂, =O, -COOH, -OCH₃, and -OCH₂CH₃.

### (Item 32)

The composition of any of the preceding items, satisfying at least one of the following conditions:
A² is Gly,
A³ is Phe,
A⁵ is Thr,
A⁷ is Cys, and
A⁸ is Lys.

### (Item 33)

The composition of any of the preceding items, wherein A¹ is Tyr, p-fluorophenylalanine, or phenethylglycine.

### (Item 34)

The composition of any of the preceding items, wherein A¹ is Tyr.

### (Item 35)

The composition of any of the preceding items, wherein A⁴ is Leu or Met.

### (Item 36)

The composition of any of the preceding items, wherein A⁴ is Met.

### (Item 37)

The composition of any of the preceding items, wherein A⁶ is Trp, Met, 8-naphthylalanine, 2-quinolylalanine, 5-chloro-Trp, or 2-benzothiazolylalanine.

### (Item 38)

The composition of any of the preceding items, wherein A⁶ is 2-benzothiazolylalanine.

### (Item 39)

The composition of any of the preceding items, wherein
A² is Gly,
A³ is Phe,
A⁵ is Thr,
A⁷ is Cys, and
A⁸ is Lys.

### (Item 40)

The composition of any of the preceding items, wherein X¹ is -COOH, a benzoyl group, a propionyl group, or a p-fluorophenylacetyl group.

### (Item 41)

The composition of any of the preceding items, wherein X² is NH₂.

### (Item 42)

The composition of any of the preceding items, comprising a pharmaceutically acceptable carrier.

### (Item 43)

A method of selectively antagonizing an erythropoietin (Epo) heteroreceptor in the presence of Epo, comprising administering a therapeutically effective amount of a peptide having a structure of -[SCHFGPLTWVCK]-, or a modified peptide based on said peptide, or a prodrug thereof, or a salt or solvate thereof (an alphabet indicates a one letter code of an amino acid) to a subject.

### (Item 44)

The method of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item 45)

The method of any of the preceding items, satisfying at least one of the following conditions:
A² is Gly,
A³ is Phe,
A⁵ is Thr,
A⁷ is Cys, and
A⁸ is Lys.

### (Item 46)

The method of any of the preceding items, wherein A¹ is Tyr, p-fluorophenylalanine, or phenethylglycine.

### (Item 47)

The method of any of the preceding items, wherein A¹ is Tyr.

### (Item 48)

The method of any of the preceding items, wherein A⁴ is Leu or Met.

### (Item 49)

The method of any of the preceding items, wherein A⁴ is Met.

### (Item 50)

The method of any of the preceding items, wherein A⁶ is Trp, Met, 8-naphthylalanine, 2-quinolylalanine, 5-chloro-Trp, or 2-benzothiazolylalanine.

### (Item 51)

The method of any of the preceding items, wherein A⁶ is 2-benzothiazolylalanine.

### (Item 52)

The method of any of the preceding items, wherein
A² is Gly,
A³ is Phe,
A⁵ is Thr,
A⁷ is Cys, and
A⁸ is Lys.

### (Item 53)

The method of any of the preceding items, wherein X¹ is -COOH, a benzoyl group, a propionyl group, or a p-fluorophenylacetyl group.

### (Item 54)

The method of any of the preceding items, wherein X² is NH₂.

### (Item 55)

The method of any of the preceding items, wherein the peptide, or the modified peptide, or the prodrug thereof, or the salt or solvate thereof has substantially no affinity to an Epo homodimer receptor in the presence of Epo.

### (Item 56)

The method of any of the preceding items, wherein the peptide, or the modified peptide, or the prodrug thereof, or the salt or solvate thereof has affinity to an Epo homodimer receptor in the absence of Epo.

### (Item 57)

The method of any of the preceding items, wherein the Epo heteroreceptor comprises an Epo receptor and a βc receptor.

### (Item 58)

The method of any of the preceding items, wherein the peptide, or the modified peptide based on said peptide, or the prodrug thereof, or the salt or solvate thereof, in the presence of Epo, does not antagonize an Epo homodimer receptor, but antagonizes an Epo heteroreceptor comprising an Epo receptor and a βc receptor.

### (Item 59)

The method of any of the preceding items for administration in combination with an anticancer agent.

### (Item 60)

The method of any of the preceding items for improving an adverse action elicited by an anticancer agent in the presence of Epo.

### (Item 61)

The method of any of the preceding items, wherein the anticancer agent comprises an angiogenesis inhibitor.

### (Item 62)

The method of any of the preceding items, wherein the anticancer agent comprises bevacizumab, tamoxifen, doxorubicin, or trastuzumab.

### (Item 63)

A method of treating or preventing a disease, disorder, or symptom characterized by the presence of a cell expressing an Epo heteroreceptor, comprising administering a therapeutically effective amount of a selective antagonist of the Epo heteroreceptor to a subject.

### (Item 64)

A method of treating or preventing a disease, disorder, or symptom characterized by the presence of a cell expressing an Epo heteroreceptor, comprising administering a therapeutically effective amount of a peptide having a structure of -[SCHFGPLTWVCK]-, or a modified peptide based on said peptide, or a prodrug thereof, or a salt or solvate thereof (an alphabet indicates a one letter code of an amino acid) to a subject.

### (Item 65)

The method of any of the preceding items, which does not induce anemia.

### (Item 66)

The method of any of the preceding items, wherein the disease is a proliferative disease, rheumatism, diabetic retinopathy, keloid, or adenomyosis.

### (Item 67)

The method of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item 68)

The method of any of the preceding items, wherein the subject has a serum erythropoietin concentration of several mIU/mL or greater (e.g., 3 mIU/mL or greater).

### (Item 69)

The method of any of the preceding items, wherein the subject has a hemoglobin concentration of 13 g/dL or less.

### (Item 70)

The method of any of the preceding items, further comprising administering an anticancer agent to the subject.

### (Item 71)

The method of any of the preceding items, wherein the anticancer agent comprises an angiogenesis inhibitor.

### (Item 72)

The method of any of the preceding items, wherein the anticancer agent comprises bevacizumab, tamoxifen, doxorubicin, or trastuzumab.

### (Item 73)

The method of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item 74)

The method of any of the preceding items, satisfying at least one of the following conditions:
A² is Gly,
A³ is Phe,
A⁵ is Thr,
A⁷ is Cys, and
A⁸ is Lys.

### (Item 75)

The method of any of the preceding items, wherein A¹ is Tyr, p-fluorophenylalanine, or phenethylglycine.

### (Item 76)

The method of any of the preceding items, wherein A¹ is Tyr.

### (Item 77)

The method of any of the preceding items, wherein A⁴ is Leu or Met.

### (Item 78)

The method of any of the preceding items, wherein A⁴ is Met.

### (Item 79)

The method of any of the preceding items, wherein A⁶ is Trp, Met, 8-naphthylalanine, 2-quinolylalanine, 5-chloro-Trp, or 2-benzothiazolylalanine.

### (Item 80)

The method of any of the preceding items, wherein A⁶ is 2-benzothiazolylalanine.

### (Item 81)

The method of any of the preceding items, wherein
A² is Gly,
A³ is Phe,
A⁵ is Thr,
A⁷ is Cys, and
A⁸ is Lys.

### (Item 82)

The method of any of the preceding items, wherein X¹ is -COOH, a benzoyl group, a propionyl group, or a p-fluorophenylacetyl group.

### (Item 83)

The method of any of the preceding items, wherein X² is NH₂.

### (Item 84)

A peptide having a structure of -[SCHFGPLTWVCK]-, or a modified peptide based on said peptide, or a prodrug thereof, or a salt or solvate thereof (an alphabet indicates a one letter code of an amino acid) for selectively antagonizing an erythropoietin (Epo) heteroreceptor in the presence of Epo.

### (Item 85)

The peptide, or the modified peptide based on said peptide, or the prodrug thereof, or the salt or solvate thereof of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH (R^{A1}) -CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item 86)

The peptide, or the modified peptide based on said peptide, or the prodrug thereof, or the salt or solvate thereof of any of the preceding items, satisfying at least one of the following conditions:
A² is Gly,
A³ is Phe,
A⁵ is Thr,
A⁷ is Cys, and
A⁸ is Lys.

### (Item 87)

The peptide, or the modified peptide based on said peptide, or the prodrug thereof, or the salt or solvate thereof of any of the preceding items, wherein A¹ is Tyr, p-fluorophenylalanine, or phenethylglycine.

### (Item 88)

The peptide, or the modified peptide based on said peptide, or the prodrug thereof, or the salt or solvate thereof of any of the preceding items, wherein A¹ is Tyr.

### (Item 89)

The peptide, or the modified peptide based on said peptide, or the prodrug thereof, or the salt or solvate thereof of any of the preceding items, wherein A⁴ is Leu or Met.

### (Item 90)

The peptide, or the modified peptide based on said peptide, or the prodrug thereof, or the salt or solvate thereof of any of the preceding items, wherein A⁴ is Met.

### (Item 91)

The peptide, or the modified peptide based on said peptide, or the prodrug thereof, or the salt or solvate thereof of any of the preceding items, wherein A⁶ is Trp, Met, 8-naphthylalanine, 2-quinolylalanine, 5-chloro-Trp, or 2-benzothiazolylalanine.

### (Item 92)

The peptide, or the modified peptide based on said peptide, or the prodrug thereof, or the salt or solvate thereof of any of the preceding items, wherein A⁶ is 2-benzothiazolylalanine.

### (Item 93)

The peptide, or the modified peptide based on said peptide, or the prodrug thereof, or the salt or solvate thereof of any of the preceding items, wherein
A² is Gly,
A³ is Phe,
A⁵ is Thr,
A⁷ is Cys, and
A⁸ is Lys.

### (Item 94)

The peptide, or the modified peptide based on said peptide, or the prodrug thereof, or the salt or solvate thereof of any of the preceding items, wherein X¹ is -COOH, a benzoyl group, a propionyl group, or a p-fluorophenylacetyl group.

### (Item 95)

The peptide, or the modified peptide based on said peptide, or the prodrug thereof, or the salt or solvate thereof of any of the preceding items, wherein X² is NH₂.

### (Item 96)

The peptide, or the modified peptide based on said peptide, or the prodrug thereof, or the salt or solvate thereof of any of the preceding items, which has substantially no affinity to an Epo homodimer receptor in the presence of Epo.

### (Item 97)

The peptide, or the modified peptide based on said peptide, or the prodrug thereof, or the salt or solvate thereof of any of the preceding items, which has affinity to an Epo homodimer receptor in the absence of Epo.

### (Item 98)

The peptide, or the modified peptide based on said peptide, or the prodrug thereof, or the salt or solvate thereof of any of the preceding items, wherein the Epo heteroreceptor comprises an Epo receptor and a βc receptor.

### (Item 99)

The peptide, or the modified peptide based on said peptide, or the prodrug thereof, or the salt or solvate thereof of any of the preceding items, which, in the presence of Epo, does not antagonize an Epo homodimer receptor, but antagonizes an Epo heteroreceptor comprising an Epo receptor and a βc receptor.

### (Item 100)

The peptide, or the modified peptide based on said peptide, or the prodrug thereof, or the salt or solvate thereof of any of the preceding items for administration in combination with an anticancer agent.

### (Item 101)

The peptide, or the modified peptide based on said peptide, or the prodrug thereof, or the salt or solvate thereof of any of the preceding items for improving an adverse action elicited by an anticancer agent in the presence of Epo.

### (Item 102)

The peptide, or the modified peptide based on said peptide, or the prodrug thereof, or the salt or solvate thereof of any of the preceding items, wherein the anticancer agent comprises an angiogenesis inhibitor.

### (Item 103)

The peptide, or the modified peptide based on said peptide, or the prodrug thereof, or the salt or solvate thereof of any of the preceding items, wherein the anticancer agent comprises bevacizumab, tamoxifen, doxorubicin, or trastuzumab.

### (Item 104)

A selective antagonist of an Epo heteroreceptor for treating or preventing a disease, disorder, or symptom characterized by the presence of a cell expressing an Epo heteroreceptor.

### (Item 105)

A peptide having a structure of -[SCHFGPLTWVCK]-, or a modified peptide based on said peptide, or a prodrug thereof, or a salt or solvate thereof (an alphabet indicates a one letter code of an amino acid) for treating or preventing a disease, disorder, or symptom characterized by the presence of a cell expressing an Epo heteroreceptor.

### (Item 106)

The selective antagonist, the peptide, or the modified peptide based on said peptide, or the prodrug thereof, or the salt or solvate thereof of any of the preceding items, wherein administration of the composition does not induce anemia.

### (Item 107)

The selective antagonist, the peptide, or the modified peptide based on said peptide, or the prodrug thereof, or the salt or solvate thereof of any of the preceding items, wherein the disease is a proliferative disease, rheumatism, diabetic retinopathy, keloid, or adenomyosis.

### (Item 108)

The selective antagonist, the peptide, or the modified peptide based on said peptide, or the prodrug thereof, or the salt or solvate thereof of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item 109)

The selective antagonist, the peptide, or the modified peptide based on said peptide, or the prodrug thereof, or the salt or solvate thereof of any of the preceding items for administration to a subject with a serum erythropoietin concentration of several mIU/mL or greater (e.g., 3 mIU/mL or greater).

### (Item 110)

The selective antagonist, the peptide, or the modified peptide based on said peptide, or the prodrug thereof, or the salt or solvate thereof of any of the preceding items for administration to a subject with a hemoglobin concentration of 13 g/dL or less.

### (Item 111)

The selective antagonist, the peptide, or the modified peptide based on said peptide, or the prodrug thereof, or the salt or solvate thereof of any of the preceding items for administration in combination with an anticancer agent.

### (Item 112)

The selective antagonist, the peptide, or the modified peptide based on said peptide, or the prodrug thereof, or the salt or solvate thereof of any of the preceding items, wherein the anticancer agent comprises an angiogenesis inhibitor.

### (Item 113)

The selective antagonist, the peptide, or the modified peptide based on said peptide, or the prodrug thereof, or the salt or solvate thereof of any of the preceding items, wherein the anticancer agent comprises bevacizumab, tamoxifen, doxorubicin, or trastuzumab.

### (Item 114)

The selective antagonist, the peptide, or the modified peptide based on said peptide, or the prodrug thereof, or the salt or solvate thereof of any of the preceding items, wherein the modified peptide has a structure of formula (I) :

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item 115)

The selective antagonist, the peptide, or the modified peptide based on said peptide, or the prodrug thereof, or the salt or solvate thereof of any of the preceding items, satisfying at least one of the following conditions:
A² is Gly,
A³ is Phe,
A⁵ is Thr,
A⁷ is Cys, and
A⁸ is Lys.

### (Item 116)

The selective antagonist, the peptide, or the modified peptide based on said peptide, or the prodrug thereof, or the salt or solvate thereof of any of the preceding items, wherein A¹ is Tyr, p-fluorophenylalanine, or phenethylglycine.

### (Item 117)

The selective antagonist, the peptide, or the modified peptide based on said peptide, or the prodrug thereof, or the salt or solvate thereof of any of the preceding items, wherein A¹ is Tyr.

### (Item 118)

The selective antagonist, the peptide, or the modified peptide based on said peptide, or the prodrug thereof, or the salt or solvate thereof of any of the preceding items, wherein A⁴ is Leu or Met.

### (Item 119)

The selective antagonist, the peptide, or the modified peptide based on said peptide, or the prodrug thereof, or the salt or solvate thereof of any of the preceding items, wherein A⁴ is Met.

### (Item 120)

The selective antagonist, the peptide, or the modified peptide based on said peptide, or the prodrug thereof, or the salt or solvate thereof of any of the preceding items, wherein A⁶ is Trp, Met, 8-naphthylalanine, 2-quinolylalanine, 5-chloro-Trp, or 2-benzothiazolylalanine.

### (Item 121)

The selective antagonist, the peptide, or the modified peptide based on said peptide, or the prodrug thereof, or the salt or solvate thereof of any of the preceding items, wherein A⁶ is 2-benzothiazolylalanine.

### (Item 122)

The selective antagonist, the peptide, or the modified peptide based on said peptide, or the prodrug thereof, or the salt or solvate thereof of any of the preceding items, wherein
A² is Gly,
A³ is Phe,
A⁵ is Thr,
A⁷ is Cys, and
A⁸ is Lys.

### (Item 123)

The selective antagonist, the peptide, or the modified peptide based on said peptide, or the prodrug thereof, or the salt or solvate thereof of any of the preceding items, wherein X¹ is -COOH, a benzoyl group, a propionyl group, or a p-fluorophenylacetyl group.

### (Item 124)

The selective antagonist, the peptide, or the modified peptide based on said peptide, or the prodrug thereof, or the salt or solvate thereof of any of the preceding items, wherein X² is NH₂.

### (Item 125)

Use of a peptide having a structure of -[SCHFGPLTWVCK]-, or a modified peptide based on said peptide, or a prodrug thereof, or a salt or solvate thereof (an alphabet indicates a one letter code of an amino acid) in the manufacture of a medicament for selectively antagonizing an erythropoietin (Epo) heteroreceptor in the presence of Epo.

### (Item 126)

The use of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):
   X¹-kcvwtl(B¹)Gfhcs-X²
   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item 127)

The use of any of the preceding items, satisfying at least one of the following conditions:
A² is Gly,
A³ is Phe,
A⁵ is Thr,
A⁷ is Cys, and
A⁸ is Lys.

### (Item 128)

The use of any of the preceding items, wherein A¹ is Tyr, p-fluorophenylalanine, or phenethylglycine.

### (Item 129)

The use of any of the preceding items, wherein A¹ is Tyr.

### (Item 130)

The use of any of the preceding items, wherein A⁴ is Leu or Met.

### (Item 131)

The use of any of the preceding items, wherein A⁴ is Met.

### (Item 132)

The use of any of the preceding items, wherein A⁶ is Trp, Met, 8-naphthylalanine, 2-quinolylalanine, 5-chloro-Trp, or 2-benzothiazolylalanine.

### (Item 133)

The use of any of the preceding items, wherein A⁶ is 2-benzothiazolylalanine.

### (Item 134)

The use of any of the preceding items, wherein
A² is Gly,
A³ is Phe,
A⁵ is Thr,
A⁷ is Cys, and
A⁸ is Lys.

### (Item 135)

The use of any of the preceding items, wherein X¹ is - COOH, a benzoyl group, a propionyl group, or a p-fluorophenylacetyl group.

### (Item 136)

The use of any of the preceding items, wherein X² is NH₂.

### (Item 137)

The use of any of the preceding items, wherein the peptide, or the modified peptide, or the prodrug thereof, or the salt or solvate thereof has substantially no affinity to an Epo homodimer receptor in the presence of Epo.

### (Item 138)

The use of any of the preceding items, wherein the peptide, or the modified peptide, or the prodrug thereof, or the salt or solvate thereof has affinity to an Epo homodimer receptor in the absence of Epo.

### (Item 139)

The use of any of the preceding items, wherein the Epo heteroreceptor comprises an Epo receptor and a βc receptor.

### (Item 140)

The use of any of the preceding items, wherein the peptide, or the modified peptide based on said peptide, or the prodrug thereof, or the salt or solvate thereof, in the presence of Epo, does not antagonize an Epo homodimer receptor, but antagonizes an Epo heteroreceptor comprising an Epo receptor and a βc receptor.

### (Item 141)

The use of any of the preceding items, wherein the medicament is for administration in combination with an anticancer agent.

### (Item 142)

The use of any of the preceding items, wherein the medicament improves an adverse action elicited by an anticancer agent in the presence of Epo.

### (Item 143)

The use of any of the preceding items, wherein the anticancer agent comprises an angiogenesis inhibitor.

### (Item 144)

The use of any of the preceding items, wherein the anticancer agent comprises bevacizumab, tamoxifen, doxorubicin, or trastuzumab.

### (Item 145)

Use of a selective antagonist of an Epo heteroreceptor in the manufacture of a medicament for treating or preventing a disease, disorder, or symptom characterized by the presence of a cell expressing an Epo heteroreceptor.

### (Item 146)

Use of a peptide having a structure of -[SCHFGPLTWVCK]-, or a modified peptide based on said peptide, or a prodrug thereof, or a salt or solvate thereof (an alphabet indicates a one letter code of an amino acid) in the manufacture of a medicament for treating or preventing a disease, disorder, or symptom characterized by the presence of a cell expressing an Epo heteroreceptor.

### (Item 147)

The use of any of the preceding items, wherein the medicament does not induce anemia.

### (Item 148)

The use of any of the preceding items, wherein the disease is a proliferative disease, rheumatism, diabetic retinopathy, keloid, or adenomyosis.

### (Item 149)

The use of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item 150)

The use of any of the preceding items, wherein the medicament is for administration to a subject with a serum erythropoietin concentration of several mIU/mL or greater (e.g., 3 mIU/mL or greater).

### (Item 151)

The use of any of the preceding items, wherein the medicament is for administration to a subject with a hemoglobin concentration of 13 g/dL or less.

### (Item 152)

The use of any of the preceding items, wherein the medicament is for administration in combination with an anticancer agent.

### (Item 153)

The use of any of the preceding items, wherein the anticancer agent comprises an angiogenesis inhibitor.

### (Item 154)

The use of any of the preceding items, wherein the anticancer agent comprises bevacizumab, tamoxifen, doxorubicin, or trastuzumab.

### (Item 155)

The use of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide:
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item 156)

The use of any of the preceding items, satisfying at least one of the following conditions:
A² is Gly,
A³ is Phe,
A⁵ is Thr,
A⁷ is Cys, and
A⁸ is Lys.

### (Item 157)

The use of any of the preceding items, wherein A¹ is Tyr, p-fluorophenylalanine, or phenethylglycine.

### (Item 158)

The use of any of the preceding items, wherein A¹ is Tyr.

### (Item 159)

The use of any of the preceding items, wherein A⁴ is Leu or Met.

### (Item 160)

The use of any of the preceding items, wherein A⁴ is Met.

### (Item 161)

The use of any of the preceding items, wherein A⁶ is Trp, Met, 8-naphthylalanine, 2-quinolylalanine, 5-chloro-Trp, or 2-benzothiazolylalanine.

### (Item 162)

The use of any of the preceding items, wherein A⁶ is 2-benzothiazolylalanine.

### (Item 163)

The use of any of the preceding items, wherein
A² is Gly,
A³ is Phe,
A⁵ is Thr,
A⁷ is Cys, and
A⁸ is Lys.

### (Item 164)

The use of any of the preceding items, wherein X¹ is - COOH, a benzoyl group, a propionyl group, or a p-fluorophenylacetyl group.

### (Item 165)

The use of any of the preceding items, wherein X² is NH₂.

### (Item X1)

A composition for use in selectively antagonizing an erythropoietin (Epo) heteroreceptor in the presence of Epo, comprising a peptide having a structure of -[SCHFGPLTWVCK]-, or a modified peptide based on said peptide, or a prodrug thereof, or a salt or solvate thereof (an alphabet indicates a one letter code of an amino acid).

### (Item X2)

The composition of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH (R^{A1}) -CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X3)

The composition of any of the preceding items, satisfying at least one of the following conditions:
A² is Gly,
A³ is Phe,
A⁵ is Thr,
A⁷ is Cys, and
A⁸ is Lys.

### (Item X4)

The composition of any of the preceding items, wherein A¹ is Tyr, p-fluorophenylalanine, or phenethylglycine.

### (Item X5)

The composition of any of the preceding items, wherein A¹ is Tyr.

### (Item X6)

The composition of any of the preceding items, wherein A⁴ is Leu or Met.

### (Item X7)

The composition of any of the preceding items, wherein A⁴ is Met.

### (Item X8)

The composition of any of the preceding items, wherein A⁶ is Trp, Met, 8-naphthylalanine, 2-quinolylalanine, 5-chloro-Trp, or 2-benzothiazolylalanine.

### (Item X9)

The composition of any of the preceding items, wherein A⁶ is 2-benzothiazolylalanine.

### (Item X10)

The composition of any of the preceding items, wherein
A² is Gly,
A³ is Phe,
A⁵ is Thr,
A⁷ is Cys, and
A⁸ is Lys.

### (Item X11)

The composition of any of the preceding items, wherein X¹ is -COOH, a benzoyl group, a propionyl group, or a p-fluorophenylacetyl group.

### (Item X12)

The composition of any of the preceding items, wherein X² is NH₂.

### (Item X13)

The composition of any of the preceding items, wherein the peptide, or the modified peptide, or the prodrug thereof, or the salt or solvate thereof has substantially no affinity to an Epo homodimer receptor in the presence of Epo.

### (Item X14)

The composition of any of the preceding items, wherein the peptide, or the modified peptide, or the prodrug thereof, or the salt or solvate thereof has affinity to an Epo homodimer receptor in the absence of Epo.

### (Item X15)

The composition of any of the preceding items, wherein the Epo heteroreceptor comprises an Epo receptor and a βc receptor.

### (Item X16)

The composition of any of the preceding items, wherein the peptide, or the modified peptide based on said peptide, or the prodrug thereof, or the salt or solvate thereof, in the presence of Epo, does not antagonize an Epo homodimer receptor, but antagonizes an Epo heteroreceptor comprising an Epo receptor and a βc receptor.

### (Item X17)

The composition of any of the preceding items, wherein the peptide, or the modified peptide based on said peptide, or the prodrug thereof, or the salt or solvate thereof selectively binds to a low affinity binding region of an EpoR.

### (Item X18)

The composition of any of the preceding items for administration in combination with an anticancer agent.

### (Item X19)

The composition of any of the preceding items for improving an adverse action elicited by an anticancer agent in the presence of Epo.

### (Item X20)

The composition of any of the preceding items, wherein the anticancer agent comprises an angiogenesis inhibitor.

### (Item X21)

The composition of any of the preceding items, wherein the anticancer agent comprises bevacizumab, tamoxifen, doxorubicin, or trastuzumab.

### (Item X22)

A composition for use in treating or preventing a disease, disorder, or symptom characterized by the presence of a cell expressing an Epo heteroreceptor, comprising a selective antagonist of an Epo heteroreceptor.

### (Item X23)

A composition for use in treating or preventing a disease, disorder, or symptom characterized by the presence of a cell expressing an Epo heteroreceptor, comprising a peptide having a structure of -[SCHFGPLTWVCK]-, or a modified peptide based on said peptide, or a prodrug thereof, or a salt or solvate thereof (an alphabet indicates a one letter code of an amino acid).

### (Item X24)

The composition of any of the preceding items, wherein administration of the composition does not induce anemia.

### (Item X25)

The composition of any of the preceding items, wherein the disease is a proliferative disease, rheumatism, diabetic retinopathy, keloid, or adenomyosis.

### (Item X26)

The composition of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item X27)

The composition of any of the preceding items for administration to a subject with a serum erythropoietin concentration of several mIU/mL or greater (e.g., 3 mIU/mL or greater).

### (Item X28)

The composition of any of the preceding items for administration to a subject with a hemoglobin concentration of 13 g/dL or less.

### (Item X29)

The composition of any of the preceding items for administration in combination with an anticancer agent.

### (Item X30)

The composition of any of the preceding items, wherein the anticancer agent comprises an angiogenesis inhibitor.

### (Item X31)

The composition of any of the preceding items, wherein the anticancer agent comprises bevacizumab, tamoxifen, doxorubicin, or trastuzumab.

### (Item X32)

The composition of any of the preceding items, wherein the modified peptide has a structure of formula (I):

X¹-SCH(A¹)(A²)(A³)(A⁴)(A⁵)(A⁶)V(A⁷)(A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, 1, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X33)

The composition of any of the preceding items, satisfying at least one of the following conditions:
A² is Gly,
A³ is Phe,
A⁵ is Thr,
A⁷ is Cys, and
A⁸ is Lys.

### (Item X34)

The composition of any of the preceding items, wherein A¹ is Tyr, p-fluorophenylalanine, or phenethylglycine.

### (Item X35)

The composition of any of the preceding items, wherein A¹ is Tyr.

### (Item X36)

The composition of any of the preceding items, wherein A⁴ is Leu or Met.

### (Item X37)

The composition of any of the preceding items, wherein A⁴ is Met.

### (Item X38)

The composition of any of the preceding items, wherein A⁶ is Trp, Met, 8-naphthylalanine, 2-quinolylalanine, 5-chloro-Trp, or 2-benzothiazolylalanine.

### (Item X39)

The composition of any of the preceding items, wherein A⁶ is 2-benzothiazolylalanine.

### (Item X40)

The composition of any of the preceding items, wherein
A² is Gly,
A³ is Phe,
A⁵ is Thr,
A⁷ is Cys, and
A⁸ is Lys.

### (Item X41)

The composition of any of the preceding items, wherein X¹ is -COOH, a benzoyl group, a propionyl group, or a p-fluorophenylacetyl group.

### (Item X42)

The composition of any of the preceding items, wherein X² is NH₂.

### (Item X43)

The composition of any of the preceding items, comprising a pharmaceutically acceptable carrier.

### (Item X44)

A agent for use in detecting an Epo heteroreceptor comprising an Epo receptor and a βc receptor.

### (Item X45)

A method of selectively antagonizing an erythropoietin (Epo) heteroreceptor in the presence of Epo, comprising administering a therapeutically effective amount of a peptide having a structure of -[SCHFGPLTWVCK]-, or a modified peptide based on said peptide, or a prodrug thereof, or a salt or solvate thereof (an alphabet indicates a one letter code of an amino acid) to a subject.

### (Item X46)

The method of any of the preceding items, wherein the modified peptide has a structure of formula (I):

X¹-SCH(A¹) (A²) (A³) (A⁴) (A⁵) (A⁶)V(A⁷) (A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, l, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X47)

The method of any of the preceding items, satisfying at least one of the following conditions:
A² is Gly,
A³ is Phe,
A⁵ is Thr,
A⁷ is Cys, and
A⁸ is Lys.

### (Item X48)

The method of any of the preceding items, wherein A¹ is Tyr, p-fluorophenylalanine, or phenethylglycine.

### (Item X49)

The method of any of the preceding items, wherein A¹ is Tyr.

### (Item X50)

The method of any of the preceding items, wherein A⁴ is Leu or Met.

### (Item X51)

The method of any of the preceding items, wherein A⁴ is Met.

### (Item X52)

The method of any of the preceding items, wherein A⁶ is Trp, Met, 8-naphthylalanine, 2-quinolylalanine, 5-chloro-Trp, or 2-benzothiazolylalanine.

### (Item X53)

The method of any of the preceding items, wherein A⁶ is 2-benzothiazolylalanine.

### (Item X54)

The method of any of the preceding items, wherein
A² is Gly,
A³ is Phe,
A⁵ is Thr,
A⁷ is Cys, and
A⁸ is Lys.

### (Item X55)

The method of any of the preceding items, wherein X¹ is -COOH, a benzoyl group, a propionyl group, or a p-fluorophenylacetyl group.

### (Item X56)

The method of any of the preceding items, wherein X² is NH₂.

### (Item X57)

The method of any of the preceding items, wherein the peptide, or the modified peptide, or the prodrug thereof, or the salt or solvate thereof has substantially no affinity to an Epo homodimer receptor in the presence of Epo.

### (Item X58)

The method of any of the preceding items, wherein the peptide, or the modified peptide, or the prodrug thereof, or the salt or solvate thereof has affinity to an Epo homodimer receptor in the absence of Epo.

### (Item X59)

The method of any of the preceding items, wherein the Epo heteroreceptor comprises an Epo receptor and a βc receptor.

### (Item X60)

The method of any of the preceding items, wherein the peptide, or the modified peptide based on said peptide, or the prodrug thereof, or the salt or solvate thereof, in the presence of Epo, does not antagonize an Epo homodimer receptor, but antagonizes an Epo heteroreceptor comprising an Epo receptor and a βc receptor.

### (Item X61)

The method of any of the preceding items, wherein the peptide, or the modified peptide based on said peptide, or the prodrug thereof, or the salt or solvate thereof selectively binds to a low affinity binding region of an EpoR.

### (Item X62)

The method of any of the preceding items for administration in combination with an anticancer agent.

### (Item X63)

The method of any of the preceding items for improving an adverse action elicited by an anticancer agent in the presence of Epo.

### (Item X64)

The method of any of the preceding items, wherein the anticancer agent comprises an angiogenesis inhibitor.

### (Item X65)

The method of any of the preceding items, wherein the anticancer agent comprises bevacizumab, tamoxifen, doxorubicin, or trastuzumab.

### (Item X66)

A method of treating or preventing a disease, disorder, or symptom characterized by the presence of a cell expressing an Epo heteroreceptor, comprising administering a therapeutically effective amount of a selective antagonist of the Epo heteroreceptor to a subject.

### (Item X67)

A method of treating or preventing a disease, disorder, or symptom characterized by the presence of a cell expressing an Epo heteroreceptor, comprising administering a therapeutically effective amount of a peptide having a structure of -[SCHFGPLTWVCK]-, or a modified peptide based on said peptide, or a prodrug thereof, or a salt or solvate thereof (an alphabet indicates a one letter code of an amino acid) to a subject.

### (Item X68)

The method of any of the preceding items, which does not induce anemia.

### (Item X69)

The method of any of the preceding items, wherein the disease is a proliferative disease, rheumatism, diabetic retinopathy, keloid, or adenomyosis.

### (Item X70)

The method of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item X71)

The method of any of the preceding items, wherein the subject has a serum erythropoietin concentration of several mIU/mL or greater (e.g., 3 mIU/mL or greater).

### (Item X72)

The method of any of the preceding items, wherein the subject has a hemoglobin concentration of 13 g/dL or less.

### (Item X73)

The method of any of the preceding items, further comprising administering an anticancer agent to the subject.

### (Item X74)

The method of any of the preceding items, wherein the anticancer agent comprises an angiogenesis inhibitor.

### (Item X75)

The method of any of the preceding items, wherein the anticancer agent comprises bevacizumab, tamoxifen, doxorubicin, or trastuzumab.

### (Item X76)

The method of any of the preceding items, wherein the modified peptide has a structure of formula (I):

X¹-SCH(A¹) (A²) (A³) (A⁴) (A⁵) (A⁶)V(A⁷) (A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, l, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X77)

The method of any of the preceding items, satisfying at least one of the following conditions:
A² is Gly,
A³ is Phe,
A⁵ is Thr,
A⁷ is Cys, and
A⁸ is Lys.

### (Item X78)

The method of any of the preceding items, wherein A¹ is Tyr, p-fluorophenylalanine, or phenethylglycine.

### (Item X79)

The method of any of the preceding items, wherein A¹ is Tyr.

### (Item X80)

The method of any of the preceding items, wherein A⁴ is Leu or Met.

### (Item X81)

The method of any of the preceding items, wherein A⁴ is Met.

### (Item X82)

The method of any of the preceding items, wherein A⁶ is Trp, Met, 8-naphthylalanine, 2-quinolylalanine, 5-chloro-Trp, or 2-benzothiazolylalanine.

### (Item X83)

The method of any of the preceding items, wherein A⁶ is 2-benzothiazolylalanine.

### (Item X84)

The method of any of the preceding items, wherein
A² is Gly,
A³ is Phe,
A⁵ is Thr,
A⁷ is Cys, and
A⁸ is Lys.

### (Item X85)

The method of any of the preceding items, wherein X¹ is -COOH, a benzoyl group, a propionyl group, or a p-fluorophenylacetyl group.

### (Item X86)

The method of any of the preceding items, wherein X² is NH₂.

### (Item X87)

A method of detecting an Epo heteroreceptor, comprising administering a detecting agent comprising an Epo receptor and a βc receptor to a subject.

### (Item X88)

A peptide having a structure of -[SCHFGPLTWVCK]-, or a modified peptide based on said peptide, or a prodrug thereof, or a salt or solvate thereof (an alphabet indicates a one letter code of an amino acid) for selectively antagonizing an erythropoietin (Epo) heteroreceptor in the presence of Epo.

### (Item X89)

The peptide, or the modified peptide based on said peptide, or the prodrug thereof, or the salt or solvate thereof of any of the preceding items, wherein the modified peptide has a structure of formula (I):

X¹-SCH(A¹) (A²) (A³) (A⁴) (A⁵) (A⁶)V(A⁷) (A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, l, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X90)

The peptide, or the modified peptide based on said peptide, or the prodrug thereof, or the salt or solvate thereof of any of the preceding items, satisfying at least one of the following conditions:
A² is Gly,
A³ is Phe,
A⁵ is Thr,
A⁷ is Cys, and
A⁸ is Lys.

### (Item X91)

The peptide, or the modified peptide based on said peptide, or the prodrug thereof, or the salt or solvate thereof of any of the preceding items, wherein A¹ is Tyr, p-fluorophenylalanine, or phenethylglycine.

### (Item X92)

The peptide, or the modified peptide based on said peptide, or the prodrug thereof, or the salt or solvate thereof of any of the preceding items, wherein A¹ is Tyr.

### (Item X93)

The peptide, or the modified peptide based on said peptide, or the prodrug thereof, or the salt or solvate thereof of any of the preceding items, wherein A⁴ is Leu or Met.

### (Item X94)

The peptide, or the modified peptide based on said peptide, or the prodrug thereof, or the salt or solvate thereof of any of the preceding items, wherein A⁴ is Met.

### (Item X95)

The peptide, or the modified peptide based on said peptide, or the prodrug thereof, or the salt or solvate thereof of any of the preceding items, wherein A⁶ is Trp, Met, 8-naphthylalanine, 2-quinolylalanine, 5-chloro-Trp, or 2-benzothiazolylalanine.

### (Item X96)

The peptide, or the modified peptide based on said peptide, or the prodrug thereof, or the salt or solvate thereof of any of the preceding items, wherein A⁶ is 2-benzothiazolylalanine.

### (Item X97)

The peptide, or the modified peptide based on said peptide, or the prodrug thereof, or the salt or solvate thereof of any of the preceding items, wherein
A² is Gly,
A³ is Phe,
A⁵ is Thr,
A⁷ is Cys, and
A⁸ is Lys.

### (Item X98)

The peptide, or the modified peptide based on said peptide, or the prodrug thereof, or the salt or solvate thereof of any of the preceding items, wherein X¹ is -COOH, a benzoyl group, a propionyl group, or a p-fluorophenylacetyl group.

### (Item X99)

The peptide, or the modified peptide based on said peptide, or the prodrug thereof, or the salt or solvate thereof of any of the preceding items, wherein X² is NH₂.

### (Item X100)

The peptide, or the modified peptide based on said peptide, or the prodrug thereof, or the salt or solvate thereof of any of the preceding items, which has substantially no affinity to an Epo homodimer receptor in the presence of Epo.

### (Item X101)

The peptide, or the modified peptide based on said peptide, or the prodrug thereof, or the salt or solvate thereof of any of the preceding items, which has affinity to an Epo homodimer receptor in the absence of Epo.

### (Item X102)

The peptide, or the modified peptide based on said peptide, or the prodrug thereof, or the salt or solvate thereof of any of the preceding items, wherein the Epo heteroreceptor comprises an Epo receptor and a βc receptor.

### (Item X103)

The peptide, or the modified peptide based on said peptide, or the prodrug thereof, or the salt or solvate thereof of any of the preceding items, which, in the presence of Epo, does not antagonize an Epo homodimer receptor, but antagonizes an Epo heteroreceptor comprising an Epo receptor and a βc receptor.

### (Item X104)

The peptide, or the modified peptide based on said peptide, or the prodrug thereof, or the salt or solvate thereof of any of the preceding items, wherein the peptide, or the modified peptide based on said peptide, or the prodrug thereof, or the salt or solvate thereof selectively binds to a low affinity binding region of an EpoR.

### (Item X105)

The peptide, or the modified peptide based on said peptide, or the prodrug thereof, or the salt or solvate thereof of any of the preceding items for administration in combination with an anticancer agent.

### (Item X106)

The peptide, or the modified peptide based on said peptide, or the prodrug thereof, or the salt or solvate thereof of any of the preceding items for improving an adverse action elicited by an anticancer agent in the presence of Epo.

### (Item X107)

The peptide, or the modified peptide based on said peptide, or the prodrug thereof, or the salt or solvate thereof of any of the preceding items, wherein the anticancer agent comprises an angiogenesis inhibitor.

### (Item X108)

The peptide, or the modified peptide based on said peptide, or the prodrug thereof, or the salt or solvate thereof of any of the preceding items, wherein the anticancer agent comprises bevacizumab, tamoxifen, doxorubicin, or trastuzumab.

### (Item X109)

A selective antagonist of an Epo heteroreceptor for treating or preventing a disease, disorder, or symptom characterized by the presence of a cell expressing an Epo heteroreceptor.

### (Item X110)

A peptide having a structure of -[SCHFGPLTWVCK]-, or a modified peptide based on said peptide, or a prodrug thereof, or a salt or solvate thereof (an alphabet indicates a one letter code of an amino acid) for treating or preventing a disease, disorder, or symptom characterized by the presence of a cell expressing an Epo heteroreceptor.

### (Item X111)

The selective antagonist, the peptide, or the modified peptide based on said peptide, or the prodrug thereof, or the salt or solvate thereof of any of the preceding items, wherein administration of the composition does not induce anemia.

### (Item X112)

The selective antagonist, the peptide, or the modified peptide based on said peptide, or the prodrug thereof, or the salt or solvate thereof of any of the preceding items, wherein the disease is a proliferative disease, rheumatism, diabetic retinopathy, keloid, or adenomyosis.

### (Item X113)

The selective antagonist, the peptide, or the modified peptide based on said peptide, or the prodrug thereof, or the salt or solvate thereof of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item X114)

The selective antagonist, the peptide, or the modified peptide based on said peptide, or the prodrug thereof, or the salt or solvate thereof of any of the preceding items for administration to a subject with a serum erythropoietin concentration of several mIU/mL or greater (e.g., 3 mIU/mL or greater).

### (Item X115)

The selective antagonist, the peptide, or the modified peptide based on said peptide, or the prodrug thereof, or the salt or solvate thereof of any of the preceding items for administration to a subject with a hemoglobin concentration of 13 g/dL or less.

### (Item X116)

The selective antagonist, the peptide, or the modified peptide based on said peptide, or the prodrug thereof, or the salt or solvate thereof of any of the preceding items for administration in combination with an anticancer agent.

### (Item X117)

The selective antagonist, the peptide, or the modified peptide based on said peptide, or the prodrug thereof, or the salt or solvate thereof of any of the preceding items, wherein the anticancer agent comprises an angiogenesis inhibitor.

### (Item X118)

The selective antagonist, the peptide, or the modified peptide based on said peptide, or the prodrug thereof, or the salt or solvate thereof of any of the preceding items, wherein the anticancer agent comprises bevacizumab, tamoxifen, doxorubicin, or trastuzumab.

### (Item X119)

The selective antagonist, the peptide, or the modified peptide based on said peptide, or the prodrug thereof, or the salt or solvate thereof of any of the preceding items, wherein the modified peptide has a structure of formula (I) :

X¹-SCH(A¹) (A²) (A³) (A⁴) (A⁵) (A⁶)V(A⁷) (A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, l, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X120)

The selective antagonist, the peptide, or the modified peptide based on said peptide, or the prodrug thereof, or the salt or solvate thereof of any of the preceding items, satisfying at least one of the following conditions:
A² is Gly,
A³ is Phe,
A⁵ is Thr,
A⁷ is Cys, and
A⁸ is Lys.

### (Item X121)

The selective antagonist, the peptide, or the modified peptide based on said peptide, or the prodrug thereof, or the salt or solvate thereof of any of the preceding items, wherein A¹ is Tyr, p-fluorophenylalanine, or phenethylglycine.

### (Item X122)

The selective antagonist, the peptide, or the modified peptide based on said peptide, or the prodrug thereof, or the salt or solvate thereof of any of the preceding items, wherein A¹ is Tyr.

### (Item X123)

The selective antagonist, the peptide, or the modified peptide based on said peptide, or the prodrug thereof, or the salt or solvate thereof of any of the preceding items, wherein A⁴ is Leu or Met.

### (Item X124)

The selective antagonist, the peptide, or the modified peptide based on said peptide, or the prodrug thereof, or the salt or solvate thereof of any of the preceding items, wherein A⁴ is Met.

### (Item X125)

The selective antagonist, the peptide, or the modified peptide based on said peptide, or the prodrug thereof, or the salt or solvate thereof of any of the preceding items, wherein A⁶ is Trp, Met, 8-naphthylalanine, 2-quinolylalanine, 5-chloro-Trp, or 2-benzothiazolylalanine.

### (Item X126)

The selective antagonist, the peptide, or the modified peptide based on said peptide, or the prodrug thereof, or the salt or solvate thereof of any of the preceding items, wherein A⁶ is 2-benzothiazolylalanine.

### (Item X127)

The selective antagonist, the peptide, or the modified peptide based on said peptide, or the prodrug thereof, or the salt or solvate thereof of any of the preceding items, wherein
A² is Gly,
A³ is Phe,
A⁵ is Thr,
A⁷ is Cys, and
A⁸ is Lys.

### (Item X128)

The selective antagonist, the peptide, or the modified peptide based on said peptide, or the prodrug thereof, or the salt or solvate thereof of any of the preceding items, wherein X¹ is -COOH, a benzoyl group, a propionyl group, or a p-fluorophenylacetyl group.

### (Item X129)

The selective antagonist, the peptide, or the modified peptide based on said peptide, or the prodrug thereof, or the salt or solvate thereof of any of the preceding items, wherein X² is NH₂.

### (Item X130)

Use of a peptide having a structure of -[SCHFGPLTWVCK]-, or a modified peptide based on said peptide, or a prodrug thereof, or a salt or solvate thereof (an alphabet indicates a one letter code of an amino acid) in the manufacture of a medicament for selectively antagonizing an erythropoietin (Epo) heteroreceptor in the presence of Epo.

### (Item X131)

The use of any of the preceding items, wherein the modified peptide has a structure of formula (I):

X¹-SCH(A¹) (A²) (A³) (A⁴) (A⁵) (A⁶)V(A⁷) (A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide;
   B¹ is Gly or D-proline, k, c, v, w, t, l, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X132)

The use of any of the preceding items, satisfying at least one of the following conditions:
A² is Gly,
A³ is Phe,
A⁵ is Thr,
A⁷ is Cys, and
A⁸ is Lys.

### (Item X133)

The use of any of the preceding items, wherein A¹ is Tyr, p-fluorophenylalanine, or phenethylglycine.

### (Item X134)

The use of any of the preceding items, wherein A¹ is Tyr.

### (Item X135)

The use of any of the preceding items, wherein A⁴ is Leu or Met.

### (Item X136)

The use of any of the preceding items, wherein A⁴ is Met.

### (Item X137)

The use of any of the preceding items, wherein A⁶ is Trp, Met, 8-naphthylalanine, 2-quinolylalanine, 5-chloro-Trp, or 2-benzothiazolylalanine.

### (Item X138)

The use of any of the preceding items, wherein A⁶ is 2-benzothiazolylalanine.

### (Item X139)

The use of any of the preceding items, wherein
A² is Gly,
A³ is Phe,
A⁵ is Thr,
A⁷ is Cys, and
A⁸ is Lys.

### (Item X140)

The use of any of the preceding items, wherein X¹ is - COOH, a benzoyl group, a propionyl group, or a p-fluorophenylacetyl group.

### (Item X141)

The use of any of the preceding items, wherein X² is NH₂.

### (Item X142)

The use of any of the preceding items, wherein the peptide, or the modified peptide, or the prodrug thereof, or the salt or solvate thereof has substantially no affinity to an Epo homodimer receptor in the presence of Epo.

### (Item X143)

The use of any of the preceding items, wherein the peptide, or the modified peptide, or the prodrug thereof, or the salt or solvate thereof has affinity to an Epo homodimer receptor in the absence of Epo.

### (Item X144)

The use of any of the preceding items, wherein the Epo heteroreceptor comprises an Epo receptor and a βc receptor.

### (Item X145)

The use of any of the preceding items, wherein the peptide, or the modified peptide based on said peptide, or the prodrug thereof, or the salt or solvate thereof, in the presence of Epo, does not antagonize an Epo homodimer receptor, but antagonizes an Epo heteroreceptor comprising an Epo receptor and a βc receptor.

### (Item X146)

The use of any of the preceding items, wherein the peptide, or the modified peptide based on said peptide, or the prodrug thereof, or the salt or solvate thereof selectively binds to a low affinity binding region of an EpoR.

### (Item X147)

The use of any of the preceding items, wherein the medicament is for administration in combination with an anticancer agent.

### (Item X148)

The use of any of the preceding items, wherein the medicament improves an adverse action elicited by an anticancer agent in the presence of Epo.

### (Item X149)

The use of any of the preceding items, wherein the anticancer agent comprises an angiogenesis inhibitor.

### (Item X150)

The use of any of the preceding items, wherein the anticancer agent comprises bevacizumab, tamoxifen, doxorubicin, or trastuzumab.

### (Item X151)

Use of a selective antagonist of an Epo heteroreceptor in the manufacture of a medicament for treating or preventing a disease, disorder, or symptom characterized by the presence of a cell expressing an Epo heteroreceptor.

### (Item X152)

Use of a peptide having a structure of -[SCHFGPLTWVCK]-, or a modified peptide based on said peptide, or a prodrug thereof, or a salt or solvate thereof (an alphabet indicates a one letter code of an amino acid) in the manufacture of a medicament for treating or preventing a disease, disorder, or symptom characterized by the presence of a cell expressing an Epo heteroreceptor.

### (Item X153)

The use of any of the preceding items, wherein the medicament does not induce anemia.

### (Item X154)

The use of any of the preceding items, wherein the disease is a proliferative disease, rheumatism, diabetic retinopathy, keloid, or adenomyosis.

### (Item X155)

The use of any of the preceding items, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

### (Item X156)

The use of any of the preceding items, wherein the medicament is for administration to a subject with a serum erythropoietin concentration of several mIU/mL or greater (e.g., 3 mIU/mL or greater).

### (Item X157)

The use of any of the preceding items, wherein the medicament is for administration to a subject with a hemoglobin concentration of 13 g/dL or less.

### (Item X158)

The use of any of the preceding items, wherein the medicament is for administration in combination with an anticancer agent.

### (Item X159)

The use of any of the preceding items, wherein the anticancer agent comprises an angiogenesis inhibitor.

### (Item X160)

The use of any of the preceding items, wherein the anticancer agent comprises bevacizumab, tamoxifen, doxorubicin, or trastuzumab.

### (Item X161)

The use of any of the preceding items, wherein the modified peptide has a structure of formula (I):

X¹-SCH(A¹) (A²) (A³) (A⁴) (A⁵) (A⁶)V(A⁷) (A⁸)-X²

wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
   has a structure of formula (II):

   X¹-kcvwtl(B¹)Gfhcs-X²

   wherein:
   X¹ is an amino terminal side of the peptide, and X² is a carboxy terminal side of the peptide:
   B¹ is Gly or D-proline, k, c, v, w, t, l, G, f, h, c, and s each indicate an amino acid, an upper case letter indicates an L form or no enantiomer, and a lower case letter indicates a D form;
   two sulfur atoms in the peptide may form a disulfide bond;
   X¹ is hydrogen, a peptide consisting of any 1 to 3 amino acids, or -C(=O)R¹;
   X² is hydrogen, a peptide consisting of any 1 to 3 amino acids, or -NR¹₂;
   each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
   each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

### (Item X162)

The use of any of the preceding items, satisfying at least one of the following conditions:
A² is Gly,
A³ is Phe,
A⁵ is Thr,
A⁷ is Cys, and
A⁸ is Lys.

### (Item X163)

The use of any of the preceding items, wherein A¹ is Tyr, p-fluorophenylalanine, or phenethylglycine.

### (Item X164)

The use of any of the preceding items, wherein A¹ is Tyr.

### (Item X165)

The use of any of the preceding items, wherein A⁴ is Leu or Met.

### (Item X166)

The use of any of the preceding items, wherein A⁴ is Met.

### (Item X167)

The use of any of the preceding items, wherein A⁶ is Trp, Met, 8-naphthylalanine, 2-quinolylalanine, 5-chloro-Trp, or 2-benzothiazolylalanine.

### (Item X168)

The use of any of the preceding items, wherein A⁶ is 2-benzothiazolylalanine.

### (Item X169)

The use of any of the preceding items, wherein
A² is Gly,
A³ is Phe,
A⁵ is Thr,
A⁷ is Cys, and
A⁸ is Lys.

### (Item X170)

The use of any of the preceding items, wherein X¹ is - COOH, a benzoyl group, a propionyl group, or a p-fluorophenylacetyl group.

### (Item X171)

The use of any of the preceding items, wherein X² is NH₂.

The present disclosure is intended so that one or more of the features described above can be provided not only as the explicitly disclosed combinations, but also as other combinations. Additional embodiments and advantages of the present disclosure are recognized by those skilled in the art by reading and understanding the following detailed description as needed.

### [Advantageous Effects of Invention]

The peptide of the present disclosure is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, and low normal myeloid cell killing capability, and can be capable of reducing side effects thereupon, such as not inducing anemia, based thereon.

### [Brief Description of Drawings]

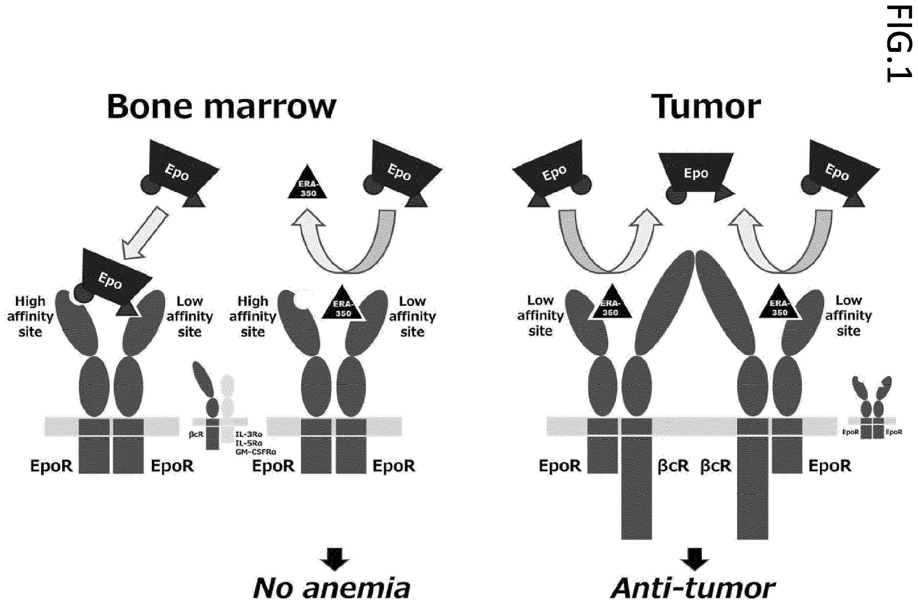

[Figure 1] Figure **1** is a schematic diagram showing the presumed mechanism of action of an anti-EpoR peptide.

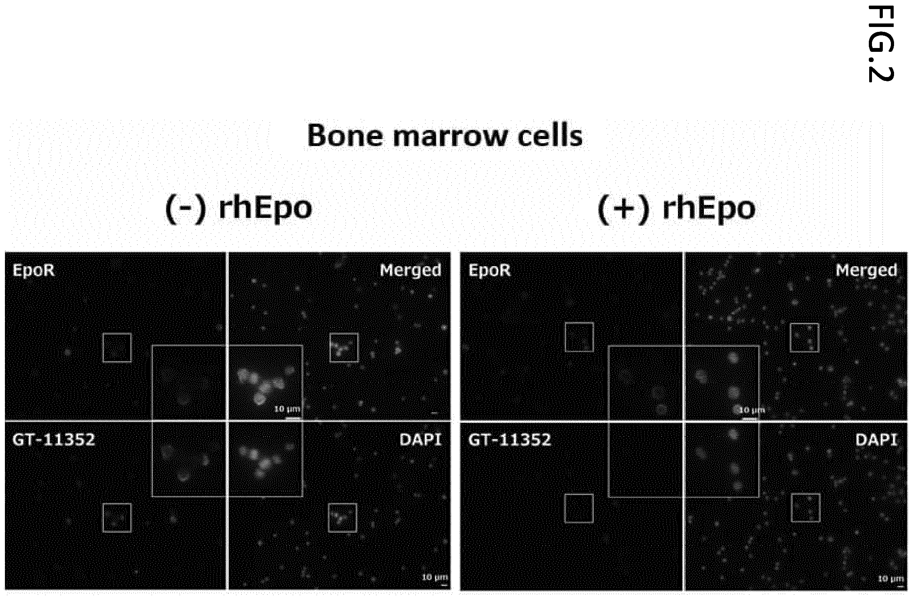

[Figure 2] Figure **2** is a fluorescence microscope picture showing the presence of anti-EpoR peptides (GT-11352) and EpoR in bone marrow cells cultured with anti-EpoR peptides in the presence ((+) rhEpo) or absence ((-) rhEpo) of recombinant human erythropoietin. Under each condition, the top left picture shows staining by an anti-EpoR antibody, the bottom left picture shows staining targeting biotin (GT-11352), the bottom right picture shows nuclear staining by DAPI, and the top right picture shows pictures thereof that have been merged. The expanded images of regions surrounded by a square are shown inside. The scale bar indicates 10 um. EpoR and anti-EpoR peptides exhibit co-localization in the absence of erythropoietin, but not in the presence of erythropoietin.

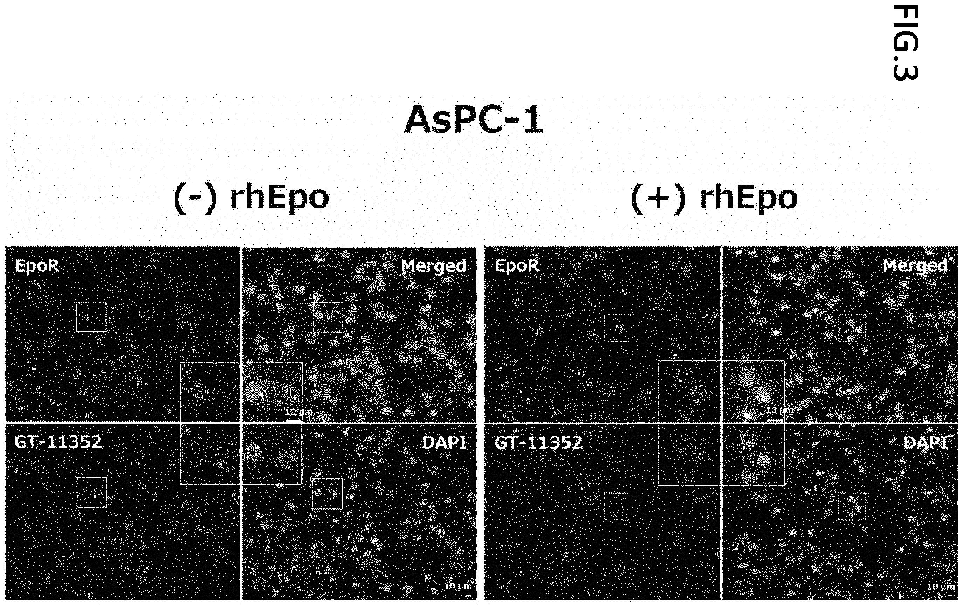

[Figure 3] Figure **3** is a fluorescence microscope picture showing the presence of anti-EpoR peptides (GT-11352) and EpoR in human pancreatic cancer derived AsPC-1 cells cultured with anti-EpoR peptides in the presence ((+) rhEpo) or absence ((-) rhEpo) of recombinant human erythropoietin. Under each condition, the top left picture shows staining by an anti-EpoR antibody, the bottom left picture shows staining targeting biotin (GT-11352), the bottom right picture shows nuclear staining by DAPI, and the top right picture shows pictures thereof that have been merged. The expanded images of regions surrounded by a square are shown inside. The scale bar indicates 10 um. EpoR and anti-EpoR peptides exhibit co-localization in the absence and in the presence of erythropoietin.

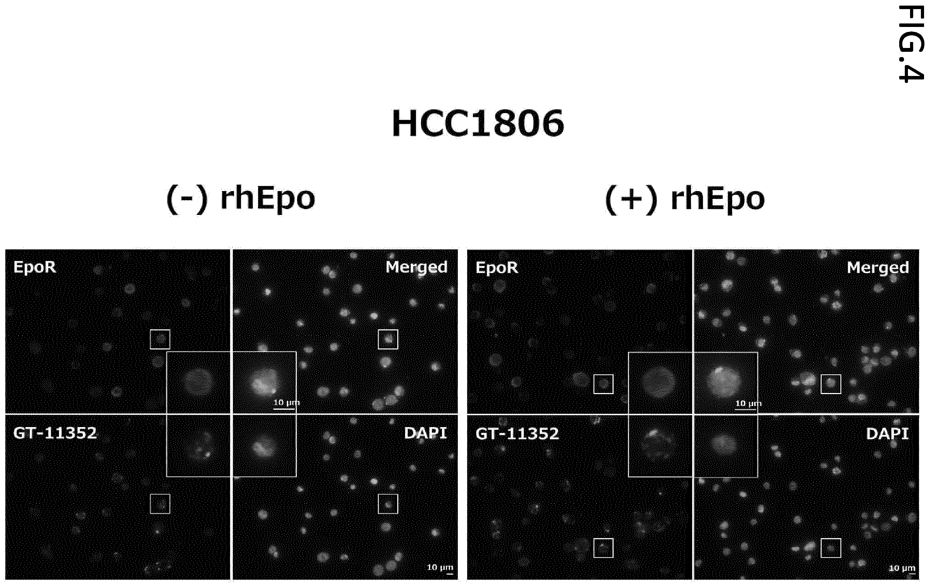

[Figure 4] Figure **4** is a fluorescence microscope picture showing the presence of anti-EpoR peptides (GT-11352) and EpoR in human breast cancer derived HCC1806 cells cultured with anti-EpoR peptides in the presence ((+) rhEpo) or absence ((-) rhEpo) of recombinant human erythropoietin. Under each condition, the top left picture shows staining by an anti-EpoR antibody, the bottom left picture shows staining targeting biotin (GT-11352), the bottom right picture shows nuclear staining by DAPI, and the top right picture shows pictures thereof that have been merged. The expanded images of regions surrounded by a square are shown inside. The scale bar indicates 10 um. EpoR and anti-EpoR peptides exhibit co-localization in the absence and in the presence of erythropoietin.

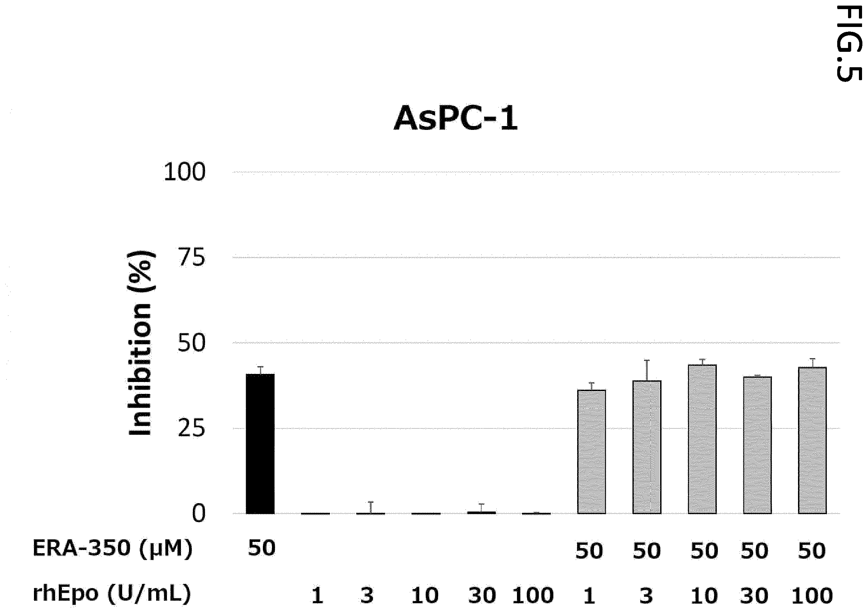

[Figure 5] Figure **5** shows results of evaluating the effect of recombinant human Epo (rhEpo) on suppression of AsPC-1 cell growth with ERA-350 by a WST assay. The vertical axis indicates the inhibition percentage relative to a control (well without test compound) under each condition. The horizontal axis indicates the agent used under each test condition and the concentration (µM) thereof. The results are denoted as the average ± standard error of three wells.

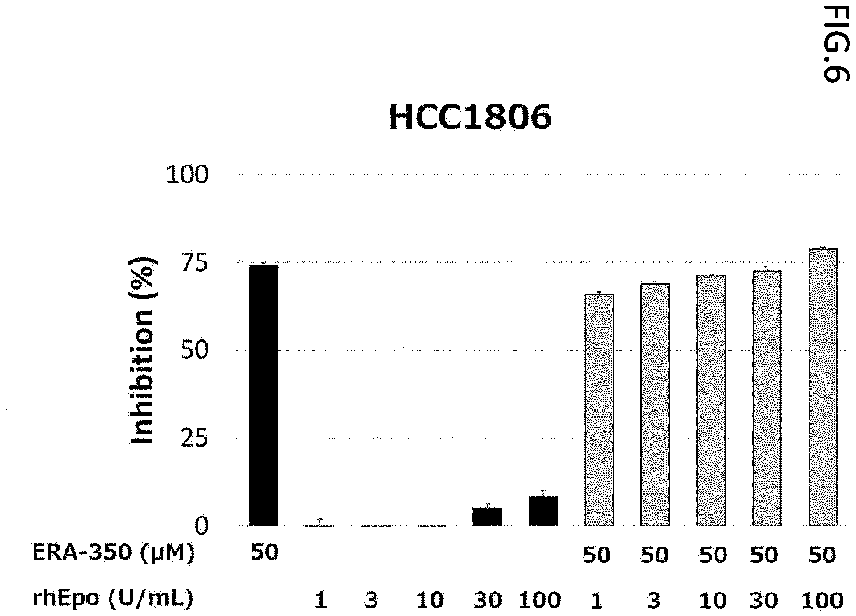

[Figure 6] Figure **6** shows results of evaluating the effect of recombinant human Epo (rhEpo) on suppression of HCC1806 cell growth with ERA-350 by a WST assay. The vertical axis indicates the inhibition percentage relative to a control (well without test compound) under each condition. The horizontal axis indicates the agent used under each test condition and the concentration (µM) thereof. The results are denoted as the average ± standard error of three wells.

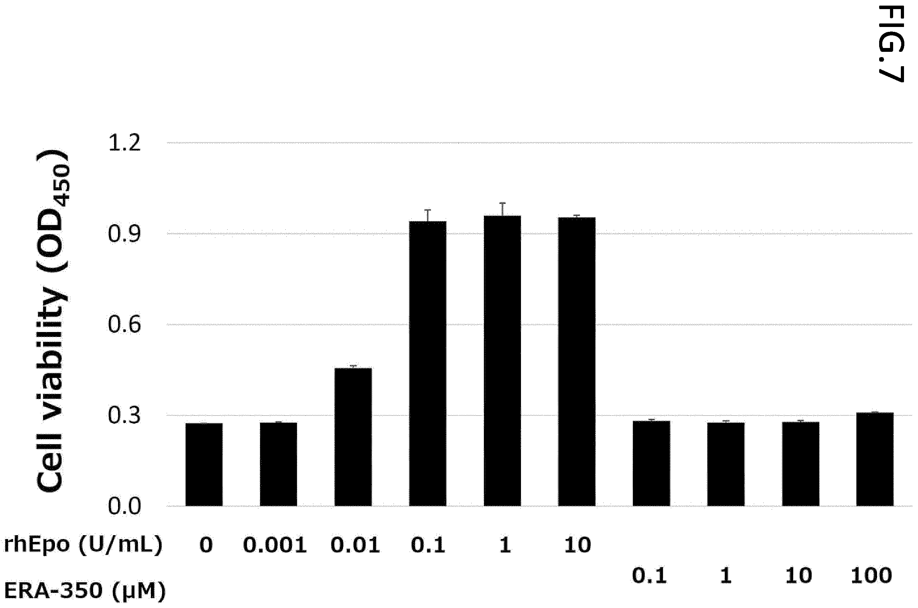

[Figure 7] Figure **7** shows results of evaluating the effect of ERA-350 on Epo dependent cancer cell growth by a WST assay. The growth of human leukemia derived UT-7/Epo cells in the presence of 0 to 10 U/mL of recombinant human Epo (rhEpo) or 0.1 to 100 µM of ERA-350 is shown. The vertical axis indicates the absorbance at 450 nm. The results are denoted as the average ± standard error of three wells.

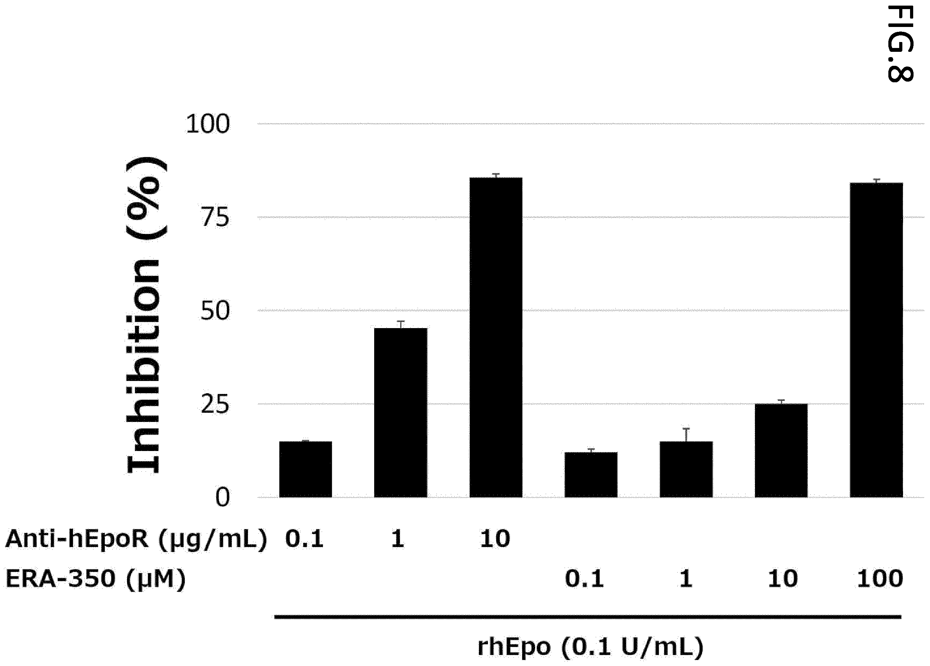

[Figure 8] Figure **8** shows results of evaluating the effect of ERA-350 on Epo dependent cancer cell growth by a WST assay. The vertical axis indicates the percentage of UT-7/Epo cell growth inhibited by addition of an anti-EpoR neutralizing antibody (clone 713210) (0.1 to 10 µg/mL) or ERA-350 (0.1 to 100 µM) in the presence of 0.1 U/mL of recombinant human Epo (rhEpo). The horizontal axis indicates the tested agent. The results are denoted as the average ± standard error of three wells.

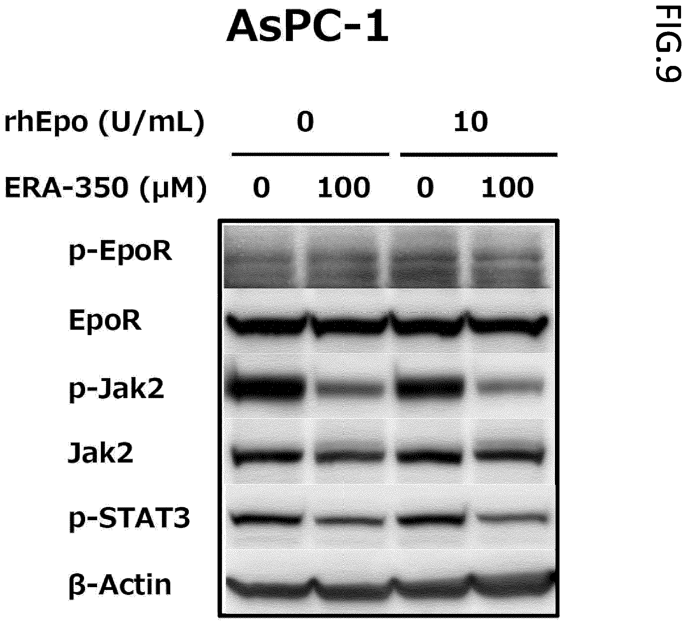

[Figure 9] Figure **9** is a Western blot of Epo signaling related molecules when AsPC-1 cells cultured in the presence or absence of ERA-350 were stimulated or not simulated with recombinant human Epo (rhEpo). The conditions of each column, from the left, are no Epo stimulation/no ERA-350, no Epo stimulation/100 µM ERA-350, with Epo stimulation/no ERA-350, and with Epo stimulation/100 µM ERA-350. Each band is, from the top, phosphorylated EpoR, EpoR, phosphorylated Jak2, Jak2, phosphorylated STAT3, and β-actin.

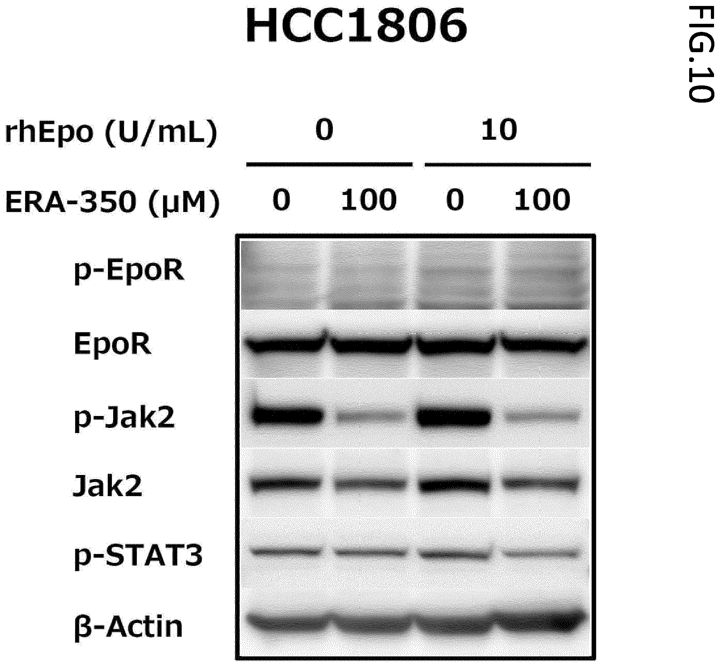

[Figure 10] Figure **10** is a Western blot of Epo signaling related molecules when HCC1806 cells cultured in the presence or absence of ERA-350 were stimulated or not simulated with recombinant human Epo (rhEpo). The conditions of each column, from the left, are no Epo stimulation/no ERA-350, no Epo stimulation/100 µM ERA-350, with Epo stimulation/no ERA-350, and with Epo stimulation/100 µM ERA-350. Each band is, from the top, phosphorylated EpoR, EpoR, phosphorylated Jak2, Jak2, phosphorylated STAT3, and β-actin.

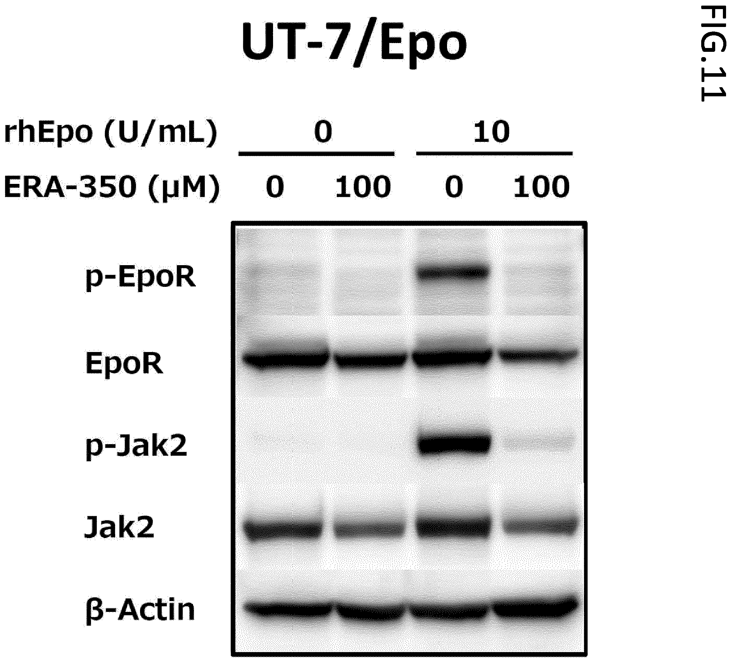

[Figure 11] Figure **11** is a Western blot of EpoR and Jak2, and phosphorylated forms thereof when UT-7/Epo cells cultured in the presence or absence of ERA-350 were stimulated or not simulated with recombinant human Epo (rhEpo). The conditions of each column, from the left, are no Epo stimulation/no ERA-350, no Epo stimulation/with 100 µM ERA-350, with Epo stimulation/no ERA-350, and with Epo stimulation/with 100 µM ERA-350. Each band is, from the top, phosphorylated EpoR, EpoR, phosphorylated Jak2, Jak2, and β-actin.

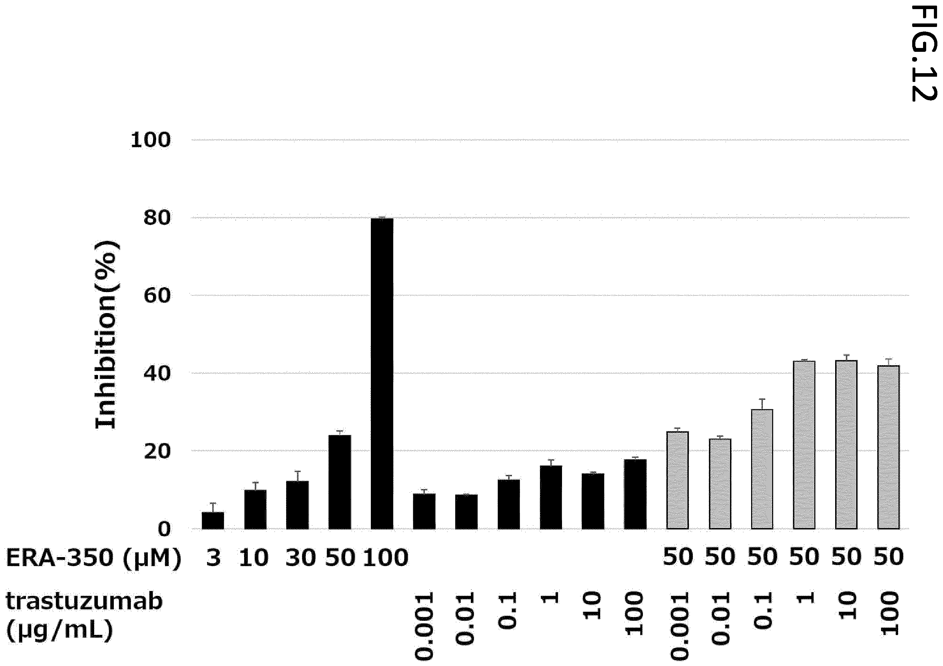

[Figure 12] Figure **12** shows results of evaluating the effect of concomitant use of trastuzumab (Herceptin^{®}) on Herceptin resistant breast cancer BT-474-R cell growth with ERA-350 by a WST assay. The vertical axis indicates the inhibition percentage relative to a control (well without test compound) under each condition. The horizontal axis indicates the agent used under each test condition and the concentration thereof (µM or µg/mL). The results are denoted as the average ± standard error of three wells.

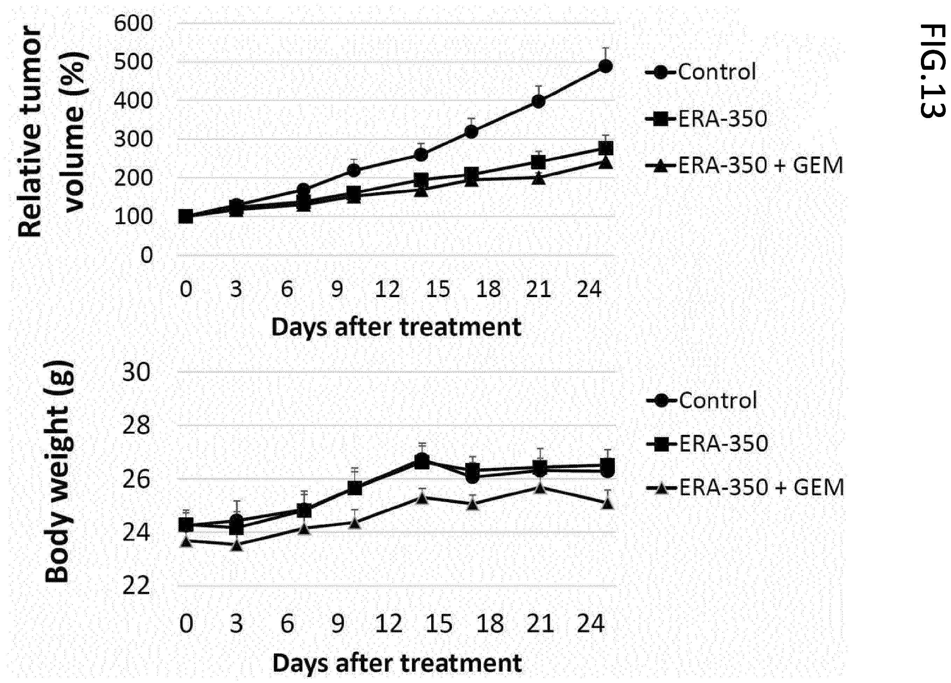

[Figure 13] Figure **13** shows results of evaluating the effect of concomitant use of gemcitabine on AsPC-1 cancer bearing mice with ERA-350. In the top panel, the vertical axis indicates the relative tumor volume at each point of time while assuming the tumor volume as of the start of the test as 100%. The horizontal axis indicates the days elapsed from the administration starting date. In the bottom panel, the vertical axis indicates the body weight at each point of time, and the horizontal axis indicates the number of days elapsed from the administration starting date. Control indicates saline administration, and ERA-350+GEM indicates concomitant use of ERA-350 and gemcitabine. The results are denoted as the average ± standard error of six mice.

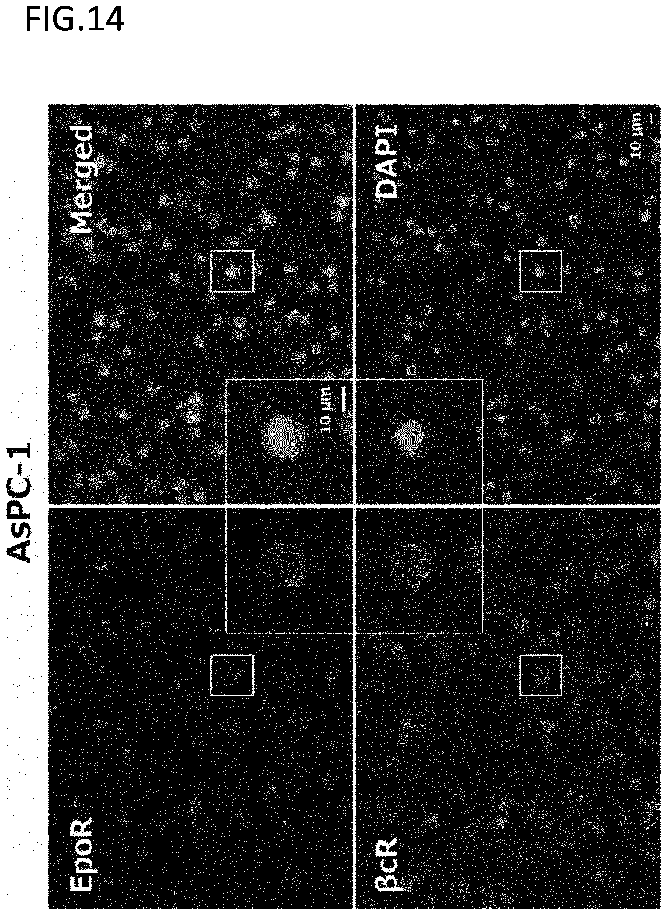

[Figure 14] Figure **14** is a fluorescence microscope picture showing the presence of EpoR and βcR in human pancreatic cancer derived AsPC-1 cells. The top left picture shows staining by an anti-EpoR antibody, the bottom left picture shows staining by an anti-βcR antibody, the bottom right picture shows nuclear staining by DAPI, and the top right picture shows the merge thereof. The expanded images of regions surrounded by a square are shown inside. The scale bar indicates 10 um. EpoR and βcR exhibit co-localization.

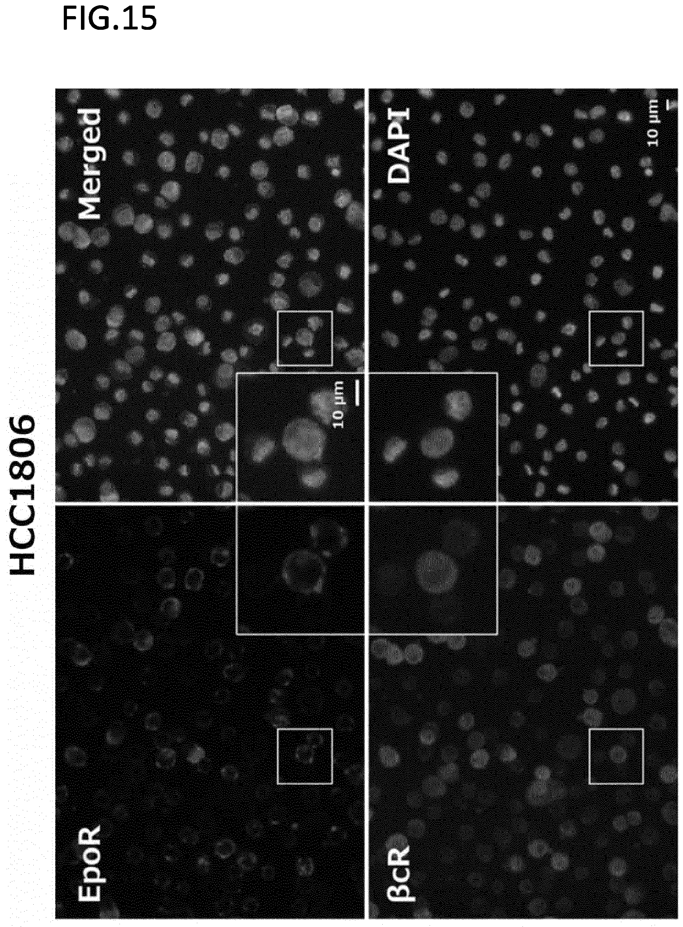

[Figure 15] Figure **15** is a fluorescence microscope picture showing the presence of EpoR and βcR in human breast cancer derived HCC1806 cells. The top left picture shows staining by an anti-EpoR antibody, the bottom left picture shows staining by an anti-βcR antibody, the bottom right picture shows nuclear staining by DAPI, and the top right picture shows pictures thereof that have been merged. The expanded images of regions surrounded by a square are shown inside. The scale bar indicates 10 um. EpoR and βcR exhibit co-localization.

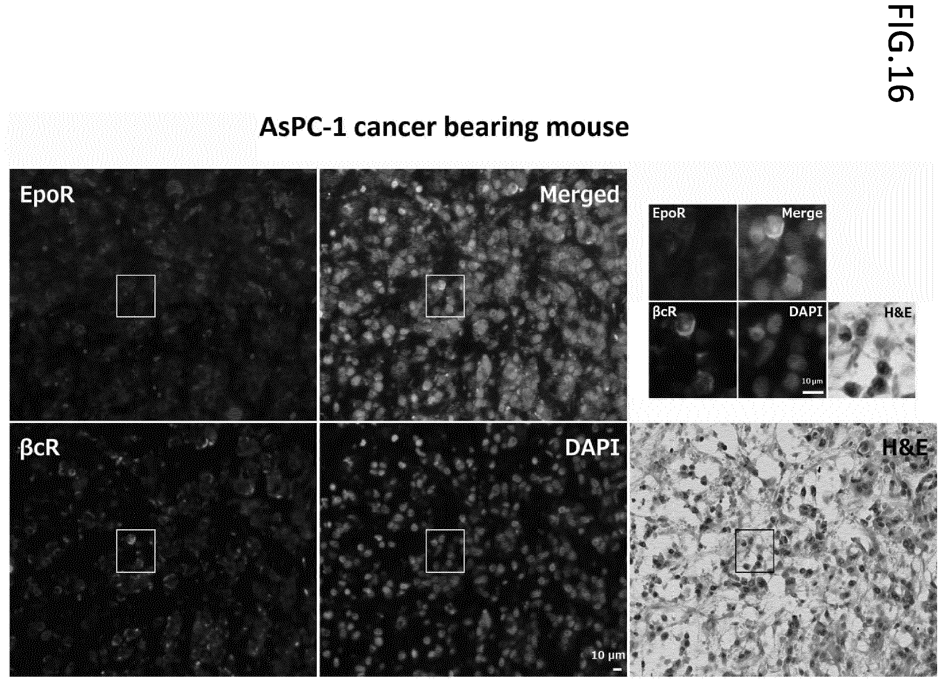

[Figure 16] Figure **16** is a fluorescence microscope picture and hematoxylin and eosin stain picture showing the presence of EpoR and βcR in tumor tissue of AsPC-1 cancer bearing mice. The top left picture shows staining by an anti-EpoR antibody, the bottom left picture shows staining by an anti-βcR antibody, the bottom center picture shows nuclear staining by DAPI, and the top center picture shows pictures thereof that have been merged. The bottom right picture is an image of hematoxylin and eosin staining. The top right pictures are expanded images of regions surrounded by a square. The scale bar indicates 10 um.

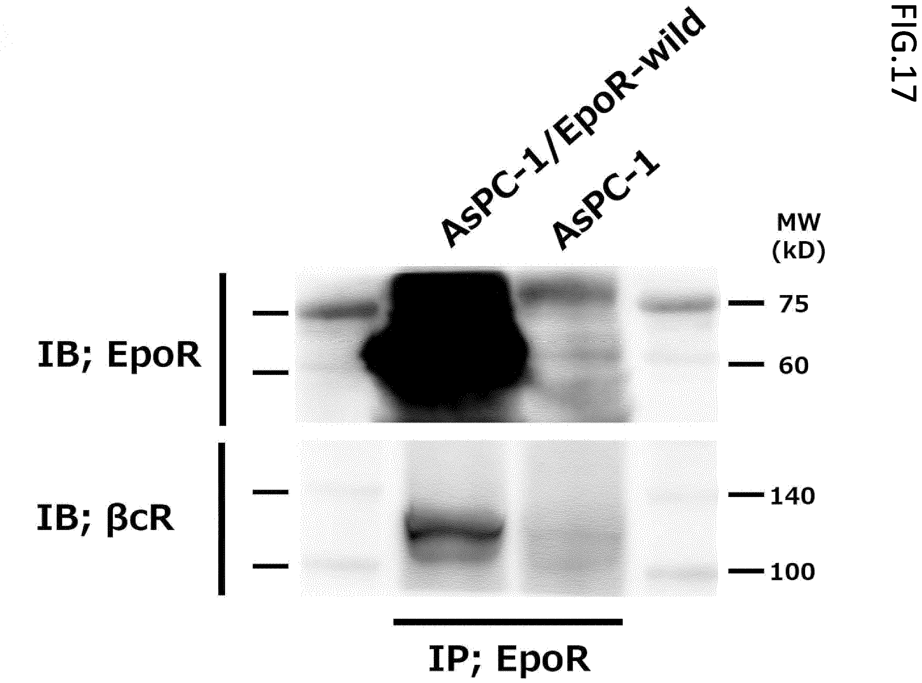

[Figure 17] Figure **17** shows an immunoblot, using an anti-EpoR antibody and an anti-βcR antibody, of a sample prepared by immunoprecipitation of a membrane fraction of the AsPC-1 wild-type strain or EpoR overexpressing strain with an anti-EpoR antibody.

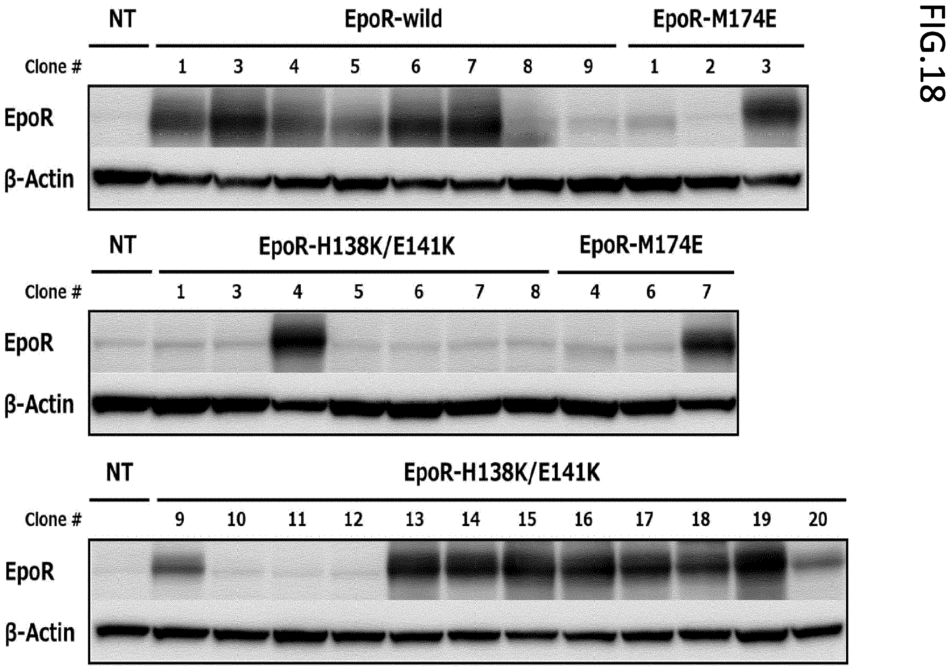

[Figure 18] Figure **18** is a Western blot showing expression of EpoR in each clone obtained by transfecting 293T cells with an expression vector encoding wild-type or mutant (M174E or H138K/E141K mutant) EpoRs. NT indicates no transfection, EpoR-wild indicates transfection with an expression vector encoding wild-type EpoR, EpoR-M174E indicates transfection with an expression vector encoding EpoR introduced with an M174E mutation, and EpoR-H138K/E141K indicates transfection with an expression vector encoding EpoR introduced with H138K and E141K mutations. The top band indicates EpoR, and the bottom band indicates β-actin.

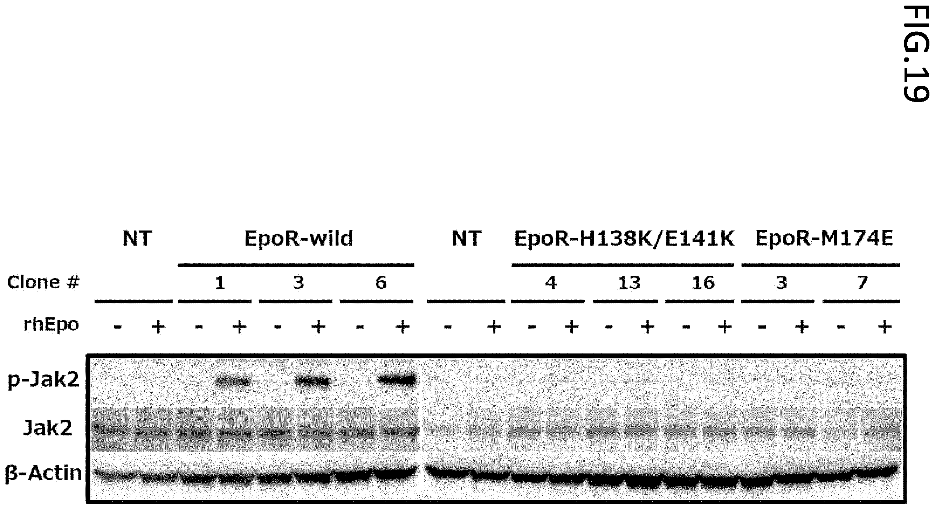

[Figure 19] Figure **19** shows Jak2 phosphorylation responsiveness to Epo stimulation using a clone observed to have overexpression of EpoR in Figure **18****.** Stimulation was applied for 5 minutes in the presence (+) or absence (-) of human recombinant Epo (rhEpo) (10 U/mL). Each band indicates, from the top, phosphorylated Jak2, Jak2, and β-actin.

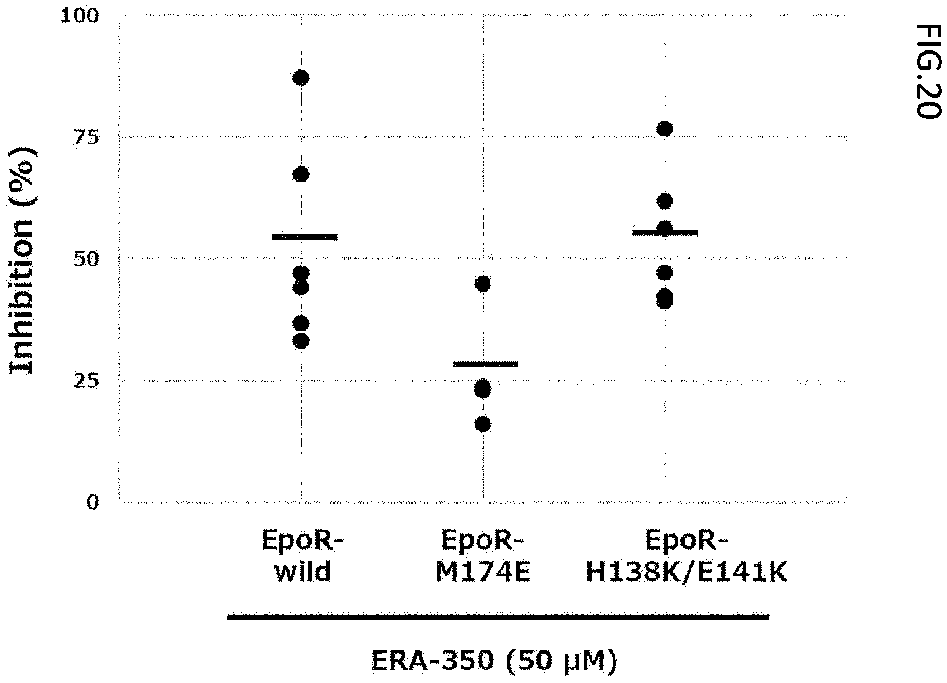

[Figure 20] Figure **20** shows the results of evaluating the effect of 50 µM of ERA-350 on the growth of wild-type or mutant EpoR overexpressing 293T cells by a WST assay. The vertical axis indicates the inhibition percentage relative to control (well without ERA-350) under each condition. Each clone was tested in three wells. The horizontal axis shows, from the left, strains introduced with an expression vector encoding an EpoR of wild-type, an EpoR introduced with a M174E mutation and an EpoR introduced with H138K and E141K mutations.

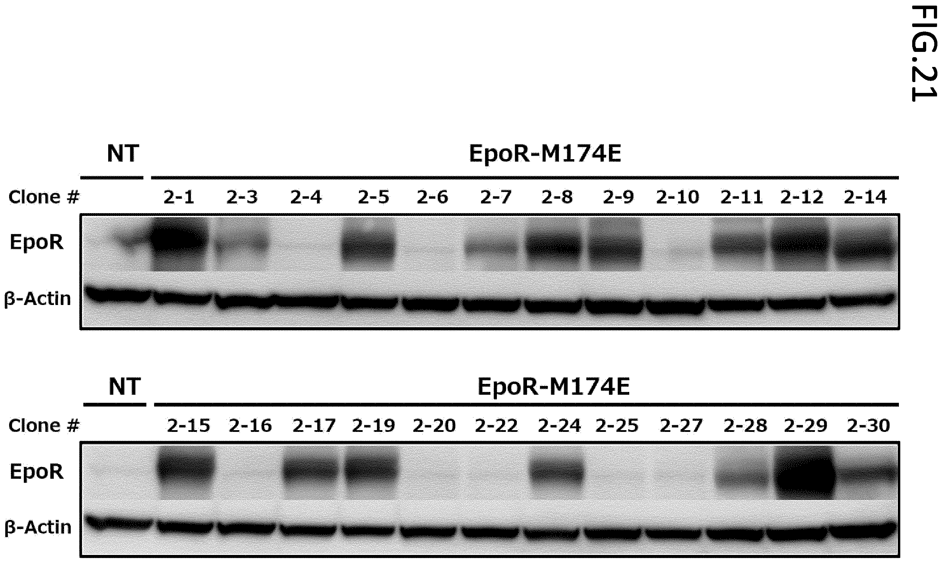

[Figure 21] Figure **21** is a Western blot showing expression of EpoR in an additional clone obtained by transfection of 293T cells with an expression vector encoding a mutant (M174E mutation) EpoR. NT indicates no transfection, and EpoR-M174E indicates transfection with an expression vector encoding EpoR introduced with an M174E mutation. The top band indicates EpoR, and the bottom band indicates β-actin.

### [Description of Embodiments]

The present disclosure is described while showing the best mode of the disclosure. Throughout the entire specification, a singular expression should be understood as encompassing the concept thereof in the plural form, unless specifically noted otherwise. Thus, singular articles (e.g., "a", "an", "the", and the like in the case of English) should also be understood as encompassing the concept thereof in the plural form, unless specifically noted otherwise. The terms used herein should also be understood as being used in the meaning that is commonly used in the art, unless specifically noted otherwise. Thus, unless defined otherwise, all terminologies and scientific technical terms that are used herein have the same meaning as the general understanding of those skilled in the art to which the present invention pertains. In case of a contradiction, the present specification (including the definitions) takes precedence.

### (Definitions)

The definitions of the terms and/or the detailed basic technology that are particularly used herein are described hereinafter as appropriate.

As used herein, "peptide" is used in the conventional meaning in the art, referring to a compound that is a polymer of a plurality of amino acids linked by a peptide bond and a modified form thereof. When a peptide sequence is described herein, the N-terminus (amino terminus) is positioned on the left, and the C-terminus (carboxy terminus) is positioned on the right, unless specifically noted otherwise. For example, in a peptide represented by a sequence of AGTCI (alanine-glycine-threonine-cysteine-isoleucine), an amino acid of alanine is free, a carboxyl group of alanine is forming a peptide bond with an amino group of glycine, an amino group of isoleucine is forming a peptide bond with a carboxyl group of cysteine, and a carboxyl group of isoleucine is free. The presence of a chemical group at the end of the sequence of a peptide indicates that an amino group or carboxyl group of the terminal amino acid of the peptide sequence is forming a bond with the chemical group. For example, in the structure CH₃-CO-SCHFGPLTWVCK-NH₂, an amino group of serine at the N-terminus is forming a bond with a CH₃-CO- group, and a carboxyl group of lysine at the C-terminus is forming a bond with an -NH₂ group. Examples of modification include, but are not limited to, acetylation, alkylation (e.g., methylation), halogen (e.g., fluoro, chloro)-substituted alkylation (e.g., fluoromethylation), alkoxylation (e.g., methoxylation), hydroxylation, halogenation, benzothiazolylation, dicyclization (naphthylation), and heterocyclization (e.g., quinolination).

As used herein, "erythropoietin" is used in the conventional meaning of the art, referring to a glycoprotein hormone with a molecular weight of 34,000 to 46,000 that acts on erythroid stem cells (progenitor cells) and stimulates differentiation induction to promote production of red blood cells. As used herein, erythropoietin can also be denoted as EPO or Epo. For human, nucleic acid sequence: NM_000799.2 and amino acid sequence: NP_000790.2 (SEQ ID NO: 16) are representative sequences.

As used herein, "erythropoietin receptor" is used in the conventional meaning in the art, referring to a receptor that binds to an erythropoietin. The receptor is expressed on bone marrow cells, white blood cells, and peripheral/central nerves in addition to red blood cells. When EPO binds to an erythropoietin receptor on red blood cells, Janus kinase 2 (JAK2) is activated. An erythropoietin receptor can also be denoted herein as EpoR. It is known to generally form a homodimer in white blood cells, red blood cell progenitor cells such as burst-forming unit-erythroid (BFU-E), colony-forming unit-erythroid (CFU-E), proerythroblast, and the like that are present in peripheral/central nerves, or bone marrows. As used herein, "Epo homodimer receptor" refers to a complex comprising two molecules of EpoR. For human, nucleic acid sequence: NM_000121.3 and amino acid sequence: NP_000112.1 (SEQ ID NO: 17) are representative sequences. It is reported that Epo binds to two locations, i.e., a high affinity binding region and a low affinity binding region, of an Epo homodimer receptor (Mol Cell. 2009 Jan 30; 33(2) : 2 6 6-7 4 . (Figure **1**). An Epo receptor may form a heterecomplex (e.g., dimer, trimer, tetramer, etc.) with an Epo receptor monomer and another type of monomer. As used herein, "Epo heteroreceptor" refers to a complex comprising an EpoR and another receptor molecule.

As used herein, "low affinity binding region" of an erythropoietin receptor refers to one of the two binding regions of an Epo homodimer receptor to which an Epo binds, and is a portion having a three-dimensional structure of an Epo receptor centered around loop 3 (amino acid residues 110 to 118) of a human Epo receptor which is the primary binding site for Epo. This can also be maintained as an Epo binding region in an Epo heteroreceptor. Loop 3 of an Epo receptor can be a site that interacts with an F residue or W residue of the anti-EpoR peptide of the present disclosure. It is understood that loop 5 (amino acid residues 168 to 177) of a human Epo receptor is a critical site that affects agonistic or antagonistic property, and an introduction of a mutation such as M174E inhibits the inherent function of the low affinity binding region. As used herein, "high affinity binding region" of an erythropoietin receptor refers to a region of the two binding regions of an Epo homodimer receptor to which Epo binds that is different from the low affinity binding region, and is a portion having a three-dimensional structure of an Epo receptor centered around a hydrophobic Epo binding region formed by loops 1, 5, and 6, and 117th F residue of a human Epo receptor, and its Epo binding capability can be lost in an Epo heteroreceptor. See R S Syed, et al., Nature. 1998 Oct 1; 395 (6701): 511-6. It is reported that an M174E mutation results in a loss of a function of a low affinity binding region, and an H138K/E141K mutation results in a loss of function of a high affinity binding region (Zhang B. K. et al. Mol Cell (2009) 33: 266).

As used herein, "βcR" is a molecule that is also referred to as CD131, CSF2RB, IL3RB, and IL5RB, and is known to form a complex with α subunits of an IL3 receptor, IL5 receptor, and GM-CSF receptor in bone marrow cells and the like. It is known to form a heterocomplex with an EpoR in cancer cells, etc. "Epo heteroreceptor" in a preferred embodiment herein can be complex comprising EpoR and βcR. Although not wishing to be bound by any theory, this is because the peptide of the present disclosure, etc. is demonstrated to selectively antagonize an Epo heteroreceptor. For human, nucleic acid sequence: NM_000395.2 and amino acid sequence: NP_000386.1 (SEQ ID NO: 18) are representative sequences. Epo can exert a tissue protecting action through an Epo heteroreceptor (Mol Med. 2012 May 9; 18(1):486-96.).

Unless specifically noted otherwise, it is understood that any reference to a biomolecule (e.g., protein, nucleic acid, Epo, EpoR, or βcR) herein is also a reference to a variant (e.g., variant with a modification in the amino acid sequence) of the molecule exerting the similar function (may not be to the same extent) as the biological function of the molecule. For example, erythropoietin (Epo) can encompass not only peptides with the amino acid sequence set forth in SEQ ID NO: 16, but also conjugates of erythropoietin and a tag molecule (biotin, etc.), variants of erythropoietin that can promote differentiation/growth of red blood cells in place of erythropoietin, etc. Such a variant can encompass fragments of the original molecule, and molecules that are identical throughout an amino acid sequence or nucleic acid sequence of the original molecule of the same size or at least 30%, 40%, 50%, 60%, 70%, 80%, 90%, 95%, 98%, or 99% identical in comparison to the original molecule sequence aligned with a computer homology program that is known in the art. Variants can encompass molecules having a modified amino acid (e.g., modification through disulfide bond formation, glycosylation, lipidation, acetylation, or phosphorylation) or a modified nucleotide (e.g., modification through methylation).

As used herein, "alkyl group", alone or as a part of another group, refers to a linear or branched saturated hydrocarbyl group with a carbon atom. An alkyl group typically can have 1 to 10 carbon atoms. In some embodiments, an alkyl group can have 1 to 8 carbon atoms, 1 to 6 carbon atoms, 1 to 4 carbon atoms, or 1 to 3 carbon atoms. Examples of C₁₋₃ alkyl group include methyl groups, ethyl groups, propyl groups, and isopropyl groups. Examples of C₁₋₄ alkyl group include the aforementioned C₁₋₃ alkyl groups, as well as butyl groups, isobutyl groups, sec-butyl groups, and tert-butyl groups. Examples of C₁₋₆ alkyl group include the aforementioned C₁₋₄ alkyl groups, as well as pentyl groups, isopentyl groups, neopentyl groups, hexyl groups, and the like. Additional examples of alkyl groups include heptyl groups, octyl groups, and the like.

As used herein, "alkylene group" is a divalent group generated by further removing one hydrogen from an "alkyl group". Specific examples of alkylene group include, but are not limited to, -CH₂-, -CH₂CH₂-, -(CH₂)₃-, -CH₂CH(CH₃)-, -(CH₂)₄-, -CH₂CH₂CH(CH₃)-, -CH₂CH(CH₃)CH₂-, -(CH₂)₅-, - CH₂CH₂CH₂CH(CH₃)-, -CH₂CH₂CH(CH₃)CH₂-, -(CH₂)₆-, -(CH₂)₇-, - (CH₂)₃-, -(CH₂)₉-, -(CH₂)₁₀-, and the like.

As used herein, "alkenyl group", alone or as a part of another group, refers to a linear or branched hydrocarbyl group having a carbon atom and one or more carbon-carbon double bonds. An alkenyl group can typically have 2 to 10 carbon atoms. In some embodiments, an alkenyl group can have 2 to 8 carbon atoms, 2 to 6 carbon atoms, or 2 to 4 carbon atoms. One or more carbon-carbon double bonds can be inside (e.g., double bond in 2-butenyl) or at a terminus (e.g., double bond in 1-butenyl). Examples of C₂₋₄ alkenyl group include ethenyl groups (vinyl groups), 1-propenyl groups, 2-propenyl groups, 1-butenyl groups, 2-butenyl groups, butadienyl groups, and the like. Examples of C₂₋₆ alkenyl group include the aforementioned C₂₋₄ alkenyl group, as well as pentenyl groups, pentadienyl groups, hexenyl groups, and the like. Additional examples of alkenyl group include heptenyl groups, octenyl groups, octatrienyl groups, and the like.

As used herein, "alkenylene group" is a divalent group generated by further removing one hydrogen from "alkenyl group". Specific examples of alkenylene group include, but are not limited to, -CH=CH-, -CH=CH-CH₂-, -CH=CH-(CH₂)₂-, - CH₂-CH=CH-CH₂-, -CH=C(CH₃)-CH₂-, -CH=CH-CH=CH-, -CH=CH-(CH₂)₃-, -CH=CH-CH=CH-CH₂-, -CH=CH-(CH₂)₄-, -CH=CH-(CH₂)₅-, - CH=CH-(CH₂)₆-, -CH=CH-(CH₂)₇-, -CH=CH-(CH₂)₈-, and the like.

As used herein, "alkoxyl group" is a monovalent group of -O-alkyl. Preferred examples of alkoxyl group include C₁₋₆ alkoxyl groups (i.e., C₁₋₆ alkyl-O-), C₁₋₄ alkoxyl groups (i.e., C₁₋₄ alkyl-O-), and the like. Specific examples of C₁₋₄ alkoxyl group include methoxyl groups (CH₃O-), ethoxyl groups (CH₃CH₂O-), n-propoxyl groups (CH₃(CH₂)₂O-), isopropoxyl groups ((CH₃)₂CHO-), n-butoxyl groups (CH₃(CH₂)₃O-), isobutoxyl groups ((CH₃)₂CHCH₂O-), tert-butoxyl groups ((CH₃)₃CO-), sec-butoxyl groups (CH₃CH₂CH(CH₃)O-), and the like. Specific examples of C₁₋₆ alkoxyl group include, but are not limited to, C₁₋₄ alkoxyl groups, n-pentyloxyl groups (CH₃(CH₂)₄O-), isopentyloxyl groups ((CH₃)₂CHCH₂CH₂O-), neopentyloxyl groups ((CH₃)₃CCH₂O-), tert-pentyloxyl groups (CH₃CH₂C(CH₃)₂O-), 1, 2-dimethylpropoxyl groups (CH₃CH(CH₃)CH(CH₃)O-), and the like.

As used herein, "aliphatic group" refers to an alkyl group, alkenyl group, and alkynyl group, and does not include cyclic hydrocarbon groups.

As used herein, "heteroaliphatic group" refers to an aliphatic group in which one part thereof is substituted with a heteroatom (e.g., nitrogen atom, oxygen atom, sulfur atom, or the like).

As used herein, the term "aryl group" refers to a single aromatic ring or a fused polycyclic system wherein at least one of the rings is aromatic and all atoms in the ring are carbon. For example, an aryl group can have 6 to 26 carbon atoms (6 to 26 members), 6 to 20 carbon atoms (6 to 20 members), 6 to 14 carbon atoms (6 to 14 members), or 6 to 12 carbon atoms (6 to 12 members). An aryl group includes a phenyl group. An aryl group includes a fused polycyclic system (e.g., cyclic system comprising 2, 3, or 4 rings) with 8 to 20 carbon atoms, wherein at least one ring is aromatic, but other rings may or may not be aromatic. Such a fused polycyclic system is optionally substituted with one or more (e.g., 1, 2, or 3) oxo groups at any carbocyclic moiety of the fused polycyclic system. Rings in a fused polycyclic system can be connected to one another via fusion, spiro, or crosslinking bond if permitted by the valency requirement. Typical examples of aryl group include, but are not limited to, phenyl groups, indenyl groups, naphthyl groups, 1,2,3,4-tetrahydronaphthyl groups, anthryl groups, pyrenyl groups, and the like.

As used herein, the term "heteroaryl group" refers to a single aromatic ring or fused polycyclic system having at least one heteroatom in a ring. The heteroatom is selected from the group consisting of oxygen, nitrogen, and sulfur. A heteroaryl group can be typically 5- to 26-membered. In some embodiments, a heteroaryl group can be 5- to 20-membered, 5- to 14-membered, 5- to 12-membered, or 5- to 10-membered. A heteroaryl group encompasses a single aromatic ring having about 1 to 6 carbon atoms and about 1 to 4 heteroatoms selected from the group consisting of oxygen, nitrogen, and sulfur in a ring. Examples of such a ring include, but are not limited to, pyridyl groups, pyrimidinyl groups, pyradinyl groups, oxazolyl groups, furyl groups, and the like. Sulfur and nitrogen atoms can also be in an oxidized form if a ring is aromatic. A heteroaryl group also encompasses fused polycyclic systems (e.g., cyclic systems comprising 2, 3, or 4 rings) in which a previously defined heteroaryl group can form a fused polycyclic system by fusing with one or more rings selected from heteroaryl (e.g., forming naphthyridinyl such as 1,8-naphthyridinyl), heterocycle (e.g., forming 1,2,3,4-tetrahydronaphthyridinyl such as 1,2,3,4-tetrahydro-1,8-naphthyridinyl), carbocycle (e.g., forming 5,6,7,8-tetrahydroquinolyl), and aryl (e.g., forming indazolyl). In some embodiments, a heteroaryl group (single aromatic ring or fused polycyclic system) has about 1 to 20 carbon atoms and about 1 to 6 heteroatoms in a heteroaryl ring. Such a fused polycyclic system is optionally substituted with one or more (e.g., 1, 2, 3 or 4) oxo groups at a carbocyclic or heterocyclic moiety of the fused ring. Rings in a fused polycyclic system can be connected to one another via fusion, spiro, or crosslinking bond if permitted by the valency requirement. It is understood that individual rings in a fused polycyclic system can be bound to one another in any order. It is understood that a position of a bond in the fused polycyclic system described above can be at any position of the fused polycyclic system including the heteroaryl, heterocycle, aryl, or carbocyclic moiety of the fused polycyclic system, and any suitable atom of the fused polycyclic system including a carbon atom and heteroatom (e.g., nitrogen). Examples of heteroaryl group include, but are not limited to, quinolyl groups, benzothiazolyl groups, pyridyl groups, pyrrolyl groups, pyradinyl groups, pyrimidinyl groups, pyridazinyl groups, pyrazolyl groups, thienyl groups, indolyl groups, imidazolyl groups, oxazolyl groups, isooxazolyl groups, thiazolyl groups, furyl groups, oxadiazolyl groups, thiadiazolyl groups, isoquinolyl groups, benzooxazolyl groups, indazolyl groups, quinoxalyl groups, quinazolyl groups, 5,6,7,8-tetrahydroisoquinolinyl benzofuranyl groups, benzoimidazolyl groups, thianaphthenyl groups, pyrrolo[2,3-b]pyridinyl groups, quinazolinyl-4(3H)-one groups, triazolyl groups, 4,5,6,7-tetrahydro-1H-indazole groups, 3b,4,4a,5-tetrahydro-1H-cyclopropa[3,4]cyclopenta[1,2-c]pyrazole groups, and the like.

As used herein, "halo" or "halogen", alone or as a part of another group, refers to fluorine (fluoro), chlorine (chloro), bromine (bromo), or iodine (iodo), unless specifically noted otherwise.

As used herein, "haloalkyl group", alone or as a part of another group, refers to an alkyl group with one or more hydrogen atoms each independently substituted with halo, unless specifically noted otherwise. Examples of haloalkyl group include -CCl₃, -CFCl₂, -CF₂Cl, -CCl₂CCl₃, -CH₂F, -CHF₂, -CH₂Cl, -CH₂Br, -CH(Cl)CH₂Br, -CH₂CH(F)CH₂Cl, and the like. A "haloalkenyl group", "haloalkynyl group", "haloaliphatic group", and the like are also defined in the same manner as the aforementioned "haloalkyl group".

As used herein, "carbocycle" or "carbocyclic group", alone or as a part of another group, refers to a monocyclic, bicyclic, or tricyclic hydrocarbon group, or polycyclic hydrocarbon group with more rings, which is completely saturated or comprises one or more unsaturated units but is not aromatic. In one embodiment, a carbocyclic group can be a monocyclic C₃₋₉ hydrocarbon group, bicyclic C₈₋₁₂ hydrocarbon group, or tricyclic C₁₀₋₁₆ hydrocarbon group. Any individual ring in the carbocyclic group described above can have 3 to 7 ring atoms. Examples of carbocyclic group include, but are not limited to, cycloalkyl groups such as cyclopropyl groups, cyclobutyl groups, cyclopentyl groups, cyclohexyl groups, cycloheptyl groups, cyclooctyl groups, and cyclononyl groups, cycloalkenyl groups such as cyclopropenyl groups, cyclobutenyl groups, cyclopentenyl groups, cyclohexenyl groups, cycloheptenyl groups, cyclooctenyl groups, and cyclononenyl groups, cycloalkynyl groups such as cyclopropynyl groups, cyclobutynyl groups, cyclopentynyl groups, cyclohexynyl groups, cycloheptynyl groups, cyclooctynyl groups, and cyclononyl groups, adamantyl groups, and the like. In a carbocyclic group, one of the atoms in the ring can be bound to the rest of the portions of the molecule when possible.

As used herein, "heterocycle" or "heterocyclic group", alone or as a part of another group, refers to a monocyclic, bicyclic, or tricyclic system, or polycyclic system with more groups, which is completely saturated or comprises one or more unsaturated units but is not aromatic, wherein at least one ring in the cyclic system comprises one or more same or different heteroatoms. In some embodiments, a "heterocycle" or "heterocyclic group" has 3 to 14 ring atoms, wherein one or more ring atoms are heteroatoms independently selected from oxygen, sulfur, nitrogen, or phosphorous, and each ring in the cyclic system comprises 3 to 8 ring atoms.

Examples of heterocyclic group include, but are not limited to, monocycles such as 2-tetrahydrofuranyl groups, 3-tetrahydrofuranyl groups, 2-tetrahydrothiophenyl groups, 3-tetrahydrothiophenyl groups, 2-morpholino groups, 3-morpholino groups, 4-morpholino groups, 2-thiomorpholino groups, 3-thiomorpholino groups, 4-thiomorpholino groups, 1-pyrrolidinyl groups, 2-pyrrolidinyl groups, 3-pyrrolidinyl groups, 1-tetrahydropiperazinyl groups, 2-tetrahydropiperazinyl groups, 3-tetrahydropiperazinyl groups, 1-piperidinyl groups, 2-piperidinyl groups, 3-piperidinyl groups, 1-pyrazolinyl groups, 3-pyrazolinyl groups, 4-pyrazolinyl groups, 5-pyrazolinyl groups, 1-piperidinyl groups, 2-piperidinyl groups, 3-piperidinyl groups, 4-piperidinyl groups, 2-thiazolidinyl groups, 3-thiazolidinyl groups, 4-thiazolidinyl groups, 1-imidazolidinyl groups, 2-imidazolidinyl groups, 4-imidazolidinyl groups, and 5-imidazolidinyl groups, and bicycles such as 3-1H-benzoimidazol-2-one groups, 3-(1-alkyl)-benzoimidazol-2-one groups, indolinyl groups, tetrahydroquinolinyl groups, tetrahydroisoquinolinyl groups, benzothiolane groups, benzodithian groups, and 1,3-dihydro-imidazol-2-one groups. In a heterocyclic group, one of the atoms in the ring can be bound to the rest of the portions of the molecule when possible.

As used herein, the term "unsaturated" means that a certain portion has one or more unsaturated units.

A "heteroaliphatic group", "heterocycle", "heterocyclic group", "heteroaryl group", or "arylene group", when substituted, can have a substituent on a heteroatom if substitutable.

If a group is "substituted", at least one hydrogen in the group is replaced with a group (substituent) other than hydrogen. The number of substituents thereof is not particularly limited, if substitutable, and is one or more. The descriptions for each group are also applicable when the group is a part of another group or a substituent thereof, unless specifically noted otherwise. If, for example, a C₁₋₆ alkyl group is substituted with a certain substituent, the number of carbons of the substituent is not included in the number of carbons of the alkyl group. The same applies to other groups.

As used herein, "amino acid" is used in the conventional meaning in the art, referring to a compound having a carboxyl group and amino group within a single molecule. Typically, an amino acid is an α amino acid, but an amino acid herein can also be an amino acid with a greater distance between a carboxyl group and amino group, such as a β amino acid and γ amino acid. Furthermore, an amino acid herein indicates an L enantiomer unless specifically noted otherwise. If an amino acid is a D enantiomer, D (or d) is appended in the front (e.g., D-Ala (or d-Ala)) or is indicated with a lower case alphabet (e.g., a). Representative examples of amino acids are provided below, but other amino acids are also intended herein.

### [Table 1]

**Table 1: Representative amino acids**

| Three letter code | One letter code | Name of amino acid |
|---|---|---|
| Gly | G | Glycine |
| Ala | A | Alanine |
| Val | V | Valine |
| Leu | L | Leucine |
| Ile | I | Isoleucine |
| Ser | S | Serine |
| Thr | T | Threonine |
| Met | M | Methionine |
| Cys | C | Cysteine |
| Phe | F | Phenylalanine |
| Tyr | T | Tyrosine |
| Trp | W | Tryptophan |
| His | H | Histidine |
| Lys | K | Lysine |
| Arg | R | Arginine |
| Glu | E | Glutamic acid |
| Asp | D | Aspartic acid |
| Gln | Q | Glutamine |
| Asn | N | Asparagine |
| Pro | P | Proline |

Exemplary amino acids other than those described in Table 1 include, but are not limited to, homoproline, phenylglycine, phenethylglycine, α-naphthylalanine, β-naphthylalanine, β-homotryptophan, quinolylalanine, homocysteine, penicillamine, sarcosine, ornithine, citrulline, hydroxyproline, modified forms of the naturally-occurring amino acids described above (modified amino acids), and modified forms of non-naturally-occurring amino acids (modified amino acids).

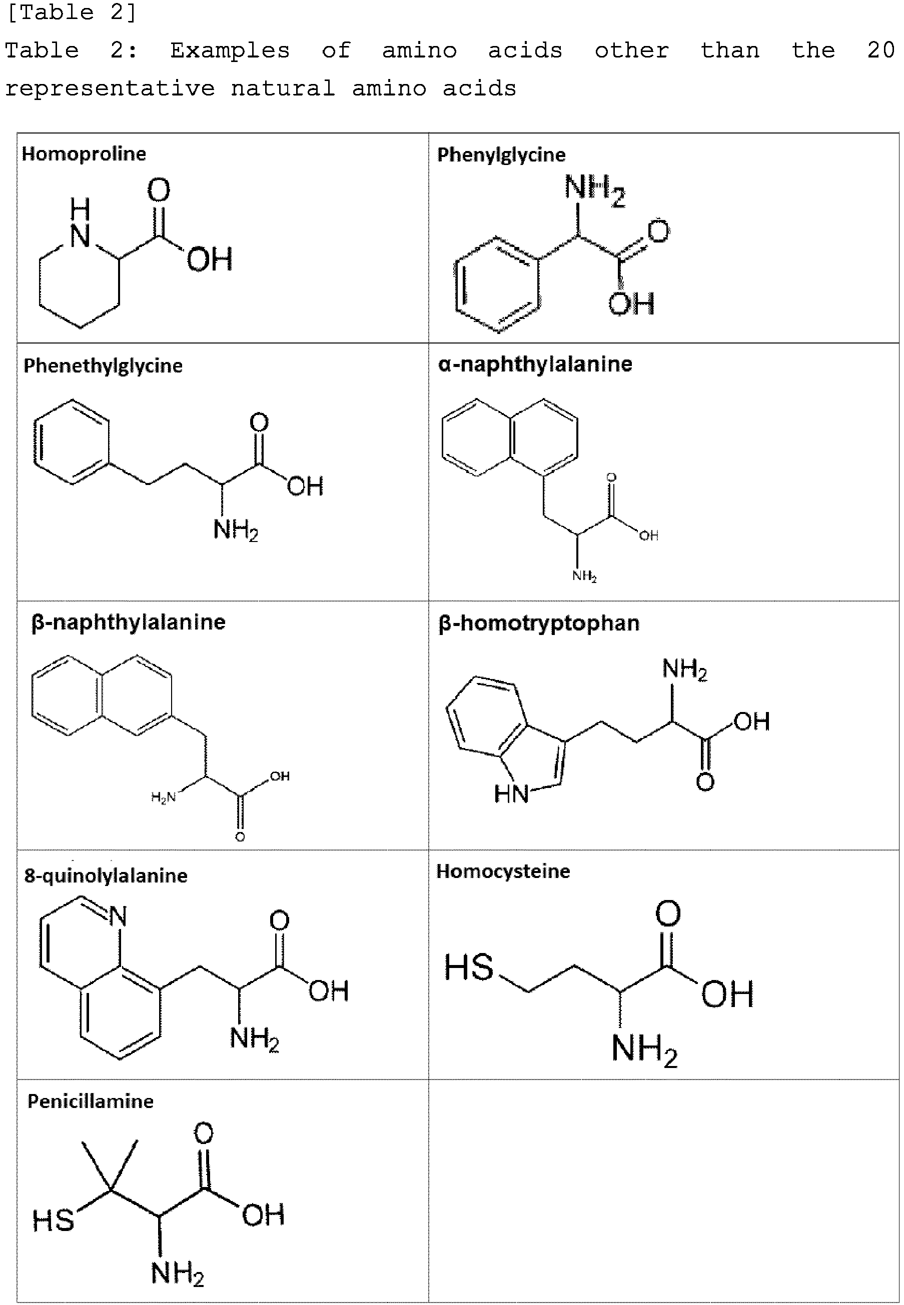

Examples of additional amino acids include the following modified amino acids.
*NH₂-CH(R')-COOH [wherein R' has a structure of (a bond or a linear or branched alkylene group or alkenylene group (e.g., C₁₋₅))- (an aryl group or heteroaryl group (e.g., 5-to 18-membered ring, and/or monocyclic, bicyclic, or tricyclic) optionally substituted with a substituent selected from the group consisting of a linear or branched alkyl group (e.g., C₁₋₃) optionally substituted with a halogen atom, a linear or branched methoxyl group (e.g., C₁₋₃) optionally substituted with a halogen atom, a halogen atom, and a hydroxyl group)]
*NH₂-CH(R')-COOH [wherein R' is -(a linear or branched alkyl group or alkenyl group (e.g., C₁₋₅))]
* 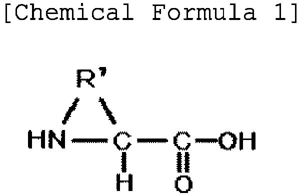
   wherein R' is a linear or branched alkylene group or alkenylene group (e.g., C₃₋₈)
   *NH₂-CH(R')-COOH [wherein R' is -(a linear or branched alkyl group or alkenyl group (e.g., C₁₋₈), wherein any one of the carbon atoms in the alkyl group or alkenyl group is optionally substituted with a sulfur atom, and may further have -SH]
   *NH₂-CH(R')-COOH [wherein R' is -(a linear or branched alkyl group or alkenyl group (e.g., C₁₋₁₀)), wherein any 1, 2, or 3 carbon atoms are optionally substituted with nitrogen atoms]

As used herein, "conservative substitution" refers to a substitution of an amino acid in a peptide with another amino acid with a similar property. The property of the peptide can be expected to be similar before and after a conservative substitution.

In one embodiment, a conservative substitution is a substitution of an amino acid in the following groups of amino acids with an amino acid within the same group.

*Group 1: glycine, alanine, valine, leucine, and isoleucine
*Group 2: serine and threonine
*Group 3: phenylalanine, tyrosine, tryptophan, homotryptophan, phenylglycine, phenethylglycine, benzothiazolylalanine, α-naphthylalanine, β-naphthylalanine, quinolylalanine, pyridylalanine, benzopyrazolylalanine, and amino acids with one or two hydrogen atoms on the aromatic ring thereof replaced with a methyl group, methoxyl group, hydroxyl group, or halogen atom
*Group 4: lysine, arginine, histidine, and ornithine
*Group 5: cysteine, methionine, homocysteine, and penicillamine
*Group 6: proline and homoproline
*Group 7: glutamic acid and aspartic acid
*Group 8: glutamine and asparagine

In one embodiment, a conservative substitution is a substitution with an amino acid including any modification among the followings: removal of any one of the -CH₂-moieties in the original amino acid, addition of a -CH₂- or -CH₃ moiety to any one portion of the amino acid, substitution of any one of the hydrogen atoms of the original amino acid with an alkyl group (e.g., methyl group), alkoxyl group (e.g., methoxyl group), hydroxyl group, or halogen atom, fusion or expansion of a ring by removal or addition of one carbon atom at any position of an aromatic ring in the original amino acid.

The peptide of the present disclosure or a salt or solvate thereof, or a prodrug thereof including a conservative substitution is for selectively antagonizing an erythropoietin (Epo) heteroreceptor in the presence of Epo (an Epo heteroreceptor selective inhibitor), and can also retain one or more characteristics from, for example, potent Epo heteroreceptor binding capability in the presence of Epo, weak Epo homodimer receptor binding capability in the presence of Epo, EpoR low affinity binding region selective binding or inhibitory capability, and low normal myeloid cell killing capability, in the similar manner as the original peptide. The peptide of the present disclosure or a salt or solvate thereof, or a prodrug thereof may also have cancer cell killing capability, reduction of normal cell killing capability, cancer cell killing capability at a low dosing frequency or the like, side effects, ability to migrate to a specific site, absorption/distribution/metabolism/excretion kinetics, bioavailability, and stability.

As used herein, "disulfide bond" for a peptide refers to an S-S bond formed within a single peptide molecule. Typically, a disulfide bond is formed between sulfur atoms that are present each in the two cysteine residues, but a bond between any sulfur atoms that are present within a peptide molecule is referred to as a disulfide bond herein. In one embodiment, a disulfide bond within a peptide molecule is formed between sulfur atoms that are present on a side chain of a peptide.

As used herein, "drug component" refers to any component that can be a constituent of a drug. Examples thereof include an active ingredient (component itself exhibiting efficacy), additive (component that is not expected to have efficacy in itself, but is expected to serve a certain role (e.g., excipient, lubricating agent, surfactant, or the like) when contained in a drug), adjuvant (enhances the efficacy of the active ingredient), and the like. A drug component can be an independent substance, or a combination of a plurality of substances or agents. A drug component can also encompass any combination such as a combination of an active ingredient and an additive, and a combination of an adjuvant and an active ingredient.

As used herein, "active ingredient" refers to a component that exerts the intended efficacy. An individual or a plurality of components can fall under an active ingredient.

As used herein, "additive" refers to any component that is not expected to have efficacy, but serves a certain role when contained in a drug. Examples thereof include pharmaceutically acceptable carriers, diluents, excipients, buffering agents, binding agents, blasting agents, diluents, flavoring agents, and lubricants.

A drug component used herein can be a purified component. The term "purified" as used herein refers to the presence of preferably at least 75% by weight, more preferably at least 85% by weight, still more preferably at least 95% by weight, and most preferably at least 98% by weight of the same type of biological agent.

As used herein, "subject" refers to an entity which is to be subjected to treatment or the like in the present disclosure.

As used herein, an "agent" is used in a broad sense, and may be any substance or other elements (e.g., energy such as light, radiation, heat, and electricity) as long as the intended object can be achieved. Examples of such a substance include, but are not limited to, proteins, polypeptides, oligopeptides, peptides, polynucleotides, oligonucleotides, nucleotides, nucleic acids (e.g., including DNA such as cDNA and genomic DNA, and RNA such as mRNA), polysaccharides, oligosaccharides, lipids, organic small molecules (e.g., hormones, ligands, information transmitting substances, organic small molecules, molecules synthesized by combinatorial chemistry, small molecules which can be utilized as a pharmaceutical product (e.g., a low molecular weight ligand), and the like), and composite molecules thereof.

As used herein, "treat (treatment or treating)" refers to the prevention of exacerbation, preferably maintaining of the current condition, more preferably alleviation, and still more preferably disappearance of a condition or disorder in case of such a condition or disorder, including being capable of exerting an effect of improving or preventing a condition of a patient or one or more symptoms accompanying the condition. Preliminary diagnosis with suitable therapy is referred to as "companion therapy" and a diagnostic agent therefor may be referred to as "companion diagnostic agent".

As used herein, "therapeutic drug (agent)" broadly refers to any agent that can treat a condition of interest. In one embodiment of the present disclosure, "therapeutic drug" can be a pharmaceutical composition comprising an active ingredient and one or more pharmaceutically acceptable carriers. A pharmaceutical composition can be manufactured by any method that is known in the technical field of pharmaceutical science, for example, by mixing an active ingredient with the carrier described above. The mode of use of a therapeutic drug is not limited, as long as the drug can be used for treatment. A therapeutic drug can be an active ingredient alone, or a mixture of an active ingredient with any component. The form of the carrier described above is not limited. Examples thereof include solids and liquids (e.g., buffer).

As used herein, "prevention" or "prophylaxis" refers to the action of taking a measure against a disease or disorder from being in such a condition prior to being in such a condition. It is possible to use the agent of the present disclosure to perform diagnosis, and if necessary use the agent of the present disclosure to prevent or take measures to prevent the disease or disorder.

As used herein, "prophylactic drug (agent)" broadly refers to any agent capable of preventing a condition of interest.

As used herein, "kit" refers to a unit providing parts to be provided (e.g., test drug, diagnostic drug, therapeutic drug, label, user manual, and the like) which are generally separated into two or more segments. Such a kit form is preferred when providing a composition, which should not be provided in a mixed state for stability or the like and is preferably used by mixing immediately prior to use. Such a kit advantageously comprises an instruction or user manual describing how the provided parts (e.g., test drug, diagnostic drug, and therapeutic drug) are used or how the reagent should be processed.

As used herein, "instruction" is a document with an explanation of the method of use of the present disclosure for a physician or other users. The instruction has an instructive description for administration of a drug of the present disclosure or the like. Further, an instruction may have a description instructing the administering manner (e.g., by oral administration, injection, or the like). The instruction is prepared in accordance with a format specified by the regulatory agency of the country in which the present disclosure is practiced (e.g., the Ministry of Health, Labour and Welfare in Japan, Food and Drug Administration (FDA) in the U.S. or the like), with an explicit description showing approval by the regulatory agency. The instruction is a so-called package insert and is typically provided in, but not limited to, paper media. The instructions may also be provided in a form such as electronic media (e.g., web sites provided on the Internet or emails).

As used herein, "diagnosis" refers to identifying various parameters associated with a disease, disorder, condition, or the like in a subject to determine the current or future state of such a disease, disorder, or condition. As used herein, "diagnosis" when narrowly defined refers to diagnosis of the current state, but when broadly defined includes "early diagnosis", "predictive diagnosis", "prediagnosis", and the like.

A procedure of formulation as a drug or the like of the present disclosure is known in the art and is described, for example, in the Japanese Pharmacopoeia, U.S. Pharmacopoeia, and other countries' pharmacopoeias. Thus, those skilled in the art can determine the amount to be used from the descriptions herein without undue experimentation.

### (Description of preferred embodiments)

Preferred embodiments of the present disclosure are described below. Embodiments described below are provided to facilitate the understanding of the present disclosure. It is understood that the scope of the present disclosure should not be limited to the following descriptions. Thus, it is apparent that those skilled in the art can make appropriate modifications within the scope of the present disclosure by referring to the descriptions herein. Those skilled in the art can appropriately combine any of the embodiments.

### <Structure of anti-EpoR peptide>

In one aspect, the present disclosure provides an anti-EpoR peptide for selectively antagonizing an erythropoietin (Epo) heteroreceptor in the presence of Epo. In a specific embodiment, the peptide of the present disclosure has one or more improved characteristics compared to CH₃-CO-SCHFGPLTWVCK-NH₂ (SEQ ID NO: 9), but the present disclosure is not limited thereto. Examples of such a characteristic that the pepetide can have include strengthened heteroreceptor selectivity in selective antagonism (erythropoietin (Epo) heteroreceptor selective inhibitor) of an Epo heteroreceptor in the presence of Epo, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure or a salt or solvate thereof, or a prodrug thereof may also have efficacy such cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, and the like.

Since the present disclosure provides a technology that can selectively antagonize an Epo heteroreceptor, a disease, disorder, or symptom characterized by the presence of a cell expressing an Epo heteroreceptor can be treated or prevented.

In this regard, "disease, disorder, or symptom characterized by the presence of a cell expressing an Epo heteroreceptor" refers to a disease, disorder, or symptom characterized by the presence of at least one cell expressing an Epo heteroreceptor. In one embodiment, "disease, disorder, or symptom characterized by the presence of a cell expressing an Epo heteroreceptor" is characterized by detection of a cell expressing 1 or more, 3 or more, 10 or more, 30 or more, 100 or more, 300 or more, or 1000 or more Epo heteroreceptors per cell on the cell surface, wherein the number of expressed Epo heteroreceptors can be estimated by any suitable means such as measurement of fluorescence intensity by a fluorescently labeled antibody for an Epo heteroreceptor. In one embodiment, "disease, disorder, or symptom characterized by the presence of a cell expressing an Epo heteroreceptor" is characterized by 0.1% or more, 0.3% or more, 1% or more, 3% or more, 10% or more, 30% or more, 50% or more, or 80% or more cells among 100 to 10000 cells at the center of a lesion site expressing an Epo heterereceptor on the cell surface, wherein the number of cells expressing an Epo heteroreceptor can be estimated by any suitable means such as FACS measurement using a fluorescently labeled antibody for an Epo heteroreceptor.

Examples of "disease, disorder, or symptom characterized by the presence of a cell expressing an Epo heteroreceptor" include, but are not limited to, a proliferative disease, rheumatism, diabetic retinopathy, keloid, and adenomyosis.

In the present disclosure, whether or not "selectively antagonizing an erythropoietin (Epo) heteroreceptor in the presence of Epo" is confirmed in a test of contacting a compound with an Epo heteroreceptor expressing cell (e.g., AsPC-1 cell or HCC1806 cell) in the presence of Epo by reduction in Jak2 phosphorylation when the compound is added relative to when the compound is not added.

The present disclosure relates to CH₃-CO-SCHFGPLTWVCK-NH₂ (SEQ ID NO: 9) or a modified peptide based thereon. A modified peptide can be a modified peptide based on a peptide having the basic structure of -[SCHFGPLTWVCK]- or a prodrug thereof or a salt thereof. For example, such a modified peptide or a prodrug thereof or a salt thereof comprises at least one modification at any part (including an amino terminus and carboxy terminus) of CH₃-CO-SCHFGPLTWVCK-NH₂ (SEQ ID NO: 9), and preferably comprises 2 modifications, 3 modifications, or 4 modifications, or modification on a modification. In a preferred embodiment, the peptide of the present disclosure comprises a modification at 1, 2, 3, or all of the portions corresponding to an amino terminus, carboxy terminus, F, and W in CH₃-CO-SCHFGPLTWVCK-NH₂ (SEQ ID NO: 9).

In another aspect, the anti-EpoR peptide of the present disclosure has a structure represented by the following formula (I).
Formula (I):
X¹-SCH(A¹) (A²) (A³) (A⁴) (A⁵) (A⁶)V(A⁷) (A⁸) -X² (SEQ ID NO: 1), wherein X¹ is an amino terminal side of the peptide, and X² is a carboxy terminal side of the peptide.

In another aspect, the present disclosure provides a composition comprising the peptide of the present disclosure, or a salt thereof, a solvate thereof, or a prodrug thereof. The peptide of the present disclosure, or a salt thereof, a solvate thereof, or a prodrug thereof, or a composition comprsing the same can be used in any suitable application.

In one embodiment of formula (I), A¹ is -NH-CH(R^{A1})-CO-, wherein R^{A1} has a structure of -(a bond, or a linear or branched alkylene group or alkenylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group). In one embodiment of formula (I), A¹ is -NH-CH(R^{A1})-CO-, wherein R^{A1} has a structure of (a bond or a linear or branched C₁₋₅ alkylene group or alkenylene group)-(a 5- to 18-membered monocyclic, bicyclic, or tricyclic aryl group or heteroaryl group optionally substituted with a substituent selected from the group consisting of a linear or branched C₁₋₃ alkyl group optionally substituted with a halogen atom, a linear or branched C₁₋₃ alkoxyl group optionally substituted with a halogen atom, a halogen atom, and a hydroxyl group), wherein any number of the substituents may be present on an aryl group or heteroaryl group at any position. In one embodiment of formula (I), A¹ is -NH-CH(R^{A1})-CO-, wherein R^{A1} is (a bond or a linear or branched C₁₋₃ alkylene group) - (a monocyclic or bicyclic aryl group or heteroaryl group (e.g., benzene, naphthalene, pyridine, thiophene, or benzothiophene) optionally substituted with a substituent selected from the group consisting of a linear or branched C₁₋₃ alkyl group (e.g., methyl group), a linear or branched C₁₋₃ alkoxyl group (e.g., methoxyl group), a halogen atom, and a hydroxyl group). In one embodiment of formula (I), A¹ is Met, or Phe, Tyr, phenylglycine, or phenethylglycine optionally substituted with a methyl group, a methoxyl group, a halogen atom, or a hydroxyl group, wherein any number of methyl groups, methoxyl groups, halogen atoms, or hydroxyl groups may be present on an aromatic ring at any position. In one embodiment of formula (I), a methyl group, methoxyl group, halogen atom, or hydroxyl group on an aromatic ring in A¹ may be present at a meta position (m-), para position (p-) or a combination thereof (3,4-). In one embodiment of formula (I), A¹ is Phe, Tyr, p-fluoro-Phe, p-chloro-Phe, m-chloro-Phe, 3,4-difluoro-Phe, phenylglycine, or phenethylglycine. Although not wishing to be bound by any theory, it is understood that the residue of A¹ is possibly present at a position corresponding to the lipophilic pocket of EpoR when an anti-EpoR peptide binds to the EpoR. Relatively high activity was observed when A¹ was p-fluoro-Phe, Tyr, phenethylglycine, or chloro-substituted Phe in an anti-EpoR peptide. A hydrogen bond is possibly formed between the residue of A¹ and EpoR. An anti-EpoR peptide having A¹ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), A² is -NH-CH(R^{A2})-CO-, wherein R^{A2} is -(H or a linear or branched C₁₋₅ alkyl group or alkenyl group). In one embodiment of formula (I), A² is Ala, D-Ala, or Gly. An anti-EpoR peptide having A² with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), A³ is -NH-CH(R^{A3})-CO-, wherein R^{A3} is -(H or a linear or branched C₁₋₅ alkyl group or alkenyl group). In one embodiment of formula (I), A³ is 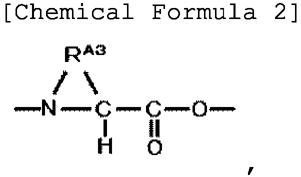 wherein R^{A3} is a linear or branched C₃₋₈ alkylene group or alkenylene group. In one embodiment of formula (I), A³ is Pro, homoproline, or Ala. An anti-EpoR peptide having A³ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc. Preferably, the peptide of formula (I) has at least one characteristic that is improved from a peptide having the structure of CH₃-CO-SCHFGPLTWVCK-NH₂ (SEQ ID NO: 9).

In one embodiment of formula (I), A⁴ is -NH-CH(R^{A4})-CO-, wherein R^{A4} is -(H or a linear or branched C₁₋₅ alkyl group or alkenyl group), wherein one of the carbon atoms in the alkyl group or alkenyl group may be replaced with a sulfur atom in one embodiment. In one embodiment of formula (I), A⁴ is Met, Leu, Ala, or Ile. In a specific embodiment of formula (I), A⁴ is Met or Leu. An anti-EpoR peptide having A⁴ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), A⁵ is -NH-CH(R^{A5})-CO-, wherein R^{A5} is -(H or a linear or branched C₁₋₅ alkyl group or alkenyl group), wherein one of the hydrogen atoms in the alkyl group or alkenyl group may be replaced with a hydroxyl group in one embodiment. In one embodiment of formula (I), A⁵ is Thr or Ala. An anti-EpoR peptide having A⁵ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduced normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), A⁶ is -NH-CH(R^{A6})-CO-, wherein R^{A1} has a structure of (a bond or a linear or branched alkylene group or alkenylene group)-(an aryl group or heteroaryl group optionally substituted with a substituent selected from the group consisting of a linear or branched alkyl group optionally substituted with a halogen atom, a linear or branched alkoxyl group (e.g., methoxyl group) optionally substituted with a halogen atom, a halogen atom, and a hydroxyl group). In one embodiment of formula (I), A⁶ is -NH-CH(R^{A6})-CO-, wherein R^{A6} is -(a bond or a linear or branched C₁₋₅ alkylene group or alkenylene group)-(a 5- to 18-membered monocyclic, bicyclic, or tricyclic aryl group or heteroaryl group optionally substituted with a substituent selected from the group consisting of a linear or branched C₁₋₃ alkyl group optionally substituted with a halogen atom, a linear or branched C₁₋₃ alkoxyl group optionally substituted with a halogen atom, a halogen atom, and a hydroxyl group), wherein any number of the substituents may be present on an aryl group or heteroaryl group at any position. In one embodiment of formula (I), A⁶ is -NH-CH(R^{A6})-CO-, wherein R^{A6} is -(a bond or a linear or branched C₁₋₃ alkylene group)-(a 5- to 18-membered monocyclic or bicyclic aryl group or heteroaryl group (e.g., benzene, indole, naphthalene, benzothiazole, and quinoline) optionally substituted with a substituent selected from the group consisting of a linear or branched C₁₋₃ alkyl group (e.g., methyl group), a linear or branched C₁₋₃ alkoxyl group (e.g., methoxyl group), a halogen atom, and a hydroxyl group). In one embodiment of formula (I), A⁶ is Met, or Trp, Phe, Tyr, β-homotryptophan, benzothiazolylalanine, α-naphthylalanine, β-naphthylalanine, or quinolylalanine optionally substituted with a methyl group, a methoxyl group, a halogen atom, or a hydroxyl group, wherein any number of methyl groups, methoxyl groups, hydroxyl groups, or halogen atoms, may be present on an aromatic ring at any position. Benzothiazolylalanine may be any of 2-benzothiazolylalanine, 4-benzothiazolylalanine, 5-benzothiazolylalanine, 6-benzothiazolylalanine, or 7-benzothiazolylalanine, and quinolylalanine may be any of 2-quinolylalanine, 3-quinolylalanine, 4-quinolylalanine, 5-quinolylalanine, 6-quinolylalanine, 7-quinolylalanine, or 8-quinolylalanine. In one embodiment of formula (I), A⁶ is Trp, Met, β-homotryptophan, 2-benzothiazolylalanine, α-naphthylalanine, β-naphthylalanine, 6-chloro-Trp, 5-chloro-Trp, 2-quinolylalanine, or 8-quinolylalanine. In a specific embodiment of formula (I), A⁶ is Trp, Met, 8-naphthylalanine, 2-quinolylalanine, 5-chloro-Trp, or 2-benzothiazolylalanine. In a more specific embodiment, A⁶ can be 2-benzothiazolylalanine. An anti-EpoR peptide having A⁶ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), A⁷ is -NH-CH(R^{A7})-CO-, wherein R^{A7} is -(H or a linear or branched C₁₋₈ alkyl group or alkenyl group), wherein one of the carbon atoms in the alkyl group or alkenyl group may be replaced with a sulfur atom in one embodiment of formula (I), and has an -SH group in one embodiment. In one embodiment, A⁷ is Cys, homocysteine, or penicillamine. An anti-EpoR peptide having A⁷ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), A⁸ is -NH-CH(R^{AB})-CO-, wherein R^{A8} is -(a linear or branched C₁₋₁₀ alkyl group or alkenyl group [wherein any 1, 2, or 3 carbon atoms are optionally replaced with nitrogen atoms]). In one embodiment of formula (I), A⁸ is Lys, Arg, or absent. An anti-EpoR peptide having A⁸ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), X¹ is hydrogen, a peptide consisting of any 1 to 3 amino acids, or -C(=O)R¹, wherein R¹ is selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, wherein each R² is independently selected from the group consisting of hydrogen, a hydroxyl group, halogen, a benzyl group optionally substituted with halogen or a hydroxyl group, - CH₃, -CH₂CH₃, -CH₂CH₂CH₃, -NH₂, -NHCH₃, -N(CH₃)₂, =O, -COOH, - OCH₃, and -OCH₂CH₃. In one embodiment, X¹ is -COOH, a propionyl group, or a benzoyl group or a phenylacetyl group optionally substituted with halogen. In one embodiment, X¹ is Gly-Thr-Tyr, Thr-Tyr, Tyr, Phe, Ala, Trp, or a moiety in which these amino terminuses are substituted with a linear or branched C₁₋₅ acyl group. In one embodiment, X¹ is a benzoyl group or a p-fluorophenylacetyl group. An anti-EpoR peptide having X¹ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), X² is hydrogen, a peptide consisting of any 1 to 3 amino acids, or -NR¹₂, wherein each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R², wherein each R² is independently selected from the group consisting of hydrogen, a hydroxyl group, halogen, a benzyl group optionally substituted with halogen or a hydroxyl group, - CH₃, -CH₂CH₃, -CH₂CH₂CH₃, -NH₂, -NHCH₃, -N(CH₃)₂, =O, -COOH, - OCH₃, and -OCH₂CH₃. In one embodiment, X² is Pro-Gln-Gly, Pro-Gln, Pro, or a moiety in which these carboxy terminuses are substituted with an amide. In one embodiment, X² is NH₂. An anti-EpoR peptide having X² with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (I), any two sulfur atoms in a peptide may form a disulfide bond. In one embodiment, a sulfur atom in A⁸ is forming a disulfide bond with another sulfur atom (e.g., sulfur atom in the 2^{nd} Cys from the amino terminus).

In an embodiment of formula (I), a compound represented by any combination of A¹ to A⁸, X¹, and X² is intended.

In one embodiment, the SCH(A¹)(A²) (A³) (A⁴) (A⁵) (A⁶)V(A⁷) (A⁸) (SEQ ID NO: 1) part in formula (I) has a structure of SCHFGPLTWVCK (SEQ ID NO: 2) with a replacement of 1, 2, 3, 4, 5, 6, 7, or 8 amino acids. In a more specific embodiment, the part has a structure of SCHFGPLTWVCK (SEQ ID NO: 2) with a replacement of 1, 2, or 3 amino acids. In one embodiment, the SCH(A¹)(A²) (A³) (A⁴) (A⁵) (A⁶)V(A⁷) (A⁸) part in formula (I) has a structure of SCHFGPLTWVCK (SEQ ID NO: 2) with a replacement at A¹, A⁶, or a combination thereof. In one embodiment, the SCH(A¹)(A²) (A³) (A⁴) (A⁵) (A⁶)V(A⁷) (A⁸) part in formula (I) is not SCHFGPLTWVCK (SEQ ID NO: 2).

In one embodiment, the anti-EpoR peptide of the present disclosure includes a modification at 1, 2, 3, or all of - Ac, -NH₂, F, and W in the structure of Ac-SCHFGPLTWVCK-NH₂ (SEQ ID NO: 9). In one embodiment, the anti-EpoR peptide of the present disclosure including a modification at F and/or W can have a different activity, e.g., for selectively antagonizing an Epo heteroreceptor in the presence of Epo (an erythropoietin (Epo) heteroreceptor selective inhibitor), or more improved activity (e.g., potent Epo heteroreceptor binding capability in the presence of Epo, weak Epo homodimer receptor binding capability in the presence of Epo, or EpoR low affinity binding region selective binding or inhibitory capability) than the peptide of Ac-SCHFGPLTWVCK-NH₂ (SEQ ID NO: 9). In one embodiment, the anti-EpoR peptide of the present disclosure including a modification at -Ac and/or -NH₂ can retain the activity (e.g., high heteroreceptor selectivity of those for selectively antagonizing an Epo heteroreceptor in the presence of Epo (an erythropoietin (Epo) heteroreceptor selective inhibitor), potent Epo heteroreceptor binding capability in the presence of Epo, weak Epo homodimer receptor binding capability in the presence of Epo, or EpoR low affinity binding region selective binding or inhibitory capability) of a peptide free of the modification. In one embodiment, the anti-EpoR peptide of the present disclosure includes a modification at an amino acid other than -Ac, - NH₂, F, and W in the structure of Ac-SCHFGPLTWVCK-NH₂ (SEQ ID NO: 9), independently from the modifications at -Ac, - NH₂, F, and W, and such modifications can be possible while retaining the activity (e.g., high heteroreceptor selectivity of those for selectively antagonizing an Epo heteroreceptor in the presence of Epo (an erythropoietin (Epo) heteroreceptor selective inhibitor), potent Epo heteroreceptor binding capability in the presence of Epo, weak Epo homodimer receptor binding capability in the presence of Epo, or EpoR low affinity binding region selective binding or inhibitory capability) of a peptide free of the modifications. For example, a modification changing L to A in SEQ ID NO: 9 can improve the hydrophilicity of the peptide.

In one embodiment, the anti-EpoR peptide of the present disclosure is a peptide having a structure in which each amino acid in the SCH(A¹)(A²) (A³) (A⁴) (A⁵) (A⁶)V(A⁷) (A⁸) (SEQ ID NO: 1) part in formula (I) is replaced with D-amino acid, and the order of amino acids is reversed, wherein X¹ and X² are defined the same as above. Such a peptide can, for example, exhibit a behavior that is different in metabolism.

In one specific embodiment of formula (I), A² can be Gly, A³ can be Phe, A⁵ can be Thr, A⁷ can be Cys, and/or A⁸ can be Lys. In one specific embodiment of formula (I), A¹ can be Tyr, p-fluorophenylalanine, or phenethylglycine. In a more specific embodiment, A¹ can be Tyr. In a specific embodiment of formula (I), A⁴ can be Leu or Met. In a more specific embodiment, A⁴ can be Met. In one specific embodiment of formula (I), A⁶ can be Trp, Met, 8-naphthylalanine, 2-quinolylalanine, 5-chloro-Trp, or 2-benzothiazolylalanine. In a more specific embodiment of formula (I), A⁶ can be 2-benzothiazolylalanine.

In one embodiment, the anti-EpoR peptide of the present disclosure has the structure represented by the following formula (II):
Formula (II):

X¹-kcvwtl(B¹)Gfhcs-X²,

wherein X¹ is an amino terminal side of the peptide, and X² is a carboxy terminal side of the peptide].

In one embodiment of formula (II), B¹ is Gly or D-proline. An anti-EpoR peptide having B¹ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (II), X¹ is hydrogen, a peptide consisting of any 1 to 3 amino acids, or -C(=O)R¹, wherein R¹ is selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, wherein each R² is independently selected from the group consisting of hydrogen, a hydroxyl group, halogen, a benzyl group optionally substituted with halogen or a hydroxyl group, - CH₃, -CH₂CH₃, -CH₂CH₂CH₃, -NH₂, -NHCH₃, -N(CH₃)₂, =O, -COOH, - OCH₃, and -OCH₂CH₃. In one embodiment, X¹ is -COOH, a propionyl group, or a benzoyl group or a phenylacetyl group optionally substituted with halogen. In one embodiment, X¹ is Gly-Thr-Tyr, Thr-Tyr, Tyr, Phe, Ala, Trp, or a moiety in which these amino terminuses are substituted with a linear or branched C₁₋₅ acyl group. In one embodiment, X is a benzoyl group or a p-fluorophenylacetyl group. An anti-EpoR peptide having X¹ with a structure within the scope described above is for selectively antagonizing an Epo heteroreceptor in the presence of Epo (an erythropoietin (Epo) heteroreceptor selective inhibitor), and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment of formula (II), X² is hydrogen, a peptide consisting of any 1 to 3 amino acids, or -NR¹₂, wherein each R¹ is selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R², and wherein each R² is independently selected from the group consisting of hydrogen, a hydroxyl group, halogen, benzyl optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃. In one embodiment, X² is Pro-Gln-Gly, Pro-Gln, Pro, or a moiety in which these carboxy terminuses are substituted with amide. In one embodiment, X² is NH₂. An anti-EpoR peptide having X² with a structure within the scope described above is for selectively antagonizing an Epo heteroreceptor in the presence of Epo (an erythropoietin (Epo) heteroreceptor selective inhibitor), and can retain one or more characteristics from, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. in the same manner as the original peptide. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment, the anti-EpoR peptide of the present disclosure is a peptide having the structure specified above having any number (e.g., 1, 2, 3, 4, 5, or 6) of amino acids replaced from an L form to a D form. In one embodiment, amino acids corresponding to those other than F and W in Ac-SCHFGPLTWVCK-NH₂ (SEQ ID NO: 9) are D-amino acids. Although not wishing to be bound by any theory, the sheet structure formed by a peptide can change by replacing some of the amino acids from an L-form to a D-form, thus D-amino acids may be introduced to retain the sheet structure. A peptide with such replacement of L-forms with D-forms is for selectively antagonizing an Epo heteroreceptor in the presence of Epo (an erythropoietin (Epo) heteroreceptor selective inhibitor), and can retain one or more characteristics from, for example, potent Epo heteroreceptor binding capability in the presence of Epo, weak Epo homodimer receptor binding capability in the presence of Epo, EpoR low affinity binding region selective binding or inhibitory capability, and low normal myeloid cell killing capability, in the same manner as the original peptide. The peptide of the present disclosure etc. may further have efficacy such as cancer cell killing capability, reduction in normal cell killing capability, or cancer cell killing capability at a low dosing frequency, side effect, ability to migrate to a specific site, absorption/distribution/metabolism/excretion kinetics, bioavailability, stability, etc. In one embodiment, amino acids corresponding to F and W in Ac-SCHFGPLTWVCK-NH₂ (SEQ ID NO: 9) can be D-amino acids. In such an embodiment, the peptide activity can change.

In one embodiment, the anti-EpoR peptide of the present disclosure is a peptide having a structure specified above having conservative substitutions of any number (e.g., 1, 2, 3, 4, 5, or 6) of amino acids. Such a peptide comprising a conservative substitution is for selectively antagonizing an Epo heteroreceptor in the presence of Epo (an erythropoietin (Epo) heteroreceptor selective inhibitor), and can retain one or more characteristics from, for example, potent Epo heteroreceptor binding capability in the presence of Epo, weak Epo homodimer receptor binding capability in the presence of Epo, EpoR low affinity binding region selective binding or inhibitory capability, and low normal myeloid cell killing capability in the same manner as the original peptide. The peptide of the present disclosure etc. may further have efficacy such as cancer cell killing capability, reduction in normal cell killing capability, cancer cell killing capability at a low dosing frequency or the like, side effects, ability to migrate to a specific site, absorption/distribution/metabolism/excretion kinetics, bioavailability, stability, etc.

In one embodiment, the anti-EpoR peptide of the present disclosure is a compound having one of the following structures (wherein X¹ and X² are defined in the same as above).
X¹-SCHFGPLTWVCK-X² [SEQ ID NO: 2]
X¹-SCH(p-fluoro-Phe)GPLTWVCK-X²
X¹-SCH(p-chloro-Phe)GPLTWVCK-X²
X¹-SCH(m-chloro-Phe)GPLTWVCK-X²
X¹-SCH(3,4-difluoro-Phe)GPLTWVCK-X²
X¹-SCHYGPLTWVCK-X² [SEQ ID NO: 3]
X¹-SCH(phenylglycine)GPLTWVCK-X²
X¹-SCH(phenethylglycine)GPLTWVCK-X²
X¹-SCHFAPLTWVCK-X² [SEQ ID NO: 4]
X¹-SCHFaPLTWVCK-X²
X¹-SCHFGALTWVCK-X² [SEQ ID NO: 5]
X¹-SCHFG(homoproline)LTWVCK-X²
X¹-SCHFGPATWVCK-X² [SEQ ID NO: 6]
X¹-SCHFGPMTWVCK-X² [SEQ ID NO: 7]
X¹-SCHFGPLTMVCK-X² [SEQ ID NO: 8]
X¹-SCHFGPLT(6-chloro-Trp)VCK-X²
X¹-SCHFGPLT(5-chloro-Trp)VCK-X²
X¹-SCHFGPLT(β-homotryptophan)VCK-X²
X¹-SCHFGPLT(α-naphthylalanine)VCK-X²
X¹-SCHFGPLT(β-naphthylalanine)VCK-X²
X¹-SCHFGPLT(8-quinolylalanine)VCK-X²
X¹-SCHFGPLT(2-quinolylalanine)VCK-X²
X¹-SCHFGPLT(2-benzothiazolylalanine)VCK-X²
X¹-SCHFGPLTWV(homocysteine)K-X²
X¹-SCHFGPLTWV(penicillamine)K-X²
X¹-SCH(3,4-difluoro-Phe)GPLT(2-benzothiazolylalanine)VCK-X²
X¹-SCH(p-fluoro-Phe)GPLT(2-benzothiazolylalanine)VCK-X²
X¹-SCHYGPLT(2-benzothiazolylalanine)VCK-X²
X¹-SCH(3,4-difluoro-Phe)GPLT(2-quinolylalanine)VCK-X²
X¹-SCH(p-fluoro-Phe)GPLT(2-quinolylalanine)VCK-X²X¹-SCHYGPLT(2-quinolylalanine)VCK-X²
X¹-SCH(3,4-difluoro-Phe)GPLT(8-quinolylalanine)VCK-X²
X¹-SCH(p-fluoro-Phe)GPLT(8-quinolylalanine)VCK-X²X¹-SCHYGPLT(8-quinolylalanine)VCK-X²
X¹-kcvwtlpGfhcs-X²
X¹-kcvwtlGGfhcs-X²
X¹-SCH(4-pyridylalanine)GPLTWVCK-X²
X¹-SCH(3-pyridylalanine)GPLTWVCK-X²
X¹-SCH(p-methoxy-Phe)GPLTWVCK-X²
X¹-SCH(m-methoxy-Phe)GPLTWVCK-X²
X¹-SCH(m-hydroxy-Phe)GPLTWVCK-X²
X¹-SCH(1-naphthylalanine)GPLTWVCK-X²
X¹-SCH(2-naphthylalanine)GPLTWVCK-X²
X¹-SCHFGPLT(2-benzothiazolylalanine)VCK-X²
X¹-SCHFGPLT(3-benzothiazolylalanine)VCK-X²
X¹-SCHYGPMT(2-benzothiazolylalanine)VCK-X²

In one embodiment, the anti-EpoR peptide of the present disclosure is a compound having one of the following structures (wherein Ac is an acetyl group).
Ac-SCHFGPLTWVCK-NH₂ [SEQ ID NO: 9]
   (benzoyl)-SCHFGPLTWVCK-NH₂
   (p-fluorophenylacetyl)-SCHFGPLTWVCK-NH₂
   (propionyl)-SCHFGPLTWVCK-NH₂
Ac-SCH(p-fluoro-Phe)GPLTWVCK-NH₂
Ac-SCH(p-chloro-Phe)GPLTWVCK-NH₂
Ac-SCH(m-chloro-Phe)GPLTWVCK-NH₂
Ac-SCH(3,4-difluoro-Phe)GPLTWVCK-NH₂
Ac-SCHYGPLTWVCK-NH₂ [SEQ ID NO: 10]
Ac-SCH(phenylglycine)GPLTWVCK-NH₂
Ac-SCH(phenethylglycine)GPLTWVCK-NH₂
Ac-SCHFAPLTWVCK-NH₂ [SEQ ID NO: 11]
Ac-SCHFaPLTWVCK-NH₂
Ac-SCHFGALTWVCK-NH₂ [SEQ ID NO: 12]
Ac-SCHFG(homoproline)LTWVCK-NH₂
Ac-SCHFGPATWVCK-NH₂ [SEQ ID NO: 13]
Ac-SCHFGPMTWVCK-NH₂ [SEQ ID NO: 14]
Ac-SCHFGPLTMVCK-NH₂ [SEQ ID NO: 15]
Ac-SCHFGPLT(6-chloro-Trp)VCK-NH₂
Ac-SCHFGPLT(5-chloro-Trp)VCK-NH₂
Ac-SCHFGPLT(β-homotryptophan)VCK-NH₂
Ac-SCHFGPLT(α-naphthylalanine)VCK-NH₂
Ac-SCHFGPLT(β-naphthylalanine)VCK-NH₂
Ac-SCHFGPLT(8-quinolylalanine)VCK-NH₂
Ac-SCHFGPLT(2-quinolylalanine)VCK-NH₂
Ac-SCHFGPLT(2-benzothiazolylalanine)VCK-NH₂
Ac-SCHFGPLTWV(homocysteine)K-NH₂
Ac-SCHFGPLTWV(penicillamine)K-NH₂
Ac-SCH(3,4-difluoro-Phe)GPLT(2-benzothiazolylalanine)VCK-NH₂
Ac-SCH(p-fluoro-Phe)GPLT(2-benzothiazolylalanine)VCK-NH₂
Ac-SCHYGPLT(2-benzothiazolylalanine)VCK-NH₂
Ac-SCH(3,4-difluoro-Phe)GPLT(2-quinolylalanine)VCK-NH₂
Ac-SCH(p-fluoro-Phe)GPLT(2-quinolylalanine)VCK-NH₂
Ac-SCHYGPLT(2-quinolylalanine)VCK-NH₂
Ac-SCH(3,4-difluoro-Phe)GPLT(8-quinolylalanine)VCK-NH₂
Ac-SCH(p-fluoro-Phe)GPLT(8-quinolylalanine)VCK-NH₂
Ac-SCHYGPLT(8-quinolylalanine)VCK-NH₂
Ac-kcvwtlpGfhcs-NH₂
Ac-kcvwtlGGfhcs-NH₂
Ac-SCH(4-pyridylalanine)GPLTWVCK-NH₂
Ac-SCH(3-pyridylalanine)GPLTWVCK-NH₂
Ac-SCH(p-methoxy-Phe)GPLTWVCK-NH₂
Ac-SCH(m-methoxy-Phe)GPLTWVCK-NH₂
Ac-SCH(m-hydroxy-Phe)GPLTWVCK-NH₂
Ac-SCH(1-naphthylalanine)GPLTWVCK-NH₂
Ac-SCH(2-naphthylalanine)GPLTWVCK-NH₂
Ac-SCHFGPLT(2-benzothiazolylalanine)VCK-NH₂
Ac-SCHFGPLT(3-benzothiazolylalanine)VCK-NH₂
Ac-SCHYGPMT(2-benzothiazolylalanine)VCK-NH₂

In one embodiment, the peptide of the present disclosure is protected with a protecting group (e.g., C1-6 acyl group such as a formyl group or an acetyl group, or the like). For example, a protecting group is introduced to OH, NH₂, SH, or the like on a side chain of an amino acid within a molecule. In one embodiment, a sugar chain or a PEG chain may be bound to the peptide of the present disclosure.

The peptide of the present disclosure can be provided in a form of salt. A salt can be either a base addition salt or an acid addition salt, but is preferably a physiologically acceptable acid addition salt. Examples of acid addition salt include salts with an inorganic acid (e.g., hydrochloric acid, phosphoric acid, hydrobromic acid, or sulfuric acid), salts with an organic acid (e.g., acetic acid, formic acid, propionic acid, fumaric acid, maleic acid, succinic acid, tartaric acid, citric acid, malic acid, oxalic acid, benzoic acid, methanesulfonic acid, benzenesulfonic acid, aspartic acid, glutamic acid, or the like), and the like. Examples of base addition salt include salts with an inorganic base (sodium, potassium, calcium, magnesium, aluminum, ammonium, or the like) and salts with an organic base (e.g., trimethylamine, triethylamine, pyridine, picoline, 2,6-lutidine, ethanolamine, diethanolamine, triethanolamine, cyclohexylamine, dicyclohexylamine, N,N'-dibenzylethylenediamine, lysine, arginine, histidine, or the like).

The peptide of the present disclosure can be in a form of a solvate. A solvent is not particularly limited, as long as it is pharmaceutically acceptable. Examples thereof include water, ethanol, glycerol, acetic acid, and the like.

The peptide of the present disclosure can be prepared as a prodrug. As a prodrug, a compound that is converted into the peptide of the present disclosure after an enzymatic reaction (oxidation, reduction, hydrolysis, or the like) or a reaction with stomach acid or the like under a physiological condition in vivo can be used. In one embodiment, examples of the prodrug of the present disclosure include compounds wherein an amino group of the peptide of the present disclosure is acylated, alkylated, or phosphorylated (e.g., eicosanoylated, alanylated, pentylaminocarbonylated, (5-methyl-2-oxo-1,3-dioxolen-4-yl)methoxycarbonylated, tetrahydrofuranylated, pyrrolidylmethylated, pivaloyloxymethylated, or tert-butylated compounds and the like), compounds wherein a hydroxyl group of the peptide of the present disclosure is acylated, alkylated, phosphorylated, or borated (e.g., acetylated, palmitoylated, propanoylated, pivaloylated, succinylated, fumarylated, alanylated, or dimethylaminomethylcarbonylated compounds and the like), and the like. These compounds can be manufactured from the peptide of the present disclosure by a known method.

The peptide of the present disclosure can be manufactured in accordance with a known peptide synthesis method. A peptide synthesis method can be, for example, either solid-phase synthesis or liquid-phase synthesis. Specifically, a peptide of interest can be manufactured by condensing an amino acid or a partial peptide that can constitute the peptide of the present disclosure with a remaining portion and eliminating a protecting group if the product has a protecting group. Examples of known methods of condension and elimination of protecting groups include the methods described in the following (1) to (3).
(1) Nobuo Izumiya et al., Pepuchido Gosei no Kiso to Jikken [Fundamentals and experiments of peptide synthesis], Maruzen (1985)
(2) The Fifth Series of Experimental Chemistry, Polymer chemistry, Vol. 26, Maruzen (2007)
(3) authored by Yasuyoshi Nogami, Patona Yakuhin Seizogaku [Partner Pharmaceutical Technochemistry], 2nd print, Nankodo (2012).

More specifically, a commercially available peptide synthesis resin can be used for the synthesis of the peptide of the present disclosure. For example, resin based on polystyrene can be used. To obtain a peptide with an amidated C-terminus, Fmoc-NH-SAL resin (Rink amide resin), MBHA resin, Sieber amide resin, PAL resin, Ramage amide resin, such resins with a linker or swelling agent such as polyethylene glycol introduced between an amino functional group and polystyrene, and the like can be used as the starting material resin. Such resin and amino acid with an α-amino group and a side chain functional group protected with a suitable protecting group (e.g., Boc or Fmoc) are used as the raw material and condensed onto the resin with various known conensing methods in accordance with the order of the sequence of the peptide of interest. A peptide is cut out from the resin at the end of the reaction while simultaneously eliminating various protecting groups, and optionally an intramolecular disulfide bond formation reaction is performed in a highly diluted solution to obtain the peptide of interest. Removal (elimination) of a protecting group and cleavage of a peptide from resin can be performed by a known method (e.g., acid treatment with trifluoroacetic acid). A peptide with an acylated N-terminus can be prepared through a reaction with acetic anhydride or the like. A crude peptide cleaved from resin can be purified using various known purification means.

### Amino acids used in peptide synthesis

Amino acids used in peptide synthesis (naturally-occurring amino acids and non-naturally-occurring amino acids) are available from any suitable supplier (e.g., Merck, Watanabe Chemical, Enamine Ltd., Bienta, ChemSpace, Sundia MediTech Company, Ltd., Combi-Blocks. Inc, AnalytiCon Discovery GmbH, PharmaBlock (Nanjing) R&D Co., Ltd, Chemexpress Co., Ltd., J&W Pharmlab, LLC, SAI Life Sciences Ltd, AstaTech, UkrOrgSyntez, Selleck Chemicals, WuXi AppTec, Vitas-M Laboratory, LTD., Bepharm, Life Chemicals Inc, Accela ChemBio Co., Ltd., Apollo Scientific Ltd, NovoChemy Ltd, Key Organics Ltd (Bionet Research), Matrix Scientific, Toronto Reserch chemicals Inc., MAYBRIDGE, Chem-Impex International, Inc., Allychem, Pharmeks Ltd., Biorelevant, AOBChem, ACRO Biosystems, Maison Chemical, Advanced ChemBlocks, Inc., Bellen, Microsource Discovery Systems, Inc., Taros Chemicals GmbH&Co.KG, Senn Chemicals AG, Sinocompound Catalysts Co., Ltd., Syngene International Ltd., BroadPharm, EDASA Scientific, Hi-tech chemistry corp, or the like). Amino acids (naturally-occurring amino acids and non-naturally-occurring amino acids) used in peptide synthesis can also be readily synthesized by those skilled in the art by using any known synthesis method (e.g., Strecker reaction (Strecker, A. (1850). Ueber die kunstliche Bildung der Milchsaure und einen neuen, dem Glycocoll homologen Korper". Ann. 75: 27-45., Strecker, A. (1854). "Ueber einen neuen aus Aldehyd-Ammoniak und Blausaure entstehenden Korper". Ann. 91: 349-351.)) A method of introducing a protecting group (e.g., Boc or Fmoc) and/or label (e.g., radioactive atom or biotin) to a given amino acid for synthesizing a peptide is also known. Those skilled in the art can readily apply such an amino acid modification.

### <Efficiency of anti-EpoR peptide>

### Properties of the peptide of the present disclosure

In one embodiment, the peptide of the present disclosure, or a salt or solvate thereof, or a prodrug thereof has one or more characteristics that are improved compared to CH₃-CO-SCHFGPLTWVCK-NH₂ (SEQ ID NO: 9). Examples of such a characteristic include lower IC₅₀ for human EpoR, potent Epo heteroreceptor binding capability in the presence of Epo, weak Epo homodimer receptor binding capability in the presence of Epo, high EpoR low affinity binding region selective binding and/or inhibitory capability, low normal myeloid cell killing capability, etc. Examples of such characteristics that the peptide of the present disclosure etc. may further have include ability to kill cancer cells (e.g., cancer cell line such as HepG2) at a lower concentration, ability of requiring a higher concentration to kill normal cells, ability to kill cancer cells at a lower dosing frequency, ability to migrate to a specific site (e.g., cancer tissue) at a greater quantity, improved absorption/distribution/metabolism/excretion kinetics, greater stability during storage, and the like. Although not wishing to be bound by any specific theory, binding selectivity of the peptide of the present disclosure, or a salt or solvate thereof, or a prodrug thereof to an Epo heteroreceptor or Epo homodimer receptor can be due to the selective affinity to a low affinity binding region of an EpoR. It is understood that Epo activates an Epo homodimer receptor by binding to both a low affinity binding region and a high affinity binding region of the Epo homodimer receptor. Meanwhile, in Epo heteroreceptors, it is understood that a high affinity binding region of an EpoR hardly functions or completely does not function, such that activation of an Epo heteroreceptor by Epo is primarily from binding to a low affinity binding region of an EpoR. For this reason, an Epo heteroreceptor can be selectively inhibited by selectively blocking a low affinity binding region of an EpoR.

A test for studying the properties described above is a test that can be readily conducted by those skilled in the art by referring to the descriptions herein, such as a contact test with cultured cell, dosing test in an animal model, blood kinetics test, and toxicological evaluation. Those skilled in the art can identify the anti-EpoR peptide of the present disclosure having at least one of the characteristics described above by preparing any peptide having the structure described above and conducting such a test on the prepared peptide.

In one embodiment for the anti-EpoR peptide of the present disclosure, if a myeloid cell (e.g., burst-forming unit-erythroid (BFU-E), colony-forming unit-erythroid (CFU-E), or proerythroblast) is incubated for 1 hour at 37°C in the presence of 10 U/mL of Epo and 100 µM of the anti-EpoR peptide of the present disclosure and the anti-EpoR peptide is fluorescently stained, the fluorescence intensity from the anti-EpoR peptide on a cell decreases, relative to incubation in the absence of Epo, at least about 10%, about 20%, about 30%, about 40%, about 50%, about 60%, about 70%, about 80%, about 90%, or about 95%.

In one embodiment for the anti-EpoR peptide of the present disclosure, if at least one type of human cancer cell line (e.g., breast cancer, pancreatic cancer, or leukemia cell line) is cultured for 72 hours in the presence of 100 U/mL of Epo and 50 µM of the anti-EpoR peptide of the present disclosure to evaluate the inhibitory effect by a WST assay, 50% or more, 60% or more, 70% or more, 80% or more, 90% or more, or 95% or more of the inhibitory effect is maintained, relative to culture in the absence of Epo.

In one embodiment, the anti-EpoR peptide of the present disclosure has an ability to kill HepG2 cells with a mean (e.g., mean for 10 segments; mean for n = 2, 3, or greater number of replicates as needed) of TB (Trypan blue) stained positive cell counts within a 1 mm² segment, which is equivalent to or greater than the result for a peptide of CH₃-CO-SCHFGPLTWVCK-NH₂ (SEQ ID NO: 9) when 1.0 to 1.5 × 10³ HepG2 cells are reacted for 24 hours in 400 µL of solution comprising 120 µM, 60 µM, 30 µM, or 15 µM of the peptide.

In one embodiment, the anti-EpoR peptide of the present disclosure can have a more potent ability to inhibit (e.g., kill) at least one type of human cancer cell line (e.g., liver cancer, pancreatic cancer, or leukemia cell line) than a peptide of CH₃-CO-SCHFGPLTWVCK-NH₂ (SEQ ID NO: 9) when evaluated by a WST assay. For example, the peptide exhibits a more potent inhibitory effect than a peptide of CH₃-CO-SCHFGPLTWVCK-NH₂ (SEQ ID NO: 9) when exposed to a human cancer cell line at 150 µM for 72 hours and evaluated by a WST assay.

In one embodiment, the anti-EpoR peptide of the present disclosure can have a more potent ability to inhibit (e.g., kill) at least one type of human cancer cell line (e.g., breast cancer cell line) than a peptide of CH₃-CO-SCHFGPLTWVCK-NH₂ (SEQ ID NO: 9) when the cell proliferation capacity is evaluated using the amount of ATP as an indicator. For example, the peptide exhibits a more potent inhibitory effect than a peptide of CH₃-CO-SCHFGPLTWVCK-NH₂ (SEQ ID NO: 9) when exposed to a human cancer cell line at 30 or 100 µM for 72 hours, and the cell proliferation capacity is evaluated using the amount of ATP as an indicator.

In one embodiment, the anti-EpoR peptide of the present disclosure preferentially or selectively binds to or inhibits a low affinity binding region of an EpoR (relative to a high affinity binding region). In one embodiment, if the anti-EpoR peptide of the present disclosure is evaluated for the inhibitory effect by a WST assay after culturing an HEK293T cell line overexpressing an EpoR, which is wild-type EpoR (SEQ ID NO: 17) introduced with an M174E mutation, for 3 days in the presence of 30 µM or 50 µM of the anti-EpoR peptide of the present disclosure, the inhibitory effect is selectively attenuated relative to a case using a cell line overexpressing a wild-type EpoR (SEQ ID NO: 17) or H138K/E141K mutant EpoR by, for example, 1% or more, 2% or more, 5% or more, 100 or more, 20% or more, or 30% or more.

In one embodiment, the anti-EpoR peptide of the present disclosure can have an ability where the CFU-E (Erythroid Colony Forming Unit) count is not significantly different, when bone marrow cells (e.g., mouse) seeded on a plate is exposed to an anti-EpoR peptide at a final concentration of 100 µM and recombinant human erythropoietin (1 U/mL) for 72 hours, relative to the result for a control of only recombinant human erythropoietin (1 U/mL).

In one embodiment, the anti-EpoR peptide of the present disclosure can have the ability to achieve at least one of the following when the anti-EpoR peptide is intraperitoneally administered for 2 or 4 weeks in a regimen of 2 or 3 times a week at 0.2 mg/day when a tumor volume (V) = length of tumor (L) × width of tumor (W)²/2 reaches about 150 to 200 mm³ in a 6-week-old male nude mouse (BALB/cSlc-nu/nu), to which about 5 × 10⁶ human pancreatic cancer derived AsPC-1 cells (ECACC, 96020930) have been transplanted subcutaneously to the right side on the back :
*the timing at which reduced tumor volume compared to an untreated control is observed is (e.g., 1 day or more, 2 days or more, 3 days or more, 4 days or more, 5 days or more, 6 days or more, or 7 days or more) earlier than when a peptide of CH₃-CO-SCHFGPLTWVCK-NH₂ (SEQ ID NO: 9) is administered;
*the tumor volume on day 21 from the start of administration is smaller (e.g., by about 100 or more, about 20% or more, about 30% or more, about 40% or more, or about 50% or more) than when a peptide of CH₃-CO-SCHFGPLTWVCK-NH₂ (SEQ ID NO: 9) is administered;
*the body weight of the mouse on day 21 from the start of administration is not different compared to an untreated control (e.g., the difference from the control is less than 10%, less than 7%, less than 5%, less than 3%, less than 2%, or less than 1%);
*the hematocrit value on day 21 from the start of administration is not different compared to an untreated control (e.g., the difference from the control is less than 30%, less than 20%, less than 15%, less than 10%, or less than 5%); and
*the blood hemoglobin concentration on day 21 from the start of administration is not different compared to an untreated control (e.g., the difference from the control is less than 30%, less than 20%, less than 15%, less than 10%, or less than 5%).

As specifically described in the Examples, the peptide of the present disclosure can suppress cancer cells even in the presence of Epo and dissociates with an EpoR in the presence of Epo in normal bone marrow cells, so that it is expected to be effectively useable for the treatment of various diseases including cancer while inducing little to no anemia.

### <Selective antagonist>

In one aspect, the present disclosure provides a composition comprising the peptide of the present disclosure or a salt thereof, or a solvate thereof, or a prodrug thereof. In a primary embodiment, the composition of the present disclosure is for selectively antagonizing an Epo heteroreceptor in the presence of Epo (an erythropoietin (Epo) heteroreceptor selective inhibitor). In a specific embodiment, the composition of the present disclosure has one or more characteristics from potent Epo heteroreceptor binding capability in the presence of Epo, weak Epo homodimer receptor binding capability in the presence of Epo, EpoR low affinity binding region selective binding or inhibitory capability, and low normal myeloid cell killing capability. The composition of the present disclosure may further have one or more characteristics from improved efficacy such as high cancer cell killing capability, low normal cell killing capability, cancer cell killing capability at a low dosing frequency or the like, reduction in side effects, ability to migrate specifically to a specific site, improved absorption/distribution/metabolism/excretion kinetics, improvement in bioavailability, and high stability. In one embodiment, the composition of the present disclosure can be used in treatment or prevention/prophylaxis of a proliferative disease, adenomyosis, or diabetic retinopathy. In one embodiment, the composition of the present disclosure is used in a subject for whom it is preferable that anemia is not induced, or a subject who is preferably not exposed to a risk of induction of anemia.

A peptide, or a modified peptide, or a prodrug thereof, or a salt thereof contained in the composition or selective antagonist of the present disclosure has substantially no affinity to, or does not antagonize, an Epo homodimer receptor in the presence of Epo (e.g., 10 U/mL). In this regard, 'substantially no' means no biologically significant affinity, which can be identified by activity that is at or below a measurement limit or specific threshold value in a specific measurement system described herein or the like. Further, 'does not antagonize' means not exhibiting biologically significant antagonism, which can be identified by activity that is at or below a measurement limit or specific threshold value in a specific measurement system described herein or the like. In one embodiment, the peptide of the present disclosure, or a modified peptide, or a prodrug thereof, or a salt thereof has affinity to an Epo homodimer receptor in the absence of Epo. For example, affinity to an Epo homodimer receptor in the absence of Epo can be confirmed if a myeloid cell (e.g., burst-forming unit-erythroid (BFU-E), colony-forming unit-erythroid (CFU-E), or proerythroblast) is incubated for 1 hour at 37°C in the absence of Epo and in the presence of 100 µM of the anti-EpoR peptide of the present disclosure, or a modified peptide, or a prodrug thereof, or a salt thereof and the anti-EpoR peptide is fluorescently stained, the fluorescence intensity from the anti-EpoR peptide on a cell decreases by at least about 10%, about 20%, about 30%, about 40%, about 50%, about 60%, about 70%, about 80%, about 90%, or about 95%, relative to a case using an Epo homodimer receptor non-expressing cell. In a preferred embodiment, an Epo heteroreceptor targeted by the present disclosure comprises an Epo receptor and a βc receptor.

Alternatively, in one embodiment of the present disclosure, the peptide of the present disclosure, or a modified peptide based on said peptide, or a prodrug thereof, or a salt thereof, in the presence of Epo, does not antagonize an Epo homodimer receptor, but antagonizes an Epo heteroreceptor comprising an Epo receptor and a βc receptor.

### <Detection of Epo heteroreceptor>

In one aspect, the present disclosure provides a method of selectively detecting an Epo heteroreceptor and means therefor. For example, means for selectively detecting an Epo heteroreceptor can be an Epo heteroreceptor recognizing antibody, a multispecific antibody that recognizes an EpoR and βcR, etc. The peptide of the present disclosure, a salt thereof, a solvate thereof, or a prodrug thereof (e.g., that specifically binds to a low affinity region of an EpoR) can also be used for this purpose. The presence of an Epo heteroreceptor (e.g., presence in a tissue other than the nerve) can be an indicator of cancer, and can be an indicator for selecting a therapy using the peptide of the present disclosure, a salt thereof, a solvate thereof, or a prodrug thereof.

### <Components of composition>

A composition comprising the peptide of the present disclosure or a salt thereof, or a solvate thereof, or a prodrug thereof can contain any additive. Examples of additive include carriers, excipients, lubricating agents, binding agents, disintegrants, solvents, solubilizers, suspending agents, isotonizing agents, buffering agents, analgesics, antiseptics, antioxidants, colorants, flavoring agents, adsorbents, and humectants.

Examples of excipients include lactose, white sugar, D-mannitol, starch, corn starch, crystalline cellulose, light anhydrous silicic acid, and the like. Examples of lubricating agents include magnesium stearate, calcium stearate, talc, colloidal silica, and the like. Examples of binding agents include crystalline cellulose, white sugar, D-mannitol, dextrin, hydroxypropyl cellulose, hydroxypropyl methylcellulose, polyvinylpyrrolidone, starch, sucrose, gelatin, methyl cellulose, sodium carboxymethyl cellulose, and the like. Examples of disintegrants include starch, carboxymethyl cellulose, calcium carboxymethyl cellulose, sodium carboxymethyl starch, L-hydroxypropyl cellulose, and the like.

Examples of solvents include injection solution, alcohol, propylene glycol, macrogol, sesame oil, corn oil, olive oil, and the like. Examples of solubilizers include polyethylene glycol, propylene glycol, D-mannitol, benzyl benzoate, ethanol, trisaminomethane, cholesterol, triethanolamine, sodium carbonate, sodium citrate, and the like. Examples of suspending agents include surfactants such as stearyl triethanolamine, sodium lauryl sulfate, lauryl aminopropionic acid, lecithin, benzalkonium chloride, benzethonium chloride, and glycerin monostearate, e.g., hydrophilic polymers such as polyvinyl alcohol, polyvinylpyrrolidone, sodium carboxymethyl cellulose, methyl cellulose, hydroxymethyl cellulose, hydroxyethyl cellulose, hydroxypropyl cellulose, and the like. Examples of isotonizing agents include glucose, D-sorbitol, sodium chloride, glycerin, D-mannitol, and the like. Examples of buffering agents include buffer such as phosphate, acetate, carbonate, and citrate and the like. Examples of analgesics include benzyl alcohol and the like. Examples of antiseptics include para-hydroxybenzoic acid ester, chlorobutanol, benzyl alcohol, phenethyl alcohol, dehydroacetic acid, sorbic acid, and the like. Examples of antioxidants include sulfite, ascorbic acid, α-tocopherol, and the like.

The prepared injection solution is generally filled in a suitable ampule. An injection composition can be dissolved in a conventional aqueous diluent upon administration and used as a solution. Examples of aqueous diluent include aqueous glucose solution, saline, Ringer's solution, nutrition supplement solution, and the like.

When an injection contains phosphoric acid or a salt thereof, the sodium phosphate or potassium phosphate concentration in the injection can be about 0.1 mM to 500 mM and preferably about 1 mM to 100 mM. Examples of methods that can be used to prepare a sterile formulation include, but are not limited to, a method of sterilizing the entire manufacturing process, a method of disinfecting with gamma rays, a method of adding an antiseptic, and the like.

In one embodiment, a composition comprising the peptide of the present disclosure or a salt thereof, or a solvate thereof, or a prodrug thereof can be provided as a kit. In one specific embodiment, a kit has (a) a container comprising the peptide of the present disclosure or a salt thereof, or a solvate thereof, or a prodrug thereof, or a composition comprising the same in a solution or lyophilized form, and optionally (b) a second container comprising a diluent or a reconstitution solution, and optionally (c) an instruction manual. In one embodiment, a kit can comprise one or more of (iii) a buffering agent, (iv) a diluent, (v) a filter, (vi) a needle, and (v) a syringe.

In one embodiment, the kit of the present disclosure comprises an instruction manual for use of the kit of the present disclosure. Examples of a suitable container include a bottle, vial (e.g., dual-chamber vial), syringe (dual-chamber syringe and the like), and test tube. Such a container can be made of various materials such as glass or plastic. In one embodiment, the kit of the present disclosure can comprise another container comprising a constituent element that is different from the peptide of the present disclosure or a salt thereof, or a solvate thereof, or a prodrug thereof.

### <Therapy, Prevention, etc.>

In another aspect, the composition of the present disclosure is particularly used in treating or preventing a disease, disorder, or symptom associated with an Epo heteroreceptor. Examples of the disease, disorder, or symptom associated with an Epo heteroreceptor include a proliferative disease, rheumatism, diabetic retinopathy, and adenomyosis. Examples of diseases that can be particularly suitably treated or prevented among proliferative diseases include glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, and leukemia.

In another aspect, the present disclosure provides a composition for use in treating or preventing a disease, disorder, or symptom characterized by the presence of a cell expressing an Epo heteroreceptor, comprising a selective antagonist of an Epo heteroreceptor.

Alternatively, the present disclosure provides a composition for use in treating or preventing a disease, disorder, or symptom characterized by the presence of a cell expressing an Epo heteroreceptor, comprising the peptide of the present disclosure or a salt thereof, or a solvate thereof, or a prodrug thereof. Examples of the disease, disorder, or symptom characterized by the presence of a cell expressing an Epo heteroreceptor include, but are not limited to, a proliferative disease, rheumatism, diabetic retinopathy, and adenomyosis. A disease, disorder, or symptom characterized by the presence of a cell expressing an Epo heteroreceptor can be identified by actually measuring the numerical value of Epo heteroreceptors in a subject or a target in a subject. Treatment or prevention can be also envisioned based on companion diagnosis that would decide to administer the composition of the present disclosure if the numerical value meets a certain level. The composition of the present disclosure can have one or more characteristics from improved efficacy such as high cancer cell killing capability, low normal cell killing capability, cancer cell killing capability at a low dosing frequency or the like, reduction in side effects, ability to migrate specifically to a specific site, improved absorption/distribution/metabolism/excretion kinetics, improvement in bioavailability, and high stability. In one embodiment, the composition of the present disclosure can be used in treating or preventing a proliferative disease, adenomyosis, or diabetic retinopathy. In one embodiment, the composition of the present disclosure is used in a subject for whom it is preferable that anemia is not induced, or a subject who is preferably not exposed to a risk of induction of anemia.

In one embodiment, the peptide of the present disclosure or a salt thereof, or a solvate thereof, or a prodrug thereof can be advantageously used as a combined drug. The peptide of the present disclosure or a salt thereof, or a solvate thereof, or a prodrug thereof can have an angiogenetic activity (e.g., on cells expressing an Epo homodimer receptor). For this reason, in one embodiment, the peptide of the present disclosure or a salt thereof, or a solvate thereof, or a prodrug thereof can exert a suppressive action on diseased tissue such as cancer tissue as well as a protective action on normal tissue (e.g., bone marrow tissue). In one embodiment, this can be advantageously used in combination with an angiogenesis inhibitor. The peptide of the present disclosure or a salt thereof, or a solvate thereof, or a prodrug thereof can also be advantageously used for a disease for which a subject has acquired, or at risk of acquiring, resistance (e.g., resistance acquired by cancer immunity) to another agent (e.g., anti-cancer agent). For this reason, in one embodiment, the peptide of the present disclosure or a salt thereof, or a solvate thereof, or a prodrug thereof can be used in combination with another agent (e.g., anticancer agent).

In one embodiment, an anticancer agent that can be used in combination with the peptide of the present disclosure or a salt thereof, or a solvate thereof, or a prodrug thereof includes an angiogenesis inhibitor, chemotherapeutic agent, platinum derivative, antimetabolite, topoisomerase inhibitor, microtubule inhibitor, anticancer antibiotics, molecularly targeted drug, etc. Examples of anticancer agents that can be used in combination with the peptide of the present disclosure or a salt thereof, or a solvate thereof, or a prodrug thereof in one emboidment include axicabtagene ciloleucel, L-asparaginase, oblimersen, talimogene laherparepvec, tisagenlecleucel, vismodegib, axitinib, asparaginase, atezolizumab, anagrelide, apalutamide, abiraterone, afatinib, aflibercept, aprepitant, abemaciclib, avelumab, alitretinoin, alectinib, alemtuzumab, ixazomib, idelalisib, inotuzumab, ipilimumab, ibritumomab, ibrutinib, imatinib, irinotecan, interferon gamma-1a, ulipristal, etirinotecan pegol, edotreotide, etoposide, entrectinib, epirubicin, everolimus, epoetin theta, epoetin beta, eribulin, erlotinib, elotuzumab, encorafenib, enzalutamide, oxaliplatin, osimertinib, obinutuzumab, ofatumumab, olaparib, olaratumab, carfilzomib, cabazitaxel, capmatinib, capecitabine, cabozantinib, carfilzomib, carmustine, quizartinib, gilteritinib, glasdegib, granisetron, crisantaspase, crizotinib, clofarabine, clofarabine, gefitinib, gemcitabine, gemtuzumab, goserelin, cobimetinib, samarium [153Sm] lexidronam pentasodium, thalidomide, cyclophosphamide, cytarabine, dinutuximab, streptozocin, sunitinib, cetuximab, cemiplimab, ceritinib, sonidegib, sorafenib, daunorubicin, dacarbazine, dacomitinib, dasatinib, dasiprotimut-t, tasonamine, dabrafenib, talazoparib, daratumumab, talaporfin sodium, darbepoetin alfa, darolutamide, thiotepa, tipiracil, tivozanib, tirabrutinib, thyrotropin alfa, tegafur/gimeracil/oteracil, degarelix, dexamethasone, decitabine, denosumab, tepotinib, temsirolimus, temozolomide, temoporfin, durvalumab, telotristat ethyl, doxorubicin, docetaxel, trastuzumab, trabectedin, trametinib, trifluridine, toremifene, nivolumab, nilotinib, necitumumab, netupitant, neratinib, nelarabine, nogitecan, paclitaxel, pazopanib, padeliporfin, panitumumab, panobinostat, palbociclib, palbociclib, palonosetron, vandetanib, pixantrone, hydroxycarbamide, binimetinib, binimetinib, vincristine, vinflunine, fosaprepitant, forodesine, busulfan, pralatrexate, brigatinib, plitidepsin, blinatumomab, fluorouracil, fluciclobine (18F), fludarabine, fulvestrant, prednisolone, plerixafor, brentuximab, florbetapir (18F), pegaspargase, bexarotene, pegfilgrastim, venetoclax, bevacizumab, pembrolizumab, vemurafenib, pemetrexed, pertuzumab, bendamustine, pembrolizumab, bosutinib, votumumab, ponatinib, pomalidomide, polatuzumab, bortezomib, borofaran (10B), masitinib, midostaurin, mifamurtide, mercaptopurine, mogamulizumab, ranimustine, lapatinib, ramucirumab, lanreotide, rituximab, ribociclib, leuprorelin, rucaparib, ruxolitinib, lutetium (177Lu) oxodotreotide, regorafenib, lenalidomide, calcium levofolinate, lenvatinib, romidepsin, lorlatinib, rolapitant, radium chloride (223Ra), and arsenic trioxide.

In one embodiment, an angiogenesis inhibitor that can be used in combination with the peptide of the present disclosure or a salt thereof, or a solvate thereof, or a prodrug thereof includes linomide, bevacizumab, angiostatin, razoxane, bevacizumab, ramucirumab, aflibercept, sunitinib, sorafenib, axitinib, cediranib, pazopanib, regorafenib, brivanib, and vandetanib.

In one embodiment, the application can be the prevention/prophylaxis or treatment of a proliferative disease (e.g., adenomyosis), rheumatism, keloid, or diabetic retinopathy. In one embodiment, the proliferative disease can be adenomyosis, malignant tumor or benign tumor (e.g., tumor such as primary, metastatic, or recurrent breast cancer, prostate cancer, pancreatic cancer, gastric cancer, lung cancer, large intestinal cancer (colon cancer, rectal cancer, or anal cancer), esophageal cancer, duodenal cancer, head and neck cancer (tongue cancer, pharyngeal cancer, laryngeal cancer, thyroid cancer), brain tumor, schwannoma, neuroblastoma, glioma, non-small cell lung cancer, small cell lung cancer, liver cancer, kidney cancer, bile duct cancer, uterine cancer (endometrial cancer or cervical cancer), ovarian cancer, bladder cancer, skin cancer, hemangioma, malignant lymphoma, malignant melanoma, bone tumor, angiofibroma, retinal sarcoma, penile cancer, pediatric solid tumor, Kaposi's sarcoma, AIDS-related Kaposi's sarcoma, maxillary sinus tumor, fibrous histiocytoma, leiomyosarcoma, rhabdomyosarcoma, liposarcoma, uterine fibroid, osteoblastoma, osteosarcoma, chondrosarcoma, cancerous mesothelioma, or leukemia). In one embodiment, a proliferative disease can be lung cancer, liver cancer, ovarian cancer, pancreatic cancer, kidney cancer, large intestinal cancer, melanoma, brain tumor, gastric cancer, or breast cancer. In one specific embodiment, a proliferative disease can be breast cancer, pancreatic cancer, liver cancer, malignant lymphoma, or leukemia (or a subpopulation consisting of any options thereof).

In one embodiment, the peptide of the present disclosure or a salt thereof, or a solvate thereof, or a prodrug thereof, or a composition comprising the same can treat a disease of interest in the presence of Epo, so that it can be advantageously used on a subject highly expressing Epo (optionally at a site of a disease). In one embodiment, the peptide of the present disclosure or a salt thereof, or a solvate thereof, or a prodrug thereof, or a composition comprising the same can be administered to a subject with a serum Epo concentration of several mIU/mL or greater (e.g., 3 mIU/mL or greater), 5 mIU/mL or greater, 10 mIU/mL or greater, 20 mIU/mL or greater, 30 mIU/mL or greater, 40 mIU/mL or greater, 50 mIU/mL or greater, 70 mIU/mL or greater, 100 mIU/mL or greater, 150 mIU/mL or greater, 200 mIU/mL or greater, 300 mIU/mL or greater, 400 mIU/mL or greater, or 500 mIU/mL or greater. A baseline value of serum Epo concentration can be 4.2 to 23.7 mIU/mL.

In one embodiment, the peptide of the present disclosure or a salt thereof, or a solvate thereof, or a prodrug thereof, or a composition comprising the same can be administered to a subject characterized by detection of 1 or more, 10 or more, 30 or more, or 100 or more cells expressing 1 or more, 3 or more, 10 or more, 30 or more, 100 or more, 300 or more, or 1000 or more Epo heteroreceptors per cell on the cell surface among 100 to 10000 cells at the center of a lesion site. In one embodiment, the peptide of the present disclosure or a salt thereof, or a solvate thereof, or a prodrug thereof, or a composition comprising the same can be administered to a subject characterized by 0.1% or more, 0.3% or more, 1% or more, 3% or more, 10% or more, 30% or more, 50% or more, or 80% or more cells among 100 to 10000 cells at the center of a lesion site expressing an Epo heterereceptor on the cell surface.

In another aspect, the peptide of the present disclosure, or a modified peptide based on said peptide, or a prodrug thereof, or a salt or solvate thereof provides a composition or medicament for improving an adverse action elicited by an anticancer agent in the presence of Epo. An adverse action can be any action known for each anticancer agent. Examples thereof include, but are not limited to, anemia.

In one embodiment, a subject administered with the peptide of the present disclosure, or a modified peptide, or a prodrug thereof, or a salt or a solvate thereof, or a composition comprising the same is a subject for whom it is preferable that anemia is not induced, or a subject who is preferably not exposed to a risk of induction of anemia. Since the peptide of the present disclosure can treat a disease of a subject without inducing anemia, such a peptide can be advantageously used especially for such a subject. In one embodiment, a subject for whom it is preferable that anemia is not induced, or a subject who is preferably not exposed to a risk of induction of anemia, can be a subject who has been diagnosed as having anemia. Examples of anemia include, but are not limited to, microcytic hypochromic anemia (iron-deficiency anemia, thalassemia, etc.), normocytic normochromic anemia (acute hemorrhage, hemolytic anemia, aplastic anemia, anemia due to hematopoietic tumor (leukemia, etc.), etc.), macrocytic normochromic anemia (pernicious anemia, myelodysplastic syndrome, etc.), renal anemia, hereditary spherocytosis, erythroenzymopathy, hereditary hemolytic anemia, paroxysmal nocturnal hemoglobinuria, disseminated intravascular coagulation syndrome, etc.

In one embodiment, a subject for whom it is preferable that anemia is not induced, or a subject who is preferably not exposed to a risk of induction of anemia, can have a hemoglobin concentration of 15 g/dl or less, 14 g/dl or less, 13 g/dl or less, 12 g/dl or less, 11 g/dl or less, 9 g/dl or less, 8 g/dl or less, 7 g/dl or less, 6 g/dl or less, or 5 g/dl or less. In one embodiment, a subject for whom it is preferable that anemia is not induced, or a subject who is preferably not exposed to a risk of induction of anemia, can be an adult male with a hemoglobin concentration of less than 13 g/dl, an adult female or child with a hemoglobin concentration of less than 12 g/dl, an elderly subject (e.g., 60 years old or older, 65 years old or older, 70 years old or older, 75 years old or older, or 80 years old or older) with a hemoglobin concentration of less than 11 g/dl.

In one embodiment, a subject for whom it is preferable that anemia is not induced, or a subject who is preferably not exposed to a risk of induction of anemia, can have a hematocrit value of 50% or less, 45% or less, 40% or less, 38% or less, 36% or less, 34% or less, 32% or less, 30% or less, 28% or less, 26% or less, 24% or less, 22% or less, or 20% or less.

In one embodiment, a subject for whom it is preferable that anemia is not induced, or a subject who is preferably not exposed to a risk of induction of anemia, can be a subject observed to have hemorrhage due to trauma, ulcer, etc (including internal bleeding) and/or hemolysis.

In one embodiment, a subject for whom it is preferable that anemia is not induced, or a subject who is preferably not exposed to a risk of induction of anemia can be a subject receiving treatment or prevention for anemia. Examples of treatment or prevention for anemia include transfusion, iron replacement therapy, vitamin B12 replacement therapy, folic acid replacement therapy, etc.

The peptide of the present disclosure or a salt thereof, or a solvate thereof, or a prodrug thereof, or a composition comprising the same can be used on any subject such as avian and mammals (e.g., mouse, rat, hamster, rabbit, cat, dog, cow, sheep, pig, monkey, human, and the like).

### <Use>

In one embodiment, the present disclosure provides use of the peptide of the present disclosure or a salt thereof, or a solvate thereof, or a prodrug thereof, or a composition or kit comprising the same, or a method of use in any suitable application. In a specific embodiment, the peptide of the present disclosure or a salt thereof, or a solvate thereof, or a prodrug thereof, or a composition or kit comprising the same is for selectively antagonizing an Epo heteroreceptor in the presence of Epo (an erythropoietin (Epo) heteroreceptor selective inhibitor) and can have, for example, potent Epo heteroreceptor binding capability in the presence of Epo, weak Epo homodimer receptor binding capability in the presence of Epo, EpoR low affinity binding region selective binding or inhibitory capability, low normal myeloid cell killing capability, etc. The peptide of the present disclosure or a salt thereof, or a solvate thereof, or a prodrug thereof, or a composition or kit comprising the same can further have one or more characteristics from improvement in efficacy such as high cancer cell killing capability, low normal cell killing capability, cancer cell killing capability at a low dosing frequency or the like, reduction in side effects, ability to specifically migrate to a specific site, improved absorption/distribution/metabolism/excretion kinetics, improvement in bioavailability, and high stability, and can therefore be used for the treatment or prevention/prophylaxis of proliferative disease (e.g., adenomyosis) or diabetic retinopathy. Thus, in one embodiment, the present disclosure provides a method for treating or preventing a proliferative disease (e.g., adenomyosis) or diabetic retinopathy in a subject (e.g., a subject for whom it is preferable that anemia is not induced, or a subject who is preferably not exposed to a risk of induction of anemia), comprising administering to the subject an effective amount of the peptide of the present disclosure or a salt thereof, or a solvate thereof, or a prodrug thereof. In another embodiment, the present disclosure provides use of the peptide of the present disclosure or a salt thereof, or a solvate thereof, or a prodrug thereof in the manufacture of a medicament for the treatment or prevention/prophylaxis of proliferative disease (e.g., adenomyosis) or diabetic retinopathy (e.g., in a subject for whom it is preferable that anemia is not induced, or a subject who is preferably not exposed to a risk of induction of anemia).

### Content

In a composition comprising the peptide of the present disclosure or a salt thereof, or a solvate thereof, or a prodrug thereof, the content of the peptide of the present disclosure or a salt thereof, or a solvate thereof, or a prodrug thereof can vary depending on the form of the composition, but can be, for example, about 0.1 to 100% by weight, about 0.5 to 50% by weight, about 1 to 30% by weight, about 5 to 20% by weight, about 10 to 99.9% by weight, about 20 to 90% by weight, about 0.1% by weight, about 0.2% by weight, about 0.5% by weight, about 1% by weight, about 2% by weight, about 5% by weight, about 10% by weight, about 20% by weight, or about 50% by weight with respect to the entire composition. The content of components other than the peptide of the present disclosure or a salt thereof, or a solvate thereof, or a prodrug thereof can vary depending on the form of the composition, but can be, for example, about 10 to 99.9% by weight or about 20 to 90% by weight with respect to the entire composition.

### Dosing regimen

The peptide of the present disclosure or a salt thereof, or a solvate thereof, or a prodrug thereof, or a composition comprising the same can be administered, for example, at a dose of about 0.005 to 50 mg, about 0.05 to 10 mg, or about 0.2 to 4 mg as the peptide of the present disclosure per 1 kg of body weight per day in a single or multiple administrations. The peptide of the present disclosure or a salt thereof, or a solvate thereof, or a prodrug thereof, or a composition or kit comprising the same can be administered at any suitable dosing frequency such as three times daily, twice daily, once daily, every other day, once every three days, once every four days, once every five days, biweekly, once every two weeks, once every three weeks, once every four weeks, once every two months, once every three months, once every four months, once every five months, once every six months, or once a year.

### Route of administration

A composition comprising the peptide of the present disclosure or a salt thereof, or a solvate thereof, or a prodrug thereof can be administered through any suitable route of administration, such as intravenous, topical intramuscular, subcutaneous, intradermal, transdermal, rectal, vaginal, oral mucosal, pulmonary mucosal, transnasal, transocular, or oral (enteric) route.

### Dosage form

A composition comprising the peptide of the present disclosure or a salt thereof, or a solvate thereof, or a prodrug thereof can be formulated into any suitable dosage form such as a solution, injection, patch, microneedle, suppository, sustained release agent, tablet (including sugar-coated tablet and film-coated tablet), powder, granule, and capsule (including soft capsule).

### Combination

The peptide of the present disclosure or a salt thereof, or a solvate thereof, or a prodrug thereof can be used in combination with any suitable agent. Examples of such an agent include endocrine therapeutic drugs, chemotherapeutic agents, immunotherapeutic agents (BRM), agents that inhibit the action of a cell growth factor, and the like.

In one embodiment, the peptide of the present disclosure or a salt thereof, or a solvate thereof, or a prodrug thereof can be used in combination with an agent which targets a molecule that is different from an EpoR. Examples of such an agent include angiogenesis inhibitors, anti-PD-1 antibodies, etc., which can exert a synergistic therapeutic effect.

In one embodiment, the peptide of the present disclosure or a salt thereof, or a solvate thereof, or a prodrug thereof can be used in combination with an anticancer agent. While many anticancer agents can exacerbate anemia, the peptide of the present disclosure or a salt thereof, or a solvate thereof, or a prodrug thereof can have an ability to not induce anemia, so that it can be combined with such an anticancer agent. Examples of anticancer agents include, but are not limited to, alkylating agent, platinum derivative, antimetabolite, topoisomerase inhibitor, microtubule inhibitor, anticancer antibiotics, molecularly targeted drug (e.g., kinase, Her2, EGFR (epidermal growth factor receptor), PI3K (phosphatidylinositol 3-kinase), mTOR (mammalian target of rapamycin), Akt, CDK (cyclin-dependent kinase), VEGFR (vascular endothelial growth factor receptor), PDGFR (platelet-derived growth factor receptor), FGFR (fibroblast growth factor receptor), c-Met, Raf, p38MAPK, CTLA-4, ALK, JAK, MEK (MAPK/ERK kinase), Hsp90, and histone deacetylase targeting agents), etc.

Examples of platinum derivatives include, but are not limited to, cisplatin, carboplatin, oxaliplatin, nedaplatin, satraplatin, and triplatin tetranitrate.

Examples of topoisomerase inhibitors include, but are not limited to, topotecan, irinotecan, nogitecan, doxorubicin, etoposide, levofloxacin, and ciprofloxacin.

Examples of microtubule inhibitors include, but are not limited to, vincristine, vinblastine, paclitaxel, docetaxel, and ixabepilone.

Examples of molecularly targeted drugs include, but are not limited to, gefitinib, trastuzumab, cetuximab, erlotinib, panitumumab, lapatinib, temsirolimus, everolimus, ipilimumab, vandetanib, crizotinib, ruxolitinib, and trametinib.

Examples of endocrine therapeutic agents include fosfestrol, diethylstilbestrol, chlorotrianiserin, medroxyprogesterone acetate, megestrol acetate, chlormadinone acetate, cyproterone acetate, danazol, allylestrenol, gestrinone, mepartricin, raloxifene, ormeloxifene, levormeloxifene, anti-estrogen (e.g., tamoxifen citrate, toremifene citrate, and the like), pill formulations, mepitiostane, testololactone, aminoglutethimide, LH-RH agonists (e.g., goserelin acetate, buserelin, leuprorelin, and the like), droloxiphene, epitiostanol, ethinylestradiol sulfonate, aromatase inhibitors (e.g., fadrozole hydrochloride, anastrozole, letrozole, exemestane, vorozole, formestane, and the like), antiandrogens (e.g., flutamide, bicalutamide, nilutamide, and the like), 5α-reductase inhibitors (e.g., finasteride, epristeride, and the like), adrenocortical hormonal agents (e.g., dexamethasone, prednisolone, betamethasone, triamcinolone, and the like), androgen synthesis inhibitors (e.g., abiraterone and the like), retinoids, drugs that slow the metabolism of retinoids (e.g., liarozole), and the like.

Examples of chemotherapeutic agents include alkylating agents, antimetabolites, anticancer antibiotics, plant derived anticancer agents, and the like.

Examples of alkylating agents include nitrogen mustard, nitrogen mustard-N-oxide hydrochloride, chlorambucil, cyclophosphamide, ifosfamide, thiotepa, carboquone, improsulfan tosilate, busulfan, nimustine hydrochloride, mitobronitol, melphalan, dacarbazine, ranimustine, estramustine phosphate sodium, triethylenemelamine, carmustine, lomustine, streptozocin, pipobroman, etoglucid, carboplatin, cisplatin, miboplatin, nedaplatin, oxaliplatin, altretamine, ambamustine, dibrospidium hydrochloride, fotemustine, prednimustine, pumitepa, ribomustin, temozolomide, treosulfan, trofosfamide, zinostatin stimalamer, carboquone, adozelesin, cystemustine, bizelesin, and the like.

Examples of antimetabolites include mercaptopurine, 6-mercaptopurine riboside, thioinosine, methotrexate, enocitabine, cytarabine, cytarabine ocphosphate, ancitabine hydrochloride, 5-FU agents (e.g., fluorouracil, tegafur, UFT, doxifluridine, carmofur, galocitabine, emitefur, and the like), aminopterin, leucovorin calcium, tabloid, butocin, calcium folinate, calcium levofolinate, cladribine, emitefur, fludarabine, gemcitabine, hydroxycarbamide, pentostatin, piritrexim, idoxuridine, mitoguazone, tiazofurin, ambamustine, and the like.

Examples of anticancer antibiotics include actinomycin D, actinomycin C, mitomycin C, chromomycin A3, bleomycin hydrochloride, bleomycin sulfate, peplomycin sulfate, daunorubicin hydrochloride, doxorubicin hydrochloride, aclarubicin hydrochloride, pirarubicin hydrochloride, epirubicin hydrochloride, neocarzinostatin, mithramycin, sarkomycin, carzinophilin, mitotane, zorubicin hydrochloride, mitoxantrone hydrochloride, idarubicin hydrochloride, and the like.

Examples of plant derived anticancer agents include etoposide, etoposide phosphate, vinblastine sulfate, vincristine sulfate, vindesine sulfate, teniposide, paclitaxel, docetaxel, vinorelbine, and the like.

Examples of immunotherapeutic agents (BRM) include picibanil, krestin, sizofiran, lentinan, ubenimex, interferon, interleukin, macrophage colony-stimulating factor, granulocyte colony-stimulating factor, lymphotoxin, BCG vaccine, corynebacterium parvum, levamisole, polysaccharide K, procodazole, and the like.

A cell growth factor is typically a peptide having a molecular weight of 20,000 or less, which is an agent exerting action at a low concentration by binding with a receptor. Examples thereof include (1) EGF or substances having substantially the same activity as EGF (e.g., EGF (epidermal growth factor), heregulin (HER3 and HER4 ligands), and the like), (2) insulin or substances having substantially the same activity as insulin (e.g., insulin, IGF (insulin-like growth factor)-1, IGF-2, and the like), (3) FGF (fibroblast growth factor) or substances having substantially the same activity as FGF (e.g., acidic FGF, basic FGF, KGF(keratinocyte growth factor), FGF-10, and the like), and (4) other cell growth factors (e.g., CSF (colony-stimulating factor), IL-2, NGF (nerve growth factor), PDGF (platelet-derived growth factor), TGF-β (transforming growth factor β), HGF (hepatocyte growth factor), VEGF (vascular endothelial growth factor)), and the like.

Examples of agents inhibiting the action of a cell growth factor include Herceptin (anti-HER2 antibody) and the like.

Examples of other agents that can be used in combination with the peptide of the present disclosure or a salt thereof, or a solvate thereof, or a prodrug thereof include L-asparaginase, aceglatone, procarbazine hydrochloride, protoporphyrin-cobalt complex salt, mercury hematoporphyrin sodium, type I topoisomerase inhibitors (e.g., irinotecan, topotecan, and the like), type II topoisomerase inhibitors (e.g., sobuzoxane and the like), lyase inhibitors, endothelin antagonists (e.g., ABT-627 and the like), differentiation inducing agents (e.g., retinoid, vitamin Ds, and the like), angiogenesis inhibitors, α-blockers (e.g., tamsulosin hydrochloride and the like), insulin resistance improving agents (e.g., pioglitazone hydrochloride, rosiglitazone (maleate), GI-262570, JTT-501, MCC-555, YM-440, KRP-297, CS-011, FK-614, (E)-4-[4-(5-methyl-2-phenyl-4-oxazolylmethoxy)benzyloxyimino]-4-phenylbutyric acid, angiotensin II antagonists (e.g., losartan, eprosartan, candesartan, cilexetil, valsartan, telmisartan, irbesartan, tasosartan, olmesartan and their active metabolites (such as candesartan), and the like), cancer antigens, DNAs, lectins, carbohydrates, lipids, and the like.

When using the peptide of the present disclosure or a salt thereof, or a solvate thereof, or a prodrug thereof with an agent used in combination, the timing of administration thereof is not limited. They can be administered simultaneously or differentially. The dosage of the peptide of the present disclosure or a salt thereof, or a prodrug thereof can be appropriately determined depending on the subject of administration, route of administration, disease, combination, or the like.

The administration manner of the peptide of the present disclosure or a salt thereof, or a solvate thereof, or a prodrug thereof and the agent used in combination is not particularly limited. Examples thereof include administration of a single formulation obtained by formulating them together, administration of two or more formulations obtained by independently formulating them via the same or different routes of administration simultaneously or differentially.

As used herein, "or" is used when "at least one or more" of the listed matters in the sentence can be employed. When explicitly described herein as "within the range" of "two values", the range also includes the two values themselves.

### (General technology)

Any molecular biological methodologies, biochemical methodologies, microbiological methodologies, and bioinformatics that is known in the art, well known, or conventional can be used herein.

Reference literatures such as scientific literatures, patents, and patent applications cited herein are incorporated herein by reference to the same extent that the entirety of each document is specifically described.

As described above, the present disclosure has been described by showing preferred embodiments to facilitate understanding. The present disclosure is described hereinafter based on Examples. The above descriptions and the following Examples are not provided to limit the present disclosure, but for the sole purpose of exemplification. Thus, the scope of the present invention is not limited to the embodiments and Examples specifically described herein and is limited only by the scope of claims.

### [Examples]

The Examples are described hereinafter. For reagents, the specific products described in the Examples were used. However, the reagents can be substituted with an equivalent product from another manufacturer (Sigma-Aldrich, Fujifilm Wako Pure Chemical, Nacalai Tesque, R & D Systems, USCN Life Science Inc., or the like).

### (Synthesis of peptides)

The following peptides were synthesized (nomenclature of each compound is shown within the parenthesis). Ac-SCHYGPLT(2-benzothiazolylalanine)VCK-NH-(biotin) (GT-11352)

### Ac-SCHYGPMT(2-benzothiazolylalanine)VCK-NH₂ (ERA-350)

Synthesis, purification, and analysis of the peptides described above were commissioned to GlyTech, Inc. (Osaka, Japan). Naturally-occurring amino acids were purchased from Merck (Germany) (Novabiochem^{®}), etc., and non-naturally-occurring amino acids were purchased from Watanabe Chemical, etc. Amino acids (including non-naturally-occurring amino acids) for synthesizing the peptides described above and protected amino acids thereof are available from any suitable supplier. Rink Amide ChemMatrix (Biotage, Sweden) was used as the starting material to synthesize a protected peptide resin of interest by sequentially extending amino acids from the resin according to the sequences described above by a condensation reaction in accordance with a program with a peptide automatic synthesizer (Prelude (Gyros Protein Technologies)). After the completion of construction of a peptide on the resin, the protected peptide resin was dried. The resulting protected peptide was treated with trifluoroacetic acid to cleave the protecting group and the resin carrier. The crude peptide obtained after cleavage from the resin carrier was purified with an HPLC system; Prominence UFLC (Shimadzu Corporation, Kyoto, Japan) or LaChrom Elite (Hitachi High-Technologies Corporation, Tokyo, Japan). The solvent condition used at the time was A/B gradient, with mobile phase A: aqueous 0.1% TFA solution and mobile phase B: 0.09% TFA/10% H₂O/90% MeCN. The purified peptide (reduced) was converted into a raw peptide (oxidized) by iodine treatment to allow formation of a disulfide bond. Each synthesized peptide was subjected to mass spectrometry to confirm that the desired structure was obtained.

Other exemplary structures of anti-EpoR peptides and performance thereof are understood by referring to WO 2020/054813.

### (Example 1)

The effect of an anti-EpoR peptide in the presence of erythropoietin in bone marrow cells and cancer cells was tested.

Bone marrow cells were harvested from mouse femur (Japan SLC, ICR, female).

The test used human pancreatic cancer derived AsPC-1 cells (ECACC, 96020930) and human breast cancer derived HCC1806 cells (ATCC, CRL-2335) that were passaged (twice/week) under the condition of 37°C and 5% CO₂~95% air using RPMI-1640 medium (Sigma, R8758-500 ML) containing 10% fetal bovine serum (biowest, S182H-500) and 1 mM sodium pyruvate (gibco, 11360-070).

These bone marrow cells (2 × 10⁶ cells) and cancer cells (AsPC-1 cells and HCC1806 cells; both 2 × 10⁵ cells) were incubated with GT-11352 (biotin labeled, 100 µM) for 1 hour at 37° C in the presence or absence of recombinant human erythropoietin (rhEpo) (10 U/mL). Subsequently, the cells were fixed with formalin, blocked, and subjected to immunofluorescent staining. The nucleus was stained by DAPI staining. The cells were observed through fluorescence with a microscope (Keyence, BZ-X800). The following antibodies were used.
*EpoR detection: rat anti-EpoR antibody (Merck, clone BCO-3H2) and anti-rat IgG-Alexa^{®} Fluor 594 (Abcam, ab150160)
*GT-11352 detection: streptavidin-FITC (Tokyo Chemical Industry, S0966)

The results are shown in Figures **2****,** **3****,** and **4****.** EpoR and GT-11352 exhibited colocalization in both bone marrow cells and cancer cells (AsPC-1 cells and HCC1806 cells) in the absence of erythropoietin. Meanwhile, in the presence of erythropoietin, EpoR and GT-11352 exhibited colocalization in cancer cells (AsPC-1 cells and HCC1806 cells), but EpoR and GT-11352 did not exhibit co-localization in bone marrow cells. This suggests that an anti-EpoR peptide does not inhibit erythropoietin action in the bone marrow, but inhibits erythropoietin action in cancer tissue. Since EpoR homodimer receptors are expressed in bone marrow cells such as red blood cell progenitor cells, an anti-EpoR peptide can bind to an EpoR homodimer receptor, but it is suggested the binding property does not prevent the binding of erythropoietin to an EpoR homodimer receptor. Meanwhile, cancer cells express an EpoR/βcR heteroreceptor. Thus, it is suggested that binding property of an anti-EpoR peptide to an EpoR/βcR heteroreceptor is not reduced by the presence of erythropoietin. In view of the results, it is inferred that the anti-EpoR peptide of the present disclosure does not bind to a high affinity binding region, but binds to a low affinity binding region of an EpoR homodimer receptor and inhibits the effect of Epo on an EpoR/βcR heteroreceptor via binding property to the low affinity binding region of the EpoR/βcR heteroreceptor (Figure **1****).** This suggests that the anti-EpoR peptide of the present disclosure suppresses EpoR/βcR heteroreceptor expressing cells of cancer, etc. without inhibiting a function that is normally exerted (production of red blood cells, etc.) by erythropoietin binding to an EpoR homodimer receptor. For this reason, the anti-EpoR peptide of the present disclosure can enable treatment of cancer without inducing anemia.

### (Example 2)

The effect of erythropoietin on a suppression of cancer cell growth by an anti-EpoR peptide was tested.

This Example used human pancreatic cancer derived AsPC-1 cells and human breast cancer derived HCC1806 cells that are similar to those of Example 1.

AsPC-1 cells or HCC1806 cells were seeded on a 96-well plate at 3 × 10³ cells/well. ERA-350 (50 µM), recombinant human Epo (1, 3, 10, 30, or 100 U/mL) or a combination thereof was added, and the cells were cultured for three days. The cell proliferation capability for each well was then measured by a WST assay (DOJINDO LABORATORIES, Cell Counting Kit-8), and the inhibition percentage for each test compound relative to the control (well without test compound) was calculated.

A WST assay is a viable cell count measuring method, which uses water-soluble tetrazolium salt WST-8 as a coloring reagent and uses water-soluble formazan generated by reduction of WST-8 as an indicator of viable cell count. NADH produced by a dehydrogenase in a cell converts WST-8 to water-soluble formazan. Since the generated water-soluble formazan has the maximum absorbance at about 450 nm, the viable cell count is indicated by measuring the absorbance at such a wavelength region. The absorbance at 450 nm was observed in the WST assay.

The results are shown in Figures **5** and **6****.** In both types of cancer cells, the suppression effect by an anti-EpoR peptide was not inhibited by addition of erythropoietin. Since cancer cells express EpoR/βcR heteroreceptors, it was suggested that binding property/inhibition by an anti-EpoR peptide to/against an EpoR/βcR heteroreceptor does not decrease due to the presence of an erythropoietin.

### (Example 3)

The effect of an anti-EpoR peptide and erythropoietin on Epo dependent cancer cells (UT-7/Epo cells) was tested.

Human leukemia derived UT-7 cells (provided by Dr. Fujita at the Hokkaido University) were passaged (twice/week) under the condition of 37°C and 5% CO₂-95% air by using D-MEM medium (Sigma, D5796-500 ML) containing recombinant human erythropoietin (rhEpo, 2 U/mL) and 10% fetal bovine serum (biowest, S182-H-500), and cultured for 12 weeks. The UT-7/Epo cells were washed three times and seeded on a 96-well microplate (1 × 10⁴/well). The cells were cultured in the presence of ERA-350 (0.1, 1, 10, or 100 µM) or rhEpo (0, 0.001, 0.01, 0.1, 1, or 10 U/mL) for 4 days (Figure **7****).** Alternatively, an anti-human EpoR neutralizing antibody (R&D Systems, clone 713210) (0.1, 1, or 10 µg/mL) or ERA-350 (0.1, 1, 10, or 100 µM) was added in the presence of rhEpo (0.1 U/mL), and the cells were cultured for 4 days. The cell proliferation capability for each well was then measured as absorbance at 450 nm by a WST assay (DOJINDO LABORATORIES, Cell Counting Kit-8) (Figure **8****).**

The results are shown in Figures **7** and **8****.** Unlike recombinant human erythropoietin, addition of an anti-EpoR peptide did not result in proliferation of UT-7/Epo cells. Thus, it was observed that an anti-EpoR peptide did not exhibit an agonistic effect on an EpoR. Meanwhile, it was confirmed that an anti-EpoR peptide exhibits similar suppression of cancer cell proliferation as an anti-EpoR neutralizing antibody even in the presence of erythropoietin and is an EpoR antagonist.

### (Example 4)

The effect of an anti-EpoR peptide and erythropoietin on an Epo signaling related protein was tested.

Human pancreatic cancer derived AsPC-1 cells and human breast cancer derived HCC1806 cells were pre-incubated with ERA-350 (0 or 100 µM) for 6 hours in a recombinant human Epo- and serum-free medium. The cells were then cultured for 5 minutes in the presence or absence of recombinant human Epo (10 U/mL). The cells were collected, whole cell lysate was prepared, and Western blot was performed. The following antibodies were used.
*Anti-phosphorylated EpoR antibody: R&D Systems, clone 690710
*Anti-EpoR antibody: Bio-Rad, VPA00265
*Anti-phosphorylated Jak2 antibody: Cell Signaling Technology, clone D15E2
*Anti-Jak2 antibody: Cell Signaling Technology, clone D2E12
*Anti-phosphorylated STAT3 antibody: Cell Signaling Technology, clone 3E2
*Anti-β-actin antibody: Medical & Biological Laboratories, clone 6D1

The results are shown in Figures **9** and **10****.** Self-phosphorylation of Jak2 and STAT3 was observed in AsPC-1 cells and HCC1806 cells, and an anti-EpoR peptide suppressed phosphorylation of Jak2 and STAT3. Further, the suppression effect due to an anti-EpoR peptide was not inhibited by addition of erythropoietin.

Likewise, UT-7/Epo cells (5 × 10⁶ cells) were cultured overnight in the presence or absence of ERA-350 (100 µM) in a recombinant human Epo- and serum-free medium. The cells were then cultured for 5 minutes in the presence or absence of recombinant human Epo (10 U/mL). The cells were collected, whole cell lysate was prepared, and Western blot was performed.

The results are shown in Figure **11****.** An anti-EpoR peptide was observed to inhibit phosphorylation of EpoR and Jak2 induced by erythropoietin. Since phosphorylation of Jak2 was inhibited by an anti-EpoR peptide in the presence of Epo, anti-EpoR peptides were confirmed to selectively antagonize Epo heteroreceptors in the presence of Epo.

### (Example 5)

The combined effect of an anti-EpoR peptide and trastuzumab (Herceptin^{®}) was tested using Herceptin resistant breast cancer cells (BT-474-R cells).

The test used herceptin resistant breast cancer BT-474-R cells (provided by Dr. Kanzaki at the Okayama University) that were passaged (twice/week) under the conditions of 37°C and 5% CO₂-95% air by using D-MEM medium (Sigma, D6046-500 ML) containing 10% fetal bovine serum (biowest, S182-H-500).

BT-474-R cells were seeded on a 96-well plate at 5 × 10³ cells/well. ERA-350 (3, 10, 30, 50, or 100 µM), trastuzumab (Selleck Chemicals, A2007) (0.001, 0.01, 0.1, 1, 10, or 100 µg/mL), or a combination thereof was added, and the cells were cultured for five days. The cell proliferation capability for each well was then measured by a WST assay (DOJINDO LABORATORIES, Cell Counting Kit-8), and the inhibition percentage for each test compound relative to the control (well without test compound) was calculated.

The results are shown in Figure **12****.** ERA-350 exhibited a potent proliferation inhibitory action on BT-474-R cells, but trastuzumab only exhibited a modest inhibitory action. Combination use of ERA-350 and trastuzumab exhibited an additive effect.

### (Example 6)

The combined effect of an anti-EpoR peptide and gemcitabine was tested using AsPC-1 cancer bearing mice.

AsPC-1 cells cultured in the similar manner as Example 1 were collected, suspended in RPMI-1640 medium containing 10% Matrigel (BD Bioscience, 354234), and transplanted subcutaneously to the right side on the back of a 6-week-old male nude mouse (Japan SLC, BALB/cSlc-nu/nu) (5 × 10⁶/0.1 mL/mouse). The length (L) and width (W) of the tumor were measured using a digital caliper. When the tumor volume (V) calculated from the formula V = L × W²/2 reached about 150 to 200 mm³, the mice were separated into groups and administered with test compounds (ERA-350 and ERA-350+gemcitabine). Saline was administered to a control group. The compounds were administered intraperitoneally (0.1 mL/mouse, 6 mice/test compound) once daily (0.2 mg/day for ERA-350, and 1 mg/day for gemcitabine), twice a week for 4 weeks. The tumor size and body weight were measured twice a week to monitor the effect of the administration of test compounds.

The results are shown in Figure **13****.** ERA-350 exhibited a clear antitumor effect, but did not affect the body weight. Combined use with gemcitabine did not decrease body weight with a minor enhancement in action.

### (Additional test)

The following tests are contemplated.
1) Bone marrow cells or cancer cells (AsPC-1 cells, HCC1806 cells, UT-7/Epo cells, etc.) and ERA-350 are cultured for a certain period of time in the presence or absence of human recombinant erythropoietin. The cells are collected to prepare whole cell lysate. Immunoprecipitation/Western blot are then performed using an anti-EpoR antibody and an anti-βcR antibody. Phosphorylation of EpoR or βcR is detected with an anti-phosphorylated tyrosine antibody, or phosphorylation of EpoR or Jak2 is detected with an anti-phosphorylated EpoR antibody and an anti-phosphorylated Jak2 antibody to study the phosphorylation suppression action of ERA-350.
2) EPOR^{HL} (High affinity site + Low affinity site), EPOR^{H} (Low affinity site mutation, M174E), and EPOR^{L} (High affinity site mutation, H138K & E141K) are each expressed in HEK293T cells. A binding reaction between each expression cell and GT-11352 and the Epo action are studied through immunofluorescence, and specific binding of GT-11352 to EPOR^{L} is studied.
3) Human cancer cells (AsPC-1 cells, HCC1806 cells, etc.) are suspended in a Matrigel containing medium, and transplanted intradermally to the back of a 5-week-old female nude mouse (5 × 10⁶/0.1 mL/mouse). After administration of a test compound (ERA-350, bevacizumab, etc.) for a certain period of time to this mouse, Evans blue solution is injected (60 mg/kg) into the caudal vein, and the mouse is slaughtered by cervical dislocation after one hour. The cancer cell transplanted site on the back skin is removed. Evans blue at the site is extracted with formamide, and then the amount of Evans blue is quantified by colorimetry to study the inhibitory action of test compounds alone and in combined use, on angiogenesis induced by cancer cells.
4) Tamoxifen resistant human breast cancer cells (MCF7/TAMR-7 cells, T47D/TR-1 cells, etc.) are added with a test compound (4-hydroxytamoxifen or ERA-350) and cultured for a certain period of time, and then the combined effect of ERA-350 on the cancer cell proliferation inhibitory action by 4-hydroxytamoxifen is studied by a WST assay.
5) Human cancer cells (breast cancer cells, leukemia cells, etc.) are added with a test compound (doxorubicin, ERA-350, or Epo) and cultured for a certain period of time, and then suppression effect by Epo on the cancer cell damaging action of doxorubicin and the enhancement effect due to the combined use of ERA-350 are studied by a WST assay.

### (Example 7)

Localization of EpoR and βcR in cancer cells was studied.

This Example used human pancreatic cancer derived AsPC-1 cells (ECACC, 96020930) and human breast cancer derived HCC1806 cells (ATCC, CRL-2335) cultured in the similar manner as Example 1. These cancer cells (2 × 10⁵ cells) were fixed with a 3.7% formalin solution, dropped onto a slide glass and air-dried. To detect EpoR, EpoR was stained with a rat anti-human EpoR antibody and a goat anti-rat IgG-Alexa^{®} Fluor 594. To detect βcR, βcR was stained with a rabbit anti-human βcR antibody and a goat anti-rabbit IgG-Alexa^{®} Fluor 488. The nucleus was stained with DAPI. Pictures were taken under a fluorescence microscope (Figures **14** and **15****).**

Tumor tissue was removed from an AsPC-1 cancer bearing mouse prepared in the similar manner as Example 6 and fixed with 4.0% paraformaldehyde. To detect EpoR, EpoR was stained with a rat anti-human EpoR antibody and a donkey F(ab')2 anti-rat IgG-Alexa^{®} Fluor 647. To detect βcR, βcR was stained with a rabbit anti-human βcR antibody and a goat anti-rabbit IgG-Alexa^{®} Fluor 488. The nucleus was stained with DAPI. Hematoxylin and eosin stain was also performed. Pictures were taken under a fluorescence microscope and optical microscope (Figure **16****).**

EpoR and βcR exhibited co-localization in cancer cells. Co-localization of EpoR and βcR can be an indicator of cancer and an indicator for administration of the anti-EpoR peptide of the present disclosure.

### (Example 8)

EpoR/βcR complex formation in cancer cells was studied.

AsPC-1 cells were transfected with a wild-type EpoR expressing vector comprising a puromycin resistant marker. Viable cells cultured in the presence of puromycin were obtained, and stable EpoR overexpressing strain was prepared. The stable expression strain was cultured and collected. Membrane fractions were prepared using a commercially available membrane protein extraction kit (Mem-PER Plus Membrane Protein Extraction Kit, Thermo Scientific). The obtained membrane fraction samples were immunoprecipitated with an anti-human EpoR monoclonal antibody (VP-2E8). The samples were subjected to immunoblot with an anti-human EpoR monoclonal antibody (BCO-3H2) or an anti-human βcR polyclonal antibody.

The results are shown in Figure **17****.** The prepared modified strain was confirmed to overexpress EpoR on a cell membrane, and EpoR was observed to form a complex with βcR.

### (Example 9)

A cell strain introduced with a mutant EpoR was prepared to study the responsiveness to an anti-EpoR peptide. It is proposed that EpoR has a high affinity binding region and a low affinity binding region for Epo. It is reported that a mutation of M174E results in the loss of function of a low affinity binding region, and mutations of H138K and E141K result in the loss of function of a high affinity binding region (Zhang B. K. et al. Mol Cell (2009) 33: 266).

### (Preparation of wild-type or mutant EpoR expressing strain)

293T cells were transfected with an expression vector encoding a wild-type or mutant (M174E mutation or H138K/E141K mutation) EpoR comprising a puromycin resistant marker. Viable cells cultured in the presence of puromycin were obtained, and a stable expression strain was prepared. The stable expression strain was cultured and collected, and whole cell lysate was prepared. Expression of EpoR was studied by Western blot by using an anti-EpoR antibody (Figure **18****).** An additional cell strain overexpressing an M174E mutant EpoR was prepared in the similar manner (Figure **21****).**

### (Checking Epo-EpoR signaling in each EpoR expressing strain)

Each stable cell strain (1 × 10⁶) was incubated for 18 hours in a serum free medium and then stimulated for 5 minutes in the presence or absence of human recombinant Epo (rhEpo) (10 U/mL). The cell strain was collected, and whole cell lysate was prepared. Signaling was studied by Western blot using an anti-phosphorylated Jak2 antibody and an anti-Jak2 antibody (Figure **19****).** As a result, enhancement in signaling with Jak2 phosphorylation was observed in cell strains expressing a wild-type EpoR, but Jak2 phosphorylation was at a much lower level in M174E or H138K/E141K mutant EpoR expressing cell strains than in a wild-type EpoR overexpressing strain. It was observed that a mutation of M174E or H138K/E141K does not completely eliminate Epo responsiveness of an EpoR.

### (Responsiveness of ERA-350 to each EpoR expressing strain)

Each EpoR expressing strain was seeded on a 96-well plate at 3 × 10³ cells/well. ERA-350 (50 µM) was added, and the cells were cultured for three days. The cell proliferation capability for each well was then measured by a WST assay (DOJINDO LABORATORIES, Cell Counting Kit-8), and the inhibition percentage relative to the control (well without ERA-350) was calculated.

The results are shown in Figure **20****.** It was observed that the inhibitory effect due to ERA-350 is decreased in an M174E mutant EpoR expressing strain compared to a wild-type or H138K/E141K mutant EpoR expressing strain. It is understood that the anti-EpoR peptide of the present disclosure specifically binds to an Epo low affinity binding region of an EpoR.

### (Example 10)

The responsiveness was compared for anti-EpoR peptides that are different from ERA-350 but within the scope of the present disclosure among the wild-type EpoR expressing strain, M174E mutant EpoR expressing strain, and H138K/E141K mutant EpoR expressing strain, in the similar protocol as Example 9. It was confirmed that other anti-EpoR peptides of the present disclosure, just like ERA-350, tend to decrease in an inhibitory effect in an M174E mutant EpoR expressing strain. Thus, it is understood that various peptides within the scope of the present disclosure have a feature of specifically binding to an Epo low affinity binding region of an EpoR, which is not limited to ERA-350.

### (Note)

As disclosed above, the present disclosure has been exemplified by the use of its preferred embodiments. However, the above descriptions and Examples are not provided to limit the present disclosure but for the sole purpose of exemplification. Thus, it is understood that the present invention is not limited to the embodiments and Examples that are specifically described herein, and the scope thereof should be interpreted based solely on the Claims. It is also understood that any patent, patent application, and references cited herein should be incorporated herein by reference in the same manner as the contents are specifically described herein.

The present application claims priority to Japanese Patent Application No. 2020-129269 filed on July 30, 2020 with the JPO. The entire content thereof is incorporated herein by reference.

### [Industrial Applicability]

The peptide of the present disclosure was found to have a significant anti-cancer activity and low anemia induction, and has applications as a pharmaceutical product. Thus, usefulness thereof is found in industries pertaining to pharmaceutical industries and active pharmaceutical ingredient manufacturers thereof.

### [Sequence Listing Free Text]

SEQ ID NO: 1: SCH(A¹) (A²) (A³) (A⁴) (A⁵) (A⁶)V(A⁷) (A⁸)
SEQ ID NO: 2: SCHFGPLTWVCK
SEQ ID NO: 3: X¹-SCHYGPLTWVCK-X²
SEQ ID NO: 4: X¹-SCHFAPLTWVCK-X²
SEQ ID NO: 5: X¹-SCHFGALTWVCK-X²
SEQ ID NO: 6: X¹-SCHFGPATWVCK-X²
SEQ ID NO: 7: X¹-SCHFGPMTWVCK-X²
SEQ ID NO: 8: X¹-SCHFGPLTMVCK-X²
SEQ ID NO: 9: Ac-SCHFGPLTWVCK-NH₂ (YS12)
SEQ ID NO: 10: Ac-SCHYGPLTWVCK-NH₂ (GT-11261)
SEQ ID NO: 11: Ac-SCHFAPLTWVCK-NH₂ (GT-11264)
SEQ ID NO: 12: Ac-SCHFGALTWVCK-NH₂ (GT-11266)
SEQ ID NO: 13: Ac-SCHFGPATWVCK-NH₂ (GT-11268)
SEQ ID NO: 14: Ac-SCHFGPMTWVCK-NH₂ (GT-11269)
SEQ ID NO: 15: Ac-SCHFGPLTMVCK-NH₂ (GT-11270)
SEQ ID NO: 16: 
MGVHECPAWLWLLLSLLSLPLGLPVLGAPPRLICDSRVLERYLLEAKEAENITTGCAEH
CSLNENITVPDTKVNFYAWKRMEVGQQAVEVWQGLALLSEAVLRGQALLVNSSQPWEPL
QLHVDKAVSGLRSLTTLLRALGAQKEAISPPDAASAAPLRTITADTFRKLFRVYSNFLR
GKLKLYTGEACRTGDR
SEQ ID NO: 17: 
MDHLGASLWPQVGSLCLLLAGAAWAPPPNLPDPKFESKAALLAARGPEELLCFTERLED
LVCFWEEAASAGVGPGNYSFSYQLEDEPWKLCRLHQAPTARGAVRFWCSLPTADTSSFV
PLELRVTAASGAPRYHRVIHINEVVLLDAPVGLVARLADESGHVVLRWLPPPETPMTSH
IRYEVDVSAGNGAGSVQRVEILEGRTECVLSNLRGRTRYTFAVRARMAEPSFGGFWSAW
SEPVSLLTPSDLDPLILTLSLILVVILVLLTVLALLSHRRALKQKIWPGIPSPESEFEG
LFTTHKGNFQLWLYQNDGCLWWSPCTPFTEDPPASLEVLSERCWGTMQAVEPGTDDEGP
LLEPVGSEHAQDTYLVLDKWLLPRNPPSEDLPGPGGSVDIVAMDEGSEASSCSSALASK
PSPEGASAASFEYTILDPSSQLLRPWTLCPELPPTPPHLKYLYLVVSDSGISTDYSSGD
SQGAQGGLSDGPYSNPYENSLIPAAEPLPPSYVACS
SEQ ID NO: 18:

MVLAQGLLSMALLALCWERSLAGAEETIPLQTLRCYNDYTSHITCRWADTQDAQRLVNV
TLIRRVNEDLLEPVSCDLSDDMPWSACPHPRCVPRRCVIPCQSFVVTDVDYFSFQPDRP
LGTRLTVTLTQHVQPPEPRDLQISTDQDHFLLTWSVALGSPQSHWLSPGDLEFEVVYKR
LQDSWEDAAILLSNTSQATLGPEHLMPSSTYVARVRTRLAPGSRLSGRPSKWSPEVCWD
SQPGDEAQPQNLECFFDGAAVLSCSWEVRKEVASSVSFGLFYKPSPDAGEEECSPVLRE
GLGSLHTRHHCQIPVPDPATHGQYIVSVQPRRAEKHIKSSVNIQMAPPSLNVTKDGDSY
SLRWETMKMRYEHIDHTFEIQYRKDTATWKDSKTETLQNAHSMALPALEPSTRYWARVR
VRTSRTGYNGIWSEWSEARSWDTESVLPMWVLALIVIFLTIAVLLALRFCGIYGYRLRR
KWEEKIPNPSKSHLFQNGSAELWPPGSMSAFTSGSPPHQGPWGSRFPELEGVFPVGFGD
SEVSPLTIEDPKHVCDPPSGPDTTPAASDLPTEQPPSPQPGPPAASHTPEKQASSFDFN
GPYLGPPHSRSLPDILGQPEPPQEGGSQKSPPPGSLEYLCLPAGGQVQLVPLAQAMGPG
QAVEVERRPSQGAAGSPSLESGGGPAPPALGPRVGGQDQKDSPVAIPMSSGDTEDPGVA
SGYVSSADLVFTPNSGASSVSLVPSLGLPSDQTPSLCPGLASGPPGAPGPVKSGFEGYV

ELPPIEGRSPRSPRNNPVPPEAKSPVLNPGERPADVSPTSPQPEGLLVLQQVGDYCFLP
GLGPGPLSLRSKPSSPGPGPEIKNLDQAFQVKKPPGQAVPQVPVIQLFKALKQQDYLSL
PPWEVNKPGEVC

## Claims

1. A composition for use in selectively antagonizing an erythropoietin (Epo) heteroreceptor in the presence of Epo, comprising a peptide having a structure of -[SCHFGPLTWVCK]-, or a modified peptide based on said peptide, or a prodrug thereof, or a salt or solvate thereof (an alphabet indicates a one letter code of an amino acid).

2. The composition of claim 1, wherein the modified peptide has a structure of formula (I):
X¹-SCH(A¹) (A²) (A³) (A⁴) (A⁵) (A⁶)V(A⁷) (A⁸)-X²
wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
has a structure of formula (II):
X¹-kcvwtl(B¹)Gfhcs-X²
wherein:
X¹ is an amino terminal side of the peptide, and X² is a carboxy terminal side of the peptide;
B¹ is Gly or D-proline, k, c, v, w, t, l, G, f, h, c, and s each indicate an amino acid, an upper case letter indicates an L form or no enantiomer, and a lower case letter indicates a D form;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

3. The composition of claim 2, satisfying at least one of the following conditions:
A² is Gly,
A³ is Phe,
A⁵ is Thr,
A⁷ is Cys, and
A⁸ is Lys.

4. The composition of claim 2 or 3, wherein A¹ is Tyr, p-fluorophenylalanine, or phenethylglycine.

5. The composition of claim 2 or 3, wherein A¹ is Tyr.

6. The composition of any one of claims 2 to 5, wherein A⁴ is Leu or Met.

7. The composition of any one of claims 2 to 6, wherein A⁴ is Met.

8. The composition of any one of claims 2 to 7, wherein A⁶ is Trp, Met, 8-naphthylalanine, 2-quinolylalanine, 5-chloro-Trp, or 2-benzothiazolylalanine.

9. The composition of any one of claims 2 to 8, wherein A⁶ is 2-benzothiazolylalanine.

10. The composition of any one of claims 2 to 9, wherein
A² is Gly,
A³ is Phe,
A⁵ is Thr,
A⁷ is Cys, and
A⁸ is Lys.

11. The composition of any one of claims 2 to 10, wherein X¹ is -COOH, a benzoyl group, a propionyl group, or a p-fluorophenylacetyl group.

12. The composition of any one of claims 2 to 11, wherein X² is NH₂.

13. The composition of any one of claims 1 to 12, wherein the peptide, or the modified peptide, or the prodrug thereof, or the salt or solvate thereof has substantially no affinity to an Epo homodimer receptor in the presence of Epo.

14. The composition of any one of claims 1 to 13, wherein the peptide, or the modified peptide, or the prodrug thereof, or the salt or solvate thereof has affinity to an Epo homodimer receptor in the absence of Epo.

15. The composition of any one of claims 1 to 14, wherein the Epo heteroreceptor comprises an Epo receptor and a βc receptor.

16. The composition of any one of claims 1 to 15, wherein the peptide, or the modified peptide based on said peptide, or the prodrug thereof, or the salt or solvate thereof, in the presence of Epo, does not antagonize an Epo homodimer receptor, but antagonizes an Epo heteroreceptor comprising an Epo receptor and a βc receptor.

17. The composition of any one of claims 1 to 16, wherein the peptide, or the modified peptide based on said peptide, or the prodrug thereof, or the salt or solvate thereof selectively binds to a low affinity binding region of an EpoR.

18. The composition of any one of claims 1 to 17 for administration in combination with an anticancer agent.

19. The composition of any one of claims 1 to 18 for improving an adverse action elicited by an anticancer agent in the presence of Epo.

20. The composition of claim 18 or 19, wherein the anticancer agent comprises an angiogenesis inhibitor.

21. The composition of any one of claims 18 to 20, wherein the anticancer agent comprises bevacizumab, tamoxifen, doxorubicin, or trastuzumab.

22. A composition for use in treating or preventing a disease, disorder, or symptom **characterized by** the presence of a cell expressing an Epo heteroreceptor, comprising a selective antagonist of an Epo heteroreceptor.

23. A composition for use in treating or preventing a disease, disorder, or symptom **characterized by** the presence of a cell expressing an Epo heteroreceptor, comprising a peptide having a structure of -[SCHFGPLTWVCK]-, or a modified peptide based on said peptide, or a prodrug thereof, or a salt or solvate thereof (an alphabet indicates a one letter code of an amino acid).

24. The composition of claim 22 or 23, wherein administration of the composition does not induce anemia.

25. The composition of any one of claims 22 to 24, wherein the disease is a proliferative disease, rheumatism, diabetic retinopathy, keloid, or adenomyosis.

26. The composition of any one of claims 22 to 25, wherein the disease is glioblastoma, melanoma, small cell esophageal carcinoma, gastric cancer, colon adenocarcinoma, pulmonary adenocarcinoma, pulmonary squamous cell carcinoma, large cell lung carcinoma, small cell lung carcinoma, breast cancer, liver cancer, pancreatic cancer, cervical epidermoid carcinoma, prostatic adenocarcinoma, or leukemia.

27. The composition of any one of claims 22 to 26 for administration to a subject with a serum erythropoietin concentration of several mIU/mL or greater (e.g., 3 mIU/mL or greater).

28. The composition of any one of claims 22 to 27 for administration to a subject with a hemoglobin concentration of 13 g/dL or less.

29. The composition of any one of claims 22 to 28 for administration in combination with an anticancer agent.

30. The composition of claim 29, wherein the anticancer agent comprises an angiogenesis inhibitor.

31. The composition of claim 29, wherein the anticancer agent comprises bevacizumab, tamoxifen, doxorubicin, or trastuzumab.

32. The composition of any one of claims 22 to 31, wherein the modified peptide has a structure of formula (I):
X¹-SCH(A¹) (A²) (A³) (A⁴) (A⁵) (A⁶)V(A⁷) (A⁸)-X²
wherein:
X¹ is an amino terminal side of the peptide, and X² is a carboxy terminal side of the peptide;
A¹ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A² is Ala, D-alanine, or Gly;
A³ is Pro, homoproline, or Ala;
A⁴ is Met, Leu, Ala, or Ile;
A⁵ is Thr or Ala;
A⁶ is -NH-CH(R^{A1})-CO-, wherein R^{A1} has a structure of (a bond, or a linear or branched alkylene group)-(an aryl group or a heteroaryl group optionally substituted with a substituent selected from the group consisting of a linear or branched alkyl group optionally substituted with a halogen atom, a linear or branched methoxyl group optionally substituted with a halogen atom, a halogen atom, and a hydroxyl group);
A⁷ is Cys, homocysteine, or penicillamine;
A⁸ is Lys, Arg, or absent;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃; or
has a structure of formula (II):
X¹-kcvwtl(B¹)Gfhcs-X²
wherein:
X¹ is an amino terminal side of the peptide, and X² is a carboxy terminal side of the peptide;
B¹ is Gly or D-proline, k, c, v, w, t, l, G, f, h, c, and s each indicate an amino acid, an upper case letter indicates an L form or no enantiomer, and a lower case letter indicates a D form;
two sulfur atoms in the peptide may form a disulfide bond;
X¹ is hydrogen, a peptide consisting of any 1 to 3 amino acids, or -C(=O)R¹;
X² is hydrogen, a peptide consisting of any 1 to 3 amino acids, or -NR¹₂;
each R¹ is independently selected from the group consisting of hydrogen, a hydroxyl group, a linear or branched C₁₋₁₀ alkyl group optionally substituted with R², a linear or branched C₁₋₁₀ alkenyl group optionally substituted with R², a linear or branched C₁₋₁₀ alkynyl group optionally substituted with R², an aromatic or non-aromatic 5- to 10-membered ring optionally substituted with R², -OR², and -NR²₂, or two R¹ attached to the same atom together form an aromatic or non-aromatic 5- to 10-membered ring optionally substituted with R²; and
each R² is independently selected from the group consisting of hydrogen, a hydroxyl group, halogen, a benzyl group optionally substituted with halogen or a hydroxyl group, -CH₃, -CH₂CH₃, -CH₂CH₂CH₃, -NH₂, -NHCH₃, -N(CH₃)₂, =O, -COOH, -OCH₃, and -OCH₂CH₃.

33. The composition of claim 32, satisfying at least one of the following conditions:
A² is Gly,
A³ is Phe,
A⁵ is Thr,
A⁷ is Cys, and
A⁸ is Lys.

34. The composition of claim 32 or 33, wherein A¹ is Tyr, p-fluorophenylalanine, or phenethylglycine.

35. The composition of claim 32 or 33, wherein A¹ is Tyr.

36. The composition of any one of claims 32 to 35, wherein A⁴ is Leu or Met.

37. The composition of any one of claims 32 to 36, wherein A⁴ is Met.

38. The composition of any one of claims 32 to 37, wherein A⁶ is Trp, Met, 8-naphthylalanine, 2-quinolylalanine, 5-chloro-Trp, or 2-benzothiazolylalanine.

39. The composition of any one of claims 32 to 38, wherein A⁶ is 2-benzothiazolylalanine.

40. The composition of any one of claims 32 to 39, wherein
A² is Gly,
A³ is Phe,
A⁵ is Thr,
A⁷ is Cys, and
A⁸ is Lys.

41. The composition of any one of claims 32 to 40, wherein X¹ is -COOH, a benzoyl group, a propionyl group, or a p-fluorophenylacetyl group.

42. The composition of any one of claims 32 to 41, wherein X² is NH₂.

43. A agent for use in detecting an Epo heteroreceptor comprising an Epo receptor and a βc receptor.
